# EUROPEAN PATENT SPECIFICATION

(11) **EP 4 401 842 B1**
(45) Date of publication and mention of the grant of the patent: **27.08.2025**
(21) Application number: 22792787.8
(22) Date of filing: 16.09.2022
(51) Int. Cl.: A61P 37/06, C07K 16/28, A61K 39/00

(54) **ANTI-HUMAN CD45RC BINDING DOMAINS AND USES THEREOF**
ANTI HUMANE CD45RC-BINDENDE DOMÄNEN UND DEREN VERWENDUNGEN
DOMAINES DE LIAISON ANTI-CD45RC HUMAIN ET LEURS UTILISATIONS

(30) Priority: 16.09.2021 EP 21306283
(43) Date of publication of application: 24.07.2024
(73) Proprietor: Aboleris Pharma, 44200 Nantes (FR); Nantes Université, 44000 Nantes (FR); INSERM (Institut National de la Santé et de la Recherche Médicale), 75013 Paris (FR)
(72) Inventor: GUILLONNEAU, Carole, 44093 Nantes Cedex 1 (FR); ANEGON, Ignacio, 44093 Nantes Cedex 1 (FR); FAVRE-BULLE, Olivier, 44000 Nantes (FR)
(74) Representative: Icosa
(86) International application number: PCT/EP2022/075794
(87) International publication number: WO 2023/041717

(56) References cited:
- WO-A1-2020/058495
- BOUCAULT LAETITIA ET AL: "Transient antibody targeting of CD45RC inhibits the development of graft-versus-host disease", vol. 4, no. 11, 9 June 2020 (2020-06-09), pages 2501 - 2515, XP055896530, ISSN: 2473-9529, Retrieved from the Internet <URL:https://ashpublications.org/bloodadvances/article-pdf/4/11/2501/1743814/advancesadv2020001688.pdf> DOI: 10.1182/bloodadvances.2020001688

## Description

### FIELD OF INVENTION

The present invention relates to antigen-binding domain specifically binding to CD45RC, and to antibodies and chimeric antigen receptors comprising the same. It further relates to their use in various methods of therapeutic purposes, including for preventing and/or reducing transplant rejection, in particular graft-versus-host disease (GvHD).

### BACKGROUND OF INVENTION

CD45 (also known as leukocyte common antigen (LCA), EC3.1.3.48, T200, Ly5, and PTPRC) constitutes the first and prototypic receptor-like protein tyrosine phosphatase (RPTP). Its expression is restricted to leukocytes, where it is one of the most abundant cell surface glycoproteins, constituting almost 10 % of the cell surface (Trowbridge & Thomas, 1994. Annu Rev Immunol. 12:85-116; Hermiston et al., 2003. Annu Rev Immunol. 21:107-37; Holmes, 2006. Immunology. 117(2):145-55).

CD45 comprises an extracellular domain, a single transmembrane domain and a large cytoplasmic domain. The transmembrane and cytoplasmic domains are highly conserved amongst species. In particular, the cytoplasmic domain of CD45 comprises two tandemly duplicated phosphatase domains, of which only the membrane-proximal domain has enzymatic activity (Desai et al., 1994. EMBO J. 13(17):4002-10). The function of the more C-terminal second phosphatase domain in CD45 remains uncertain although it is suggested that it may contribute to CD45 activity indirectly by stabilizing the first domain. Through this cytoplasmic domain, CD45 functions as a central regulator of phosphotyrosine levels at least in lymphocytes, by modulating the activity of Src family of tyrosine-protein kinases (such as Lck in T cells; or Lyn, Fyn and Lck in B cells) (Palacios & Weiss, 2004. Oncogene. 23(48):7990-8000; Lowell, 2004. Mol Immunol. 41(6-7):631-43).

By contrast with the transmembrane and cytoplasmic domains, the N-terminus extracellular domain of CD45 shows a higher polymorphism among different leukocyte lineages. Indeed, this extracellular domain is heavily glycosylated and contains three alternatively spliced exons (4, 5, and 6 - which encode the A, B and C determinants, respectively) that are both O-linked glycosylated and sialylated (Hermiston et al., 2003. Annu Rev Immunol. 21:107-37; Holmes, 2006. Immunology. 117(2):145-55). CD45 isoforms differing in size, shape, and charge can therefore be generated by a dynamically-controlled alternative splicing in both leukocyte differentiation and cellular activation, leading to changes in the extracellular domain of the molecule (Hall et al., 1988. J Immunol. 141(8):2781-7; Lynch, 2004. Nat Rev Immunol. 4(12):931-40)*.*

The largest CD45 isoform containing all three alternatively spliced exons, CD45RABC, is approximately 235 kDa, while the smallest isoform lacking all three exons, CD45RO, is approximately 180 kDa. In between, isoforms comprising only two (*e.g.*, CD45RAB, CD45RBC) or only one (*e.g.*, CD45RB) of the three exons are possible.

While the function of the different CD45 isoforms is not clear, differential expression of these isoforms has been associated with the level of activation of T cells and allows dissociation of naive vs memory T cells (Birkeland et al., 1989. Proc Natl Acad Sci USA. 86(17):6734-8). For example, CD45RA is present on peripheral naive mature CD4⁺ T cells, while CD45RO is expressed on activated and memory CD4⁺ T cells. CD45RABC is expressed on B cells and their precursors, on a sub-group of dendritic cells and other antigen-presenting cells. Effector memory RA T cells (T_{EMRA}), a subtype of terminally differentiated memory T cells, also re-express the naive T cell marker CD45RA (Koch et al., 2008. Immun Ageing. 5:6). Importantly, this pattern of isoforms expression is highly conserved across species emphasizing its functional role and importance (Hermiston et al., 2003. Annu Rev Immunol. 21:107-37).

The particular expression pattern of the CD45RC isoform on CD4⁺ and CD8⁺ T cells allows to differentiate between functionally distinct alloreactive T cell subsets that behave differently in terms of proliferation and cytokine secretion. In rodents for example, it has been shown that both CD4⁺ and CD8⁺ CD45RC^{high} T cells are precursors and/or potent Tₕ1 effector cells capable of promoting transplant rejection and organ inflammation (Spickett et al., 1983. J Exp Med. 158(3):795-810; Xystrakis et al., 2004. Eur J Immunol. 34(2):408-17), while T cells expressing undetectable or low levels of CD45RC^{low/-} are Tₕ2 and regulatory T cells and inhibit allograft rejection, graft-versus-host disease (GvHD) and cell-mediated autoimmune diseases (Xystrakis et al., 2004. Blood. 104(10):3294-30; Guillonneau et al., 2007. J Clin Invest. 117(4):1096-106; Powrie & Mason, 1990. J Exp Med. 172(6):1701-8). In humans, a high proportion of CD45RC⁺ CD8⁺ T cells before transplantation has been correlated with decreased graft survival in kidney transplanted patients (Ordonez et al., 2013. PLoS One. 8(7):e69791).

The elimination of the CD45RC^{high} T cells population represents therefore a promising approach for inducing immune tolerance in human, thus for preventing, reducing and/or treating transplant rejection (in particular GvHD) and autoimmune diseases.

GvHD - or graft-*versus*-host-disease - is a significant cause of morbidity and mortality in stem cell transplant patients. It is a T cell-mediated immunoreactive process in which donor cells react against recipient cells. Presently, immunosuppression with immunomodulating drugs such as corticosteroids are the mainstay of GvHD prevention. Whilst progresses have been made with improvements in survival outcomes over time, corticosteroids do not prevent GvHD in a high proportion of patients (less than 50 % of patients with acute GvHD and 40-50 % of patients with chronic GvHD depending on initial disease severity - Garnett et al., 2013. Ther Adv Hematol. 4(6):366-378), are associated with significant toxicities, and many of the currently available salvage therapies are associated with increased immunosuppression and infectious complications. Thus, there remains an unmet need for the development of new treatment strategies for GvHD to improve long-term post-transplant outcomes.

The Inventors have previously described that the depletion of CD45RC^{high} T cells represents a new therapy strategy in preventing or reducing transplant rejection by decreasing aggressive effector T cells, while increasing tolerogenic regulatory T cells. Indeed, transient anti-CD45RC mAb treatment triggers rapid CD45RC^{high} T cell apoptosis, while preserving memory immunity. Moreover, the Inventors showed that short term anti-CD45RC antibody treatment results in permanent allograft survival with no signs of chronic rejection (International patent application WO 2016/016442; Picarda et al., 2017. JCI Insight. 2(3):e90088). They have also surprisingly demonstrated that treatment with an anti-CD45RC antibody specifically depleting CD45RC^{high} T cells improved muscle strength (Ouisse et al., 2019. Front Immunol. 10:2131**),** and prevented and treated monogenic diseases, such as Duchenne muscular dystrophy (DMD) or autoimmune polyendocrinopathy-candidiasis-ectodermal dystrophy (APECED) (International patent application WO 2019/115791).

Recently, the Inventors have generated *de novo* humanized anti-human CD45RC antibodies which showed higher efficiency at inducing target T cell apoptosis and improved therapeutic effect, *inter alia* in an immune humanized NSG mouse model of xenogeneic GvHD, in comparison to the commercially available anti-CD45RC antibody MT2 (International patent application WO 2020/058495).

Here, the Inventors took a step further and developed an improved version of these already-improved anti-human CD45RC antibodies of WO 2020/058495, with even better therapeutic effect.

### SUMMARY

The present invention relates to an antigen-binding domain specifically binding to CD45RC, wherein said antigen-binding domain comprises:
(a) a heavy chain variable region (HCVR) which comprises the following three CDRs:
   (i) V_{H}-CDR1 of sequence SEQ ID NO: 10;
   (ii) V_{H}-CDR2 of sequence SEQ ID NO: 11; and
   (iii) V_{H}-CDR3 of sequence SEQ ID NO: 12; and
(b) a light chain variable region (LCVR) which comprises the following three CDRs:
   (i) V_{L}-CDR1 of sequence SEQ ID NO: 13;
   (ii) V_{L}-CDR2 of sequence SEQ ID NO: 14; and
   (iii) V_{L}-CDR3 of sequence SEQ ID NO: 15,
wherein X in SEQ ID NO: 13 is either absent or is selected from the group consisting of Asn (N), Ser (S) and Gly (G); preferably X in SEQ ID NO: 13 is absent.

In one embodiment, the antigen-binding domain comprises:
1) a HCVR of sequence SEQ ID NO: 24 and a LCVR of sequence SEQ ID NO: 25; or
2) a HCVR comprising the three CDRs with SEQ ID NOs: 10, 11 and 12 and framework regions sharing at least 70% sequence identity with the framework regions of SEQ ID NO: 24, and a LCVR comprising the three CDRs with SEQ ID NOs: 13, 14 and 15 and framework regions sharing at least 70% sequence identity with the framework regions of SEQ ID NO: 25, wherein the antigen-binding domain retains its binding specificity to CD45RC;
   wherein X₁ in SEQ ID NO: 25 is either absent or is selected from the group consisting of Asn (N), Ser (S) and Gly (G); preferably X in SEQ ID NO: 13 is absent; and
   wherein X₂ in SEQ ID NO: 25 is selected from the group consisting of Tyr (Y) and Phe (F), preferably X₂ in SEQ ID NO: 25 is Tyr (Y).

In one embodiment, the antigen-binding domain is selected from the group consisting of a single-chain variable fragment (scFv), a tandem-di-scFv, a tandem-tri-scFv, a scFv-Fc, a minibody, a maxibody, a diabody, a triabody, a Fv, a Fab, a Fab', a Fab'-SH, and a F(ab')2.

The present invention further relates to an antibody or antigen-binding fragment thereof specifically binding to CD45RC, wherein said antibody comprises an antigen-binding domain according to the present invention.

In one embodiment, the antibody or antigen-binding fragment thereof further comprises a heavy chain constant region (HCCR) and a light chain constant region (LCCR),
preferably wherein the HCCR comprises an amino acid sequence sharing at least 70% sequence identity with the amino acid sequence of any one of SEQ ID NOs: 26 to 28, more preferably with SEQ ID NO: 26 and the LCCR comprises an amino acid sequence sharing at least 70% sequence identity with the amino acid sequence of SEQ ID NO: 29; and
wherein the antibody or antigen-binding fragment thereof retains its binding specificity to CD45RC.

The present invention further relates to a chimeric antigen receptor (CAR) specifically binding to CD45RC, comprising:
i) at least one extracellular antigen-binding domain according to the present invention;
ii) optionally, an extracellular hinge domain,
iii) at least one transmembrane domain,
   preferably the transmembrane domain is selected from the group consisting of the CD8 transmembrane domain and the CD28 transmembrane domain,
   more preferably the transmembrane domain is selected from the group consisting of the CD8 transmembrane domain with an amino acid sequence sharing at least 70% sequence identity with the amino acid sequence of SEQ ID NO: 49 and the CD28 transmembrane domain with an amino acid sequence sharing at least 70% sequence identity with the amino acid sequence of SEQ ID NO: 50;
iv) at least one intracellular signaling domain comprising at least one primary signaling domain and optionally, one or more costimulatory signaling domain,
   preferably the at least one primary signaling is a signaling domain of CD3ζ,
   preferably the at least one primary signaling is a signaling domain of CD3ζ with an amino acid sequence sharing at least 70% sequence identity with the amino acid sequence of SEQ ID NO: 51.

The present invention further relates to an immune cell population expressing the chimeric antigen receptor according to the present invention at its cell surface.

In one embodiment, the immune cells of said population are selected from the group comprising or consisting of CD4⁺ T cells, CD8⁺ T cells, double positive T cells, double negative T cells, γδ T cells, NK cells, NKT cell, B cells, macrophages, or dendritic cells.

The present invention further relates to a nucleic acid encoding the antigen-binding domain according to the present invention, the antibody or antigen-binding fragment thereof according to the present invention, or the CAR according to the present invention, preferably under control of regulatory elements.

The present invention further relates to the antigen-binding domain according to the present invention, the antibody or antigen-binding fragment thereof according to the present invention, the immune cell population according to the present invention, or the nucleic acid according to the present invention, for use as a medicament.

The present invention further relates to the antigen-binding domain according to the present invention, the antibody or antigen-binding fragment thereof according to the present invention, the immune cell population according to the present invention, or the nucleic acid according to the present invention, for use in inducing immune tolerance in a subject in need thereof.

The present invention further relates to the antigen-binding domain according to the present invention, the antibody or antigen-binding fragment thereof according to the present invention, the immune cell population according to the present invention, or the nucleic acid according to the present invention, for use in depleting CD45RC^{high} cells in a subject in need thereof.

The present invention further relates to the antigen-binding domain according to the present invention, the antibody or antigen-binding fragment thereof according to the present invention, the immune cell population according to the present invention, or the nucleic acid according to the present invention, for use in preventing and/or reducing transplant rejection in a subject in need thereof.

In one embodiment, preventing and/or reducing transplant rejection comprises or consists of preventing and/or reducing graft-versus-host-disease (GvHD).

The present invention further relates to the antigen-binding domain according to the present invention, the antibody or antigen-binding fragment thereof according to the present invention, the immune cell population according to the present invention, or the nucleic acid according to the present invention, for use in preventing, reducing and/or treating CD45RC^{high}-related diseases, disorders or conditions in a subject in need thereof, preferably wherein CD45RC^{high}-related diseases, disorders or conditions are selected from the group consisting of autoimmune diseases, undesired immune responses, monogenic diseases, lymphoma, and cancer.

### DEFINITIONS

### "Antibody" or "Immunoglobulin"

As used herein, the term "immunoglobulin" refers to a protein having a combination of two heavy and two light chains whether or not it possesses any relevant specific immunoreactivity. "Antibodies" refers to such assemblies which have significant known specific immunoreactive activity to an antigen of interest (*e.g*., human CD45RC). The term "anti-hCD45RC antibodies" is used herein to refer to antibodies which exhibit immunological specificity for human CD45RC protein. As explained elsewhere herein, "specificity" for human CD45RC does not exclude cross-reaction with species in which CD45RC is expressed.

Antibodies and immunoglobulins comprise light and heavy chains, with or without an interchain covalent linkage between them. Basic immunoglobulin structures in vertebrate systems are relatively well understood. The generic term "immunoglobulin" comprises five distinct classes of antibody that can be distinguished biochemically. Although the following discussion will generally be directed to the IgG class of immunoglobulin molecules, all five classes of antibodies are within the scope of the present invention. With regard to IgG, immunoglobulins comprise two identical light polypeptide chains of molecular weight of about 23 kDa, and two identical heavy chains of molecular weight of about 53-70 kDa. The four chains are joined by disulfide bonds in a "Y" configuration wherein the light chains bracket the heavy chains starting at the mouth of the "Y" and continuing through the variable region. The light chains of an antibody are classified as either kappa (κ) or lambda (λ). Each heavy chain class may be bonded with either a κ or λ light chain. In general, the light and heavy chains are covalently bonded to each other, and the "tail" regions of the two heavy chains are bonded to each other by covalent disulfide linkages or non-covalent linkages when the immunoglobulins are generated either by hybridomas, B cells or genetically engineered host cells. In the heavy chain, the amino acid sequences run from an N-terminus at the forked ends of the Y configuration to the C-terminus at the bottom of each chain. Those skilled in the art will appreciate that heavy chains are classified as gamma (γ), mu (µ), alpha (α), delta (δ) or epsilon (ε) with some subclasses among them (*e.g.*, γ1-γ4). It is the nature of this chain that determines the "class" of the antibody as IgG, IgM, IgA IgD or IgE, respectively. The immunoglobulin subclasses or "isotypes" (*e.g*., IgG1, IgG2, IgG3, IgG4, IgA1, etc.) are well characterized and are known to confer functional specialization. Modified versions of each of these classes and isotypes are readily discernable to the skilled artisan in view of the instant disclosure and, accordingly, are within the scope of the present invention. As indicated above, the variable region of an antibody allows the antibody to selectively recognize and specifically bind epitopes on antigens. That is, the light chain variable domain (V_{L} domain) and heavy chain variable domain (V_{H} domain) of an antibody combine to form the variable region that defines a three-dimensional antigen binding site. This quaternary antibody structure forms the antigen binding site presents at the end of each arm of the "Y". More specifically, the antigen binding site is defined by three complementarity determining regions (CDRs) on each of the V_{H} and V_{L} chains.

### "Antibody-dependent cell-mediated cytotoxicity" or "ADCC"

As used herein, the term **"antibody-dependent cell-mediated cytotoxicity"** or **"ADCC"** refers to a form of cytotoxicity in which the Fc region of antibodies bound to an antigen-bearing target cell binds Fcγ receptors (FcγRs) of the CD16a type present mainly on NK cells (DiLillo & Ravetch, 2015. Cancer Immunol Res. 3(7):704-13). Binding of certain IgG isotypes to other FcγRs, such as CD32 or CD64, triggers the activation of cells such as monocytes, macrophages and PMNs and secretion of various substances such as lyase, perforin, granzyme, TFN, etc. to mediate inflammation target cell destruction. Engagement of CD 16a on NK cells also triggers production of cytokines (Anegón et al., 1988. J Exp Med. 167(2):452-72). To assess ADCC activity of a molecule of interest, an *in vitro* ADCC assay, such as that described in US patents 5,500,362 or 5,821,337, may be performed.

### "Antibody-dependent cell-mediated phagocytosis" or "ADCP"

As used herein, the term **"antibody-dependent cell-mediated phagocytosis"** or **"ADCP"** refers to a form of phagocytosis in which the Fc region of antibodies bound to an antigen-bearing target cell binds Fcγ receptors (FcγRs) present on certain phagocytes (*e.g*., neutrophils, monocytes, macrophages and dendritic cells). This binding triggers the initiation of a phagocytosis process to mediate target cell destruction.

### "Antigen-binding fragment" or "antigen-binding domain"

As used herein, the terms "antigen-binding fragment" or "antigen-binding domain" refer to a part or region of an antibody or immunoglobulin, which comprises fewer amino acid residues than a whole antibody, but which is capable of binding an antigen and/or of competing with a whole antibody for antigen binding (*e.g.*, for specific binding to human CD45RC). Antigen-binding fragments encompasses, without any limitation, single chain antibodies, Fv, Fab, Fab', Fab'-SH and F(ab')2.

### "Complement dependent cytotoxicity" or "CDC"

The term **"complement dependent cytotoxicity"** or **"CDC"** refers to the lysis of a target cell in the presence of complement. Activation of the classical complement pathway is initiated by the binding of the first component of the complement system to antibodies which are bound to their cognate antigen. To assess complement activation, a CDC assay such as, *e.g.,* the one described in Gazzano-Santoro et al., 1997. J Immunol Methods. 202(2):163-71, may be performed.

### "CD45"

As used herein, the term "CD45" (also known as CD45R or PTPRC) refers to a transmembrane glycoprotein existing in different isoforms. These distinct isoforms of CD45 differ in their extracellular domain structures which arise from alternative splicing of 3 variable exons (exons 4, 5 and 6) coding for the A, B and C determinants, respectively, of the CD45 extracellular region. Antibodies reactive with restricted epitope are clustered as "CD45R". Hence, anti-CD45RA, anti-CD45RB and anti-CD45RC antibodies recognize CD45 isoforms which include the expression of the A, B and C determinants, respectively. In the absence of all three A, B and C determinants, the isoform is then referred to as CD45RO. The various isoforms of CD45 have different extracellular domains, but have an identical extracellular sequence proximal to the membrane, as well as for the transmembrane domain and a large cytoplasmic tail segments containing two tandemly homologous highly conserved phosphatase domains of approximately 300 residues. CD45 and its isoforms non-covalently associate with lymphocyte phosphatase-associated phosphoprotein (LPAP) on T and B lymphocytes. CD45 has been reported to be associated with several other cell surface antigens, including CD1, CD2, CD3, and CD4. CD45 is involved in signaling lymphocytes activation. When preceded by the letter "h" (*e.g.*, hCD45), it is implied that the CD45 is of human origin.

### "CD45RC"

As used herein, the term "CD45RC" refers to a 200-220 kDa single chain type I membrane glycoprotein well-known from the skilled artisan. CD45RC is an alternative splicing isoform of CD45 comprising exon 6 encoding the C determinant (hence the terminology CD45RC, *i.e.,* CD45 Restricted to the C determinant). An amino acid sequence of human CD45RC is given in **SEQ ID NO: 1,** corresponding to UniProt Accession number P08575-10. This CD45RC isoform is expressed on B and NK cells, and a subset of CD8⁺ T cells and CD4⁺ T cells, but not on CD8⁺ or CD4⁺ Treg, CD14⁺ monocytes or PMN (Picarda et al., 2017. JCI Insight. 2(3):e90088). While some monoclonal antibodies can recognize an epitope in the portion of CD45 common to all the different isoforms (these are termed anti-CD45 antibodies), other monoclonal antibodies have restricted specificity to a given isoform, depending on which determinant they recognize (A, B or C). When preceded by the letter "h" (*e.g.*, hCD45RC), it is implied that the CD45RC is of human origin.

### "CDR" or "complementarity determining region"

As used herein, the term "CDR" or "complementarity determining region" means the non-contiguous antigen combining sites found within the variable region of both heavy and light chain polypeptides. CDRs were identified according to the rules of **Table 1,** as deduced from Kabat et al., 1991. Sequences of proteins of immunological interest (5th ed.). Bethesda, MD: U.S. Dep. of Health and Human Services; and Chothia & Lesk, 1987. J Mol Biol. 196(4):901-17:

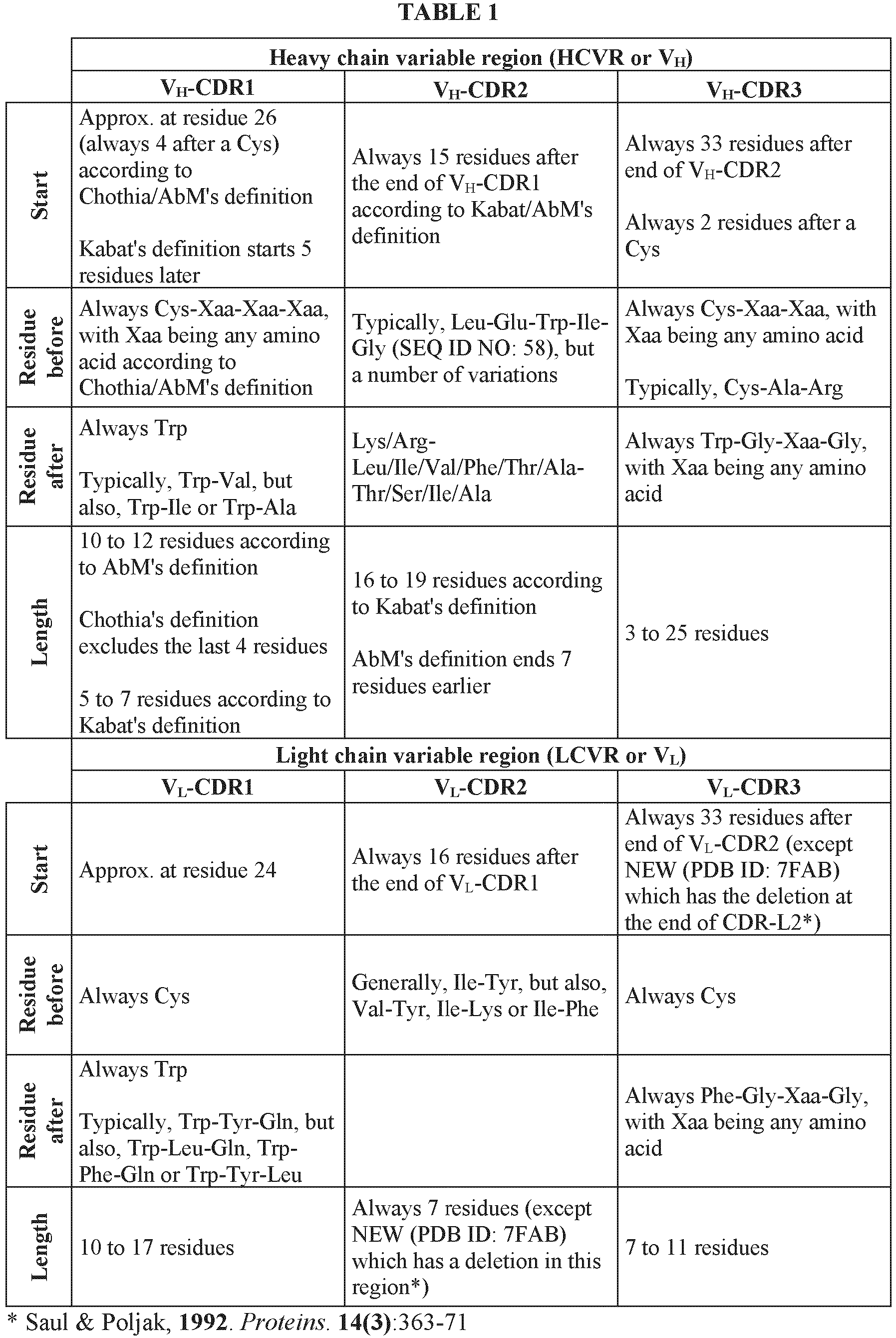

### "Engineered"

As used herein, the term "engineered" or "modified" refers to a cell that has been transfected, transformed or transduced.

### "Epitope"

As used herein, the term "epitope" refers to a specific arrangement of amino acids located on a protein or proteins to which an antibody or binding fragment thereof binds. Epitopes often consist of a chemically active surface grouping of molecules such as amino acids or sugar side chains, and have specific three-dimensional structural characteristics as well as specific charge characteristics. Epitopes can be linear (or sequential) or conformational, *i.e.*, involving two or more sequences of amino acids in various regions of the antigen that may not necessarily be contiguous.

### "Framework region" or "FR" or "non-CDR regions"

As used herein, the terms "framework region", "FR" or "non-CDR regions" include the amino acid residues that are part of the variable region, but are not part of the CDRs (*e.g.*, using the Kabat/Chothia definition of CDRs). Therefore, a variable region framework is between about 100-120 amino acids in length but includes only those amino acids outside of the CDRs.

For the specific example of a HCVR and for the CDRs as defined by Kabat/Chothia:
- FR1 may correspond to the domain of the variable region encompassing amino acids 1-25 according to Chothia/AbM's definition, or 5 residues later according to Kabat's definition;
- FR2 may correspond to the domain of the variable region encompassing amino acids 36-49;
- FR3 may correspond to the domain of the variable region encompassing amino acids 67-98; and
- FR4 may correspond to the domain of the variable region from amino acids 104-110 to the end of the variable region.

The framework regions for the light chain are similarly separated by each of the LCVR's CDRs. In naturally occurring antibodies, the six CDRs present on each monomeric antibody are short, non-contiguous sequences of amino acids that are specifically positioned to form the antigen binding site as the antibody assumes its three-dimensional configuration in an aqueous environment. The remainders of the heavy and light variable domains show less inter-molecular variability in amino acid sequence and are termed the framework regions. The framework regions largely adopt a β-sheet conformation and the CDRs form loops which connect, and in some cases form part of, the β-sheet structure. Thus, these framework regions act to form a scaffold that provides for positioning the six CDRs in correct orientation by inter-chain, non-covalent interactions. The antigen binding site formed by the positioned CDRs defines a surface complementary to the epitope on the immunoreactive antigen. This complementary surface promotes the non-covalent binding of the antibody to the immunoreactive antigen epitope. The position of CDRs can be readily identified by one of ordinary skill in the art.

### "Heavy chain region"

As used herein, the term "heavy chain region" includes amino acid sequences derived from the constant domains of an immunoglobulin heavy chain. A protein comprising a heavy chain region comprises at least one of a C_{H}1 domain, a hinge (*e.g.*, upper, middle, and/or lower hinge region) domain, a C_{H}2 domain, a C_{H}3 domain, or a variant or fragment thereof. In an embodiment, the antibody or binding fragment thereof according to the present invention may comprise the Fc region of an immunoglobulin heavy chain (*e.g.*, a hinge portion, a C_{H}2 domain, and a C_{H}3 domain). In another embodiment, the antibody or binding fragment thereof according to the present invention lacks at least a region of a constant domain (*e.g.*, all or part of a C_{H}2 domain). In certain embodiments, at least one, and preferably all, of the constant domains are derived from a human immunoglobulin heavy chain. For example, in one preferred embodiment, the heavy chain region comprises a fully human hinge domain. In other preferred embodiments, the heavy chain region comprising a fully human Fc region (*e.g.*, hinge, C_{H}2 and C_{H}3 domain sequences from a human immunoglobulin). In certain embodiments, the constituent constant domains of the heavy chain region are from different immunoglobulin molecules. For example, a heavy chain region of a protein may comprise a C_{H}2 domain derived from an IgG1 molecule and a hinge region derived from an IgG3 or IgG4 molecule. In other embodiments, the constant domains are chimeric domains comprising regions of different immunoglobulin molecules. For example, a hinge may comprise a first region from an IgG1 molecule and a second region from an IgG3 or IgG4 molecule. As set forth above, it will be understood by one of ordinary skill in the art that the constant domains of the heavy chain region may be modified such that they vary in amino acid sequence from the naturally occurring (wild-type) immunoglobulin molecule. That is, the antibody or binding fragment thereof according to the present invention may comprise alterations or modifications to one or more of the heavy chain constant domains (C_{H}1, hinge, C_{H}2 or C_{H}3) and/or to the light chain constant domain (C_{L}). Exemplary modifications include additions, deletions or substitutions of one or more amino acids in one or more domains.

### "Hinge region" or "hinge domain"

As used herein, the terms "hinge region" or "hinge domain" are used interchangeably and include the region of an immunoglobulin heavy chain that joins the C_{H}1 domain to the C_{H}2 domain. This hinge region comprises approximately 25 residues and is flexible, thus allowing the two N-terminal antigen binding regions to move independently. Hinge regions can be subdivided into three distinct domains: upper, middle, and lower hinge domains (Roux et al., 1998. J Immunol. 161(8):4083-90). When in the context of a chimeric antigen receptor (CAR), the terms "hinge region" or "hinge domain" refer to the region that connects the extracellular binding domain with the transmembrane domain.

### "Identity" or "identical"

As used herein, the term "identity" or "identical", when used in a relationship between the sequences of two or more amino acid sequences, or of two or more nucleic acid sequences, refers to the degree of sequence relatedness between amino acid sequences or nucleic acid sequences, as determined by the number of matches between strings of two or more amino acid residues or nucleic acid residues. "Identity" measures the percent of identical matches between the smaller of two or more sequences with gap alignments (if any) addressed by a particular mathematical model or computer program (*i.e.*, "algorithms").

Identity of related amino acid sequences or nucleic acid sequences can be readily calculated by known methods. Such methods include, but are not limited to, those described in Lesk, 1988. Computational molecular biology: Sources and methods for sequence analysis. New York, NY: Oxford University Press; Smith, 1993. Biocomputing: Informatics and genome projects. San Diego, CA: Academic Press; Griffin & Griffin, 1994. Computer analysis of sequence data, Part 1. Totowa, NJ: Humana Press; von Heijne, 1987. Sequence analysis in molecular biology: treasure trove or trivial pursuit. San Diego, CA: Academic press; Gribskov & Devereux, 1991. Sequence analysis primer. New York, NY: Stockton Press; Carillo et al., 1988. SIAM J Appl Math. 48(5):1073-82.

Preferred methods for determining identity are designed to give the largest match between the sequences tested. Methods of determining identity are described in publicly available computer programs. Preferred computer program methods for determining identity between two sequences include the GCG program package, including GAP (Genetics Computer Group, University of Wisconsin, Madison, WI; Devereux et al., 1984. Nucleic Acids Res. 12(1 Pt 1):387-95), BLASTP, BLASTN, and FASTA (Altschul et al., 1990. J Mol Biol. 215(3):403-10). The BLASTX program is publicly available from the National Center for Biotechnology Information (NCBI) and other sources (BLAST Manual, Altschul et al. NCB/NLM/NIH Bethesda, Md. 20894). The well-known Smith Waterman algorithm may also be used to determine identity.

### "Immunospecific", "specific for" or "specifically bind"

As used herein, an antibody or binding fragment thereof is said to be "immunospecific", "specific for" or to "specifically bind" an antigen if it reacts at a detectable level with said antigen (*e.g*., hCD45RC), preferably with an affinity constant (K_{A}) of greater than or equal to about 10⁶ M⁻¹, preferably greater than or equal to about 10⁷ M⁻¹, 10⁸ M⁻¹, 5×10⁸ M⁻¹, 10⁹ M⁻¹, 5×10⁹ M⁻¹ or more.

Affinity of an antibody or binding fragment thereof for its cognate antigen is also commonly expressed as an equilibrium dissociation constant (K_{D}). an antibody or binding fragment thereof is said to be "immunospecific", "specific for" or to "specifically bind" an antigen if it reacts at a detectable level with said antigen (*e.g*., hCD45RC), preferably with a K_{D} of less than or equal to 10⁻⁶ M, preferably less than or equal to 10⁻⁷ M, 5×10⁻⁸ M, 10⁻⁸ M, 5×10⁻⁹ M, 10⁻⁹ M or less.

Affinities of antibodies or binding fragment thereof can be readily determined using conventional techniques, for example, those described by Scatchard, 1949. Ann NY Acad Sci. 51:660-672. Binding properties of an antibody or binding fragment thereof to antigens, cells or tissues may generally be determined and assessed using immunodetection methods including, for example, ELISA, immunofluorescence-based assays, such as immuno-histochemistry (IHC) and/or fluorescence-activated cell sorting (FACS) or by surface plasmon resonance (SPR, *e.g.*, using BIAcore^{®}).

### "Monoclonal antibody"

As used herein, the term "monoclonal antibody" refers to an antibody obtained from a population of substantially homogeneous antibodies, *i.e.*, the individual antibodies comprised in the population are identical except for possible naturally occurring mutations that may be present in minor amounts. Monoclonal antibodies are highly specific, being directed against a single antigenic site. Furthermore, in contrast to polyclonal antibody preparations that include different antibodies directed against different determinants (epitopes), each monoclonal antibody is directed against a single determinant on the antigen. In addition to their specificity, the monoclonal antibodies are advantageous in that they may be synthesized uncontaminated by other antibodies. The modifier "monoclonal" is not to be construed as requiring production of the antibody by any particular method. For example, the monoclonal antibodies or binding fragment thereof according to the present invention may be prepared by the hybridoma methodology first described by Kohler et al., 1975. Nature. 256(5517):495-7, or may be made using recombinant DNA methods in bacterial, eukaryotic animal or plant cells (US patent 4,816,567). The "monoclonal antibodies" may also be isolated from phage antibody libraries using the techniques described in Clackson et al., 1991. Nature. 352(6336):624-8 and Marks et al., 1991. J Mol Biol. 222(3):581-97, for example.

### "Prevent" or "preventing" or "prevention"

As used herein, the terms "prevent", "preventing" and "prevention" refer to prophylactic and preventative measures, wherein the object is to reduce the chances that a subject will develop the pathologic condition or disorder over a given period of time. Such a reduction may be reflected, *e.g.*, in a delayed onset of at least one symptom of the pathologic condition or disorder in the subject.

### "Single-chain variable fragment"

As used herein, the term "single-chain variable fragment", also abbreviated **"sFv"** or **"scFv",** refers to an antigen-binding domain comprising a V_{H} and a V_{L} antibody domain connected into a single amino acid chain. Preferably, the scFv amino acid sequence comprises a peptide linker between the V_{H} and the V_{L} domains that enables the scFv to form the desired structure for antigen binding (Plückthun, 1994. Antibodies from Escherichia coli. In Rosenberg & Moore (Eds.), The pharmacology of monoclonal antibodies*.* Handbook of Experimental Pharmacology, 113:269-315. Springer: Berlin, Heidelberg). Divalent, trivalent and higher scFv, called tandem-di-scFv, tandem-tri-scFv, etc., can be engineered by linking two, three or more scFv together. Alternatively, two scFv can be forced to dimerize into a diabody by using a linker peptide that is too short (typically of about 5 to 10 amino acids) between the V_{H} and the V_{L} domains. Diabodies are described more fully in European patent EP 0 404 097, International patent application WO 1993/011161, and in Holliger et al., 1993. Proc Natl Acad Sci USA. 90(14):6444-8. Alternatively, an even shorter linker peptide (typically of about 1 to 2 amino acids) leads to the formation of scFv trimers, also called triabodies or tribodies.

### "Subject"

As used herein, the term "subject" refers to a mammal, preferably a human. In one embodiment, a subject may be a "patient", *i.e.*, a warm-blooded animal, more preferably a human, who/which is awaiting the receipt of, or is receiving medical care or was/is/will be the object of a medical procedure, or is monitored for the development of a disease. The term "mammal" refers here to any mammal, including humans, domestic and farm animals, and zoo, sports, or pet animals, such as dogs, cats, cattle, horses, sheep, pigs, goats, rabbits, etc. Preferably, the mammal is a primate, more preferably a human.

### "Treating" or "treatment" or "alleviation"

As used herein, the terms "treating" or "treatment" or "alleviation" refer to therapeutic treatment, excluding prophylactic or preventative measures; wherein the object is to slow down (lessen) the targeted pathologic condition or disorder. Those in need of treatment include those already with the disorder as well those suspected to have the disorder. A subject is successfully "treated" for the targeted pathologic condition or disorder if, after receiving a therapeutic amount of the isolated antibody or binding fragment thereof, nucleic acid, expression vector, composition, pharmaceutical composition or medicament according to the present invention, said subject shows observable and/or measurable reduction in or absence of one or more of the following: reduction in the number of CD45RC^{high} cells; reduction in the percent of total cells that are CD45RC^{high}; relief to some extent, of one or more of the symptoms associated with the specific disease or condition; reduced morbidity and mortality; and/or improvement in quality of life issues. The above parameters for assessing successful treatment and improvement in the disease are readily measurable by routine procedures familiar to a physician.

### "Variable region" or "variable domain"

As used herein, the term "variable" refers to the fact that certain regions of the variable domains V_{H} and V_{L} differ extensively in sequence among antibodies and are used in the binding and specificity of each particular antibody for its target antigen. However, the variability is not evenly distributed throughout the variable domains of antibodies. It is concentrated in three segments called "hypervariable loops" in each of the V_{L} domain and the V_{H} domain which form part of the antigen binding site.

The first, second and third hypervariable loops of the Vλ light chain domain are referred to herein as L1 (λ), L2 (λ) and L3 (λ) and may be defined as comprising residues 24-33 (L1(λ), consisting of 9, 10 or 11 amino acid residues), 49-53 L2 (λ), consisting of 3 residues) and 90-96 (L3(λ), consisting of 6 residues) in the V_{L} domain (Morea et al., 2000. Methods. 20(3):267-79).

The first, second and third hypervariable loops of the Vκ light chain domain are referred to herein as L1(κ), L2(x) and L3(x) and may be defined as comprising residues 25-33 (L1(κ), consisting of 6, 7, 8, 11, 12 or 13 residues), 49-53 (L2(κ), consisting of 3 residues) and 90-97 (L3(κ), consisting of 6 residues) in the V_{L} domain (Morea et al., 2000. Methods. 20(3):267-79).

The first, second and third hypervariable loops of the V_{H} domain are referred to herein as H1, H2 and H3 and may be defined as comprising residues 25-33 (H1, consisting of 7, 8 or 9 residues), 52-56 (H2, consisting of 3 or 4 residues) and 91-105 (H3, highly variable in length) in the V_{H} domain (Morea et al., 2000. Methods. 20(3):267-79).

Unless otherwise indicated, the terms L1, L2 and L3 respectively refer to the first, second and third hypervariable loops of a V_{L} domain, and encompass hypervariable loops obtained from both Vκ and Vλ isotypes. The terms H1, H2 and H3 respectively refer to the first, second and third hypervariable loops of the V_{H} domain, and encompass hypervariable loops obtained from any of the known heavy chain isotypes, including gamma (γ), mu (µ), alpha (α), delta (δ) or epsilon (ε). The hypervariable loops L1, L2, L3, H1, H2 and H3 may each comprise part of a "complementarity determining region" or "CDR", as defined hereinabove.

### "Vector"

As used herein, the term "vector" refers to a carrier nucleic acid molecule into which a nucleic acid sequence can be inserted, for introduction into a cell where it can be replicated. A nucleic acid sequence can be "exogenous", which means that it is foreign to the cell into which the vector is being introduced or that the sequence is homologous to a sequence in the cell but in a position within the host cell nucleic acid in which the sequence is ordinarily not found. Vectors include DNA vectors, RNA vectors, plasmids, phagemids, phage derivatives, viruses (*e.g*., bacteriophages, animal viruses, and plant viruses), cosmids, and artificial chromosomes (*e.g*., YACs). One skilled in the art would be well equipped to construct a vector through standard recombinant techniques known in the art.

### DETAILED DESCRIPTION

In a first aspect, the present invention relates to an antigen-binding domain, specifically binding to CD45RC, and preferably to human CD45RC (hCD45RC). Hence, the antigen-binding domain of the invention is a **"CD45RC-binding domain",** and preferably a **"hCD45RC-binding domain".**

In one embodiment, the CD45RC-binding domain specifically binds to hCD45RC with SEQ ID NO: 1.

| |
|---|
| SEQ ID NO: 1 MTMYLWLKLLAFGFAFLDTEVFVTGQSPTPSPTDVPGERSTASTFPTDPVSPLTT TLSLAHHSSAALPARTSNTTITANTSDAYLNASETTTLSPSGSAVISTTTIATTPSK PTCDEKYANITVDYLYNKETKLFTAKLNVNENVECGNNTCTNNEVHNLTECK NASVSISHNSCTAPDKTLILDVPPGVEKFQLHDCTQVEKADTTICLKWKNIETFT CDTQNITYRFQCGNMIFDNKEIKLENLEPEHEYKCDSEILYNNHKFTNASKIIKT DFGSPGEPQIIFCRSEAAHQGVITWNPPQRSFHNFTLCYIKETEKDCLNLDKNLIK YDLQNLKPYTKYVLSLHAYIIAKVQRNGSAAMCHFTTKSAPPSQVWNMTVSM TSDNSMHVKCRPPRDRNGPHERYHLEVEAGNTLVRNESHKNCDFRVKDLQYS TDYTFKAYFHNGDYPGEPFILHHSTSYNSKALIAFLAFLIIVTSIALLVVLYKIYD LHKKRSCNLDEQQELVERDDEKQLMNVEPIHADILLETYKRKIADEGRLFLAEF QSIPRVFSKFPIKEARKPFNQNKNRYVDILPYDYNRVELSEINGDAGSNYINASYI DGFKEPRKYIAAQGPRDETVDDFWRMIWEQKATVIVMVTRCEEGNRNKCAEY WPSMEEGTRAFGDVVVKINQHKRCPDYIIQKLNIVNKKEKATGREVTHIQFTS WPDHGVPEDPHLLLKLRRRVNAFSNFFSGPIVVHCSAGVGRTGTYIGIDAMLEG LEAENKVDVYGYVVKLRRQRCLMVQVEAQYILIHQALVEYNQFGETEVNLSE LHPYLHNMKKRDPPSEPSPLEAEFQRLPSYRSWRTQHIGNQEENKSKNRNSNVI PYDYNRVPLKHELEMSKESEHDSDESSDDDSDSEEPSKYINASFIMSYWKPEVM IAAQGPLKETIGDFWQMIFQRKVKVIVMLTELKHGDQEICAQYWGEGKQTYGD IEVDLKDTDKSSTYTLRVFELRHSKRKDSRTVYQYQYTNWSVEQLPAEPKELIS MIQVVKQKLPQKNSSEGNKHHKSTPLLIHCRDGSQQTGIFCALLNLLESAETEE VVDIFQVVKALRKARPGMVSTFEQYQFLYDVIASTYPAQNGQVKKNNHQEDKI EFDNEVDKVKQDANCVNPLGAPEKLPEAKEQAEGSEPTSGTEGPEHSVNGPAS PALNQGS |

In one embodiment, the CD45RC-binding domain specifically binds to the extracellular domain of hCD45RC. In one embodiment, the CD45RC-binding domain specifically binds to at least one epitope present on the extracellular domain of hCD45RC.

In one embodiment, the CD45RC-binding domain specifically binds to the C determinant encoded by exon 6 of hCD45. In one embodiment, the CD45RC-binding domain specifically binds to at least one epitope on the C determinant encoded by exon 6 of hCD45.

In one embodiment, the amino acid sequence of the C determinant encoded by exon 6 of hCD45 comprises or consists of SEQ ID NO: 2.

| |
|---|
| **SEQ ID NO: 2** |
| DVPGERSTASTFPTDPVSPLTTTLSLAHHSSAALPARTSNTTITANTS |

In one embodiment, the CD45RC-binding domain specifically binds to at least one epitope comprising or consisting of a sequence sharing at least about 70%, preferably at least 75%, 80%, 85%, 90%, 91%, 92%, 93%, 94%, 95%, 96%, 97%, 98%, 99% or more identity with SEQ ID NO: 2 or a fragment thereof.

In one embodiment, the CD45RC-binding domain specifically binds to at least one epitope comprising or consisting of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46 or 47 amino acids of SEQ ID NO: 2; or of a sequence sharing at least about 70%, preferably at least 75%, 80%, 85%, 90%, 91%, 92%, 93%, 94%, 95%, 96%, 97%, 98%, 99% or more identity with SEQ ID NO: 2.

In one embodiment, the CD45RC-binding domain specifically binds to at least one epitope comprising or consisting of at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46 or 47 contiguous amino acids of SEQ ID NO: 2; or of a sequence sharing at least about 70%, preferably at least 75%, 80%, 85%, 90%, 91%, 92%, 93%, 94%, 95%, 96%, 97%, 98%, 99% or more identity with SEQ ID NO: 2.

In one embodiment, a fragment of the at least one epitope comprising or consisting of SEQ ID NO: 2 comprises or consists of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46 or 47 amino acid residues, preferably contiguous amino acid residues.

In one embodiment, a fragment of the at least one epitope comprising or consisting of SEQ ID NO: 2 comprises or consists of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46 or 47 amino acid residues spread over a span of 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 360, 370, 380, 390, 400, 410, 420, 430, 440, 450, 460, 470, 480, 490, 500, 510, 520, 530, 540, 550, 560, 570, 580, 590, 600, 610, 620, 630, 640, 650, 660, 670, 680, 690, 700, 710, 720, 73, 740, 750, 760, 770, 780, 790, 800, 810, 820, 830, 840, 850, 860, 870, 880, 890, 900, 910, 920, 930, 940, 950, 960, 970, 980, 990, 1000, 1010, 1020, 1030, 1040, 1050, 1060, 1070, 1080, 1090, 1100 or more contiguous amino acid residues of a sequence comprising or consisting of SEQ ID NO: 2.

In one embodiment, a sequence comprising SEQ ID NO: 2 is the sequence of hCD45RC set forth in SEQ ID NO: 1.

In one embodiment, a sequence comprising SEQ ID NO: 2 is the sequence of hCD45, also known as hCD45RABC (*i.e.*, comprising the A, B and C determinants), as set forth in SEQ ID NO: 3, corresponding to UniProt Accession number P08575-3.

| |
|---|
| SEQ ID NO: 3  |
| |

In one embodiment, the CD45RC-binding domain does not bind to the A determinant encoded by exon 4 of hCD45. In one embodiment, the CD45RC-binding domain does not bind to at least one epitope on the A determinant encoded by exon 4 of hCD45.

In one embodiment, the amino acid sequence of the A determinant encoded by exon 4 of hCD45 comprises or consists of SEQ ID NO: 4.

| |
|---|
| SEQ ID NO: 4 GLTTAKMPSVPLSSDPLPTHTTAFSPASTFERENDFSETTTSLSPDNTSTQVSPDS LDNASAFNTT |

In one embodiment, the CD45RC-binding domain does not bind to the B determinant encoded by exon 5 of hCD45. In one embodiment, the CD45RC-binding domain does not bind to at least one epitope on the B determinant encoded by exon 5 of hCD45.

In one embodiment, the amino acid sequence of the B determinant encoded by exon 4 of hCD45 comprises or consists of SEQ ID NO: 5.

| |
|---|
| **SEQ ID NO: 5** |
| GVSSVQTPHLPTHADSQTPSAGTDTQTFSGSAANAKLNPTPGSNAIS |

In one embodiment, the CD45RC-binding domain does not bind to hCD45RA. In one embodiment, the CD45RC-binding domain does not bind to at least one epitope of hCD45RA.

In one embodiment, the amino acid sequence of hCD45RA comprises or consists of SEQ ID NO: 6, corresponding to UniProt Accession number P08575-8.

| |
|---|
| SEQ ID NO: 6 MTMYLWLKLLAFGFAFLDTEVFVTGQSPTPSPTGLTTAKMPSVPLSSDPLPTHT TAFSPASTFERENDFSETTTSLSPDNTSTQVSPDSLDNASAFNTTDAYLNASETT TLSPSGSAVISTTTIATTPSKPTCDEKYANITVDYLYNKETKLFTAKLNVNENVE CGNNTCTNNEVHNLTECKNASVSISHNSCTAPDKTLILDVPPGVEKFQLHDCTQ VEKADTTICLKWKNIETFTCDTQNITYRFQCGNMIFDNKEIKLENLEPEHEYKC DSEILYNNHKFTNASKIIKTDFGSPGEPQIIFCRSEAAHQGVITWNPPQRSFHNFT LCYIKETEKDCLNLDKNLIKYDLQNLKPYTKYVLSLHAYIIAKVQRNGSAAMC HFTTKSAPPSQVWNMTVSMTSDNSMHVKCRPPRDRNGPHERYHLEVEAGNTL VRNESHKNCDFRVKDLQYSTDYTFKAYFHNGDYPGEPFILHHSTSYNSKALIAF LAFLIIVTSIALLVVLYKIYDLHKKRSCNLDEQQELVERDDEKQLMNVEPIHADI LLETYKRKIADEGRLFLAEFQSIPRVFSKFPIKEARKPFNQNKNRYVDILPYDYN RVELSEINGDAGSNYINASYIDGFKEPRKYIAAQGPRDETVDDFWRMIWEQKAT VIVMVTRCEEGNRNKCAEYWPSMEEGTRAFGDVVVKINQHKRCPDYIIQKLNI VNKKEKATGREVTHIQFTSWPDHGVPEDPHLLLKLRRRVNAFSNFFSGPIVVHC SAGVGRTGTYIGIDAMLEGLEAENKVDVYGYVVKLRRQRCLMVQVEAQYILIH QALVEYNQFGETEVNLSELHPYLHNMKKRDPPSEPSPLEAEFQRLPSYRSWRTQ HIGNQEENKSKNRNSNVIPYDYNRVPLKHELEMSKESEHDSDESSDDDSDSEEP SKYINASFIMSYWKPEVMIAAQGPLKETIGDFWQMIFQRKVKVIVMLTELKHG DQEICAQYWGEGKQTYGDIEVDLKDTDKSSTYTLRVFELRHSKRKDSRTVYQY QYTNWSVEQLPAEPKELISMIQVVKQKLPQKNSSEGNKHHKSTPLLIHCRDGSQ QTGIFCALLNLLESAETEEVVDIFQVVKALRKARPGMVSTFEQYQFLYDVIAST |
| YPAQNGQVKKNNHQEDKIEFDNEVDKVKQDANCVNPLGAPEKLPEAKEQAEG SEPTSGTEGPEHSVNGPASPALNQGS |

In one embodiment, the CD45RC-binding domain does not bind to hCD45RB. In one embodiment, the CD45RC-binding domain does not bind to at least one epitope of hCD45RB.

In one embodiment, the amino acid sequence of hCD45RB comprises or consists of SEQ ID NO: 7, corresponding to UniProt Accession number P08575-9.

| |
|---|
| SEQ ID NO: 7 MTMYLWLKLLAFGFAFLDTEVFVTGQSPTPSPTGVSSVQTPHLPTHADSQTPSA GTDTQTFSGSAANAKLNPTPGSNAISDAYLNASETTTLSPSGSAVISTTTIATTPS KPTCDEKYANITVDYLYNKETKLFTAKLNVNENVECGNNTCTNNEVHNLTEC KNASVSISHNSCTAPDKTLILDVPPGVEKFQLHDCTQVEKADTTICLKWKNIETF TCDTQNITYRFQCGNMIFDNKEIKLENLEPEHEYKCDSEILYNNHKFTNASKIIK TDFGSPGEPQIIFCRSEAAHQGVITWNPPQRSFHNFTLCYIKETEKDCLNLDKNLI KYDLQNLKPYTKYVLSLHAYIIAKVQRNGSAAMCHFTTKSAPPSQVWNMTVS MTSDNSMHVKCRPPRDRNGPHERYHLEVEAGNTLVRNESHKNCDFRVKDLQY STDYTFKAYFHNGDYPGEPFILHHSTSYNSKALIAFLAFLIIVTSIALLVVLYKIY DLHKKRSCNLDEQQELVERDDEKQLMNVEPIHADILLETYKRKIADEGRLFLAE FQSIPRVFSKFPIKEARKPFNQNKNRYVDILPYDYNRVELSEINGDAGSNYINAS YIDGFKEPRKYIAAQGPRDETVDDFWRMIWEQKATVIVMVTRCEEGNRNKCA EYWPSMEEGTRAFGDVVVKINQHKRCPDYIIQKLNIVNKKEKATGREVTHIQFT SWPDHGVPEDPHLLLKLRRRVNAFSNFFSGPIVVHCSAGVGRTGTYIGIDAMLE GLEAENKVDVYGYVVKLRRQRCLMVQVEAQYILIHQALVEYNQFGETEVNLS ELHPYLHNMKKRDPPSEPSPLEAEFQRLPSYRSWRTQHIGNQEENKSKNRNSNV IPYDYNRVPLKHELEMSKESEHDSDESSDDDSDSEEPSKYINASFIMSYWKPEV MIAAQGPLKETIGDFWQMIFQRKVKVIVMLTELKHGDQEICAQYWGEGKQTY GDIEVDLKDTDKSSTYTLRVFELRHSKRKDSRTVYQYQYTNWSVEQLPAEPKE LISMIQVVKQKLPQKNSSEGNKHHKSTPLLIHCRDGSQQTGIFCALLNLLESAET EEVVDIFQVVKALRKARPGMVSTFEQYQFLYDVIASTYPAQNGQVKKNNHQE |
| DKIEFDNEVDKVKQDANCVNPLGAPEKLPEAKEQAEGSEPTSGTEGPEHSVNG PASPALNQGS |

In one embodiment, the CD45RC-binding domain does not bind to hCD45RAB. In one embodiment, the CD45RC-binding domain does not bind to at least one epitope of hCD45RAB.

In one embodiment, the amino acid sequence of hCD45RAB comprises or consists of SEQ ID NO: 8, corresponding to UniProt Accession number P08575-5.

| |
|---|
| SEQ ID NO: 8 MTMYLWLKLLAFGFAFLDTEVFVTGQSPTPSPTGLTTAKMPSVPLSSDPLPTHT TAFSPASTFERENDFSETTTSLSPDNTSTQVSPDSLDNASAFNTTGVSSVQTPHLP THADSQTPSAGTDTQTFSGSAANAKLNPTPGSNAISDAYLNASETTTLSPSGSAV ISTTTIATTPSKPTCDEKYANITVDYLYNKETKLFTAKLNVNENVECGNNTCTN NEVHNLTECKNASVSISHNSCTAPDKTLILDVPPGVEKFQLHDCTQVEKADTTI CLKWKNIETFTCDTQNITYRFQCGNMIFDNKEIKLENLEPEHEYKCDSEILYNNH KFTNASKIIKTDFGSPGEPQIIFCRSEAAHQGVITWNPPQRSFHNFTLCYIKETEK DCLNLDKNLIKYDLQNLKPYTKYVLSLHAYIIAKVQRNGSAAMCHFTTKSAPPS QVWNMTVSMTSDNSMHVKCRPPRDRNGPHERYHLEVEAGNTLVRNESHKNC DFRVKDLQYSTDYTFKAYFHNGDYPGEPFILHHSTSYNSKALIAFLAFLIIVTSIA LLVVLYKIYDLHKKRSCNLDEQQELVERDDEKQLMNVEPIHADILLETYKRKIA DEGRLFLAEFQSIPRVFSKFPIKEARKPFNQNKNRYVDILPYDYNRVELSEINGD AGSNYINASYIDGFKEPRKYIAAQGPRDETVDDFWRMIWEQKATVIVMVTRCE EGNRNKCAEYWPSMEEGTRAFGDVVVKINQHKRCPDYIIQKLNIVNKKEKATG REVTHIQFTSWPDHGVPEDPHLLLKLRRRVNAFSNFFSGPIVVHCSAGVGRTGT YIGIDAMLEGLEAENKVDVYGYVVKLRRQRCLMVQVEAQYILIHQALVEYNQ FGETEVNLSELHPYLHNMKKRDPPSEPSPLEAEFQRLPSYRSWRTQHIGNQEEN KSKNRNSNVIPYDYNRVPLKHELEMSKESEHDSDESSDDDSDSEEPSKYINASFI MSYWKPEVMIAAQGPLKETIGDFWQMIFQRKVKVIVMLTELKHGDQEICAQY WGEGKQTYGDIEVDLKDTDKSSTYTLRVFELRHSKRKDSRTVYQYQYTNWSV EQLPAEPKELISMIQVVKQKLPQKNSSEGNKHHKSTPLLIHCRDGSQQTGIFCAL LNLLESAETEEVVDIFQVVKALRKARPGMVSTFEQYQFLYDVIASTYPAQNGQ |
| VKKNNHQEDKIEFDNEVDKVKQDANCVNPLGAPEKLPEAKEQAEGSEPTSGTE GPEHSVNGPASPALNQGS |

In one embodiment, the CD45RC-binding domain does not bind to hCD45RO. In one embodiment, the CD45RC-binding domain does not bind to at least one epitope of hCD45RO.

In one embodiment, the amino acid sequence of hCD45RO comprises or consists of SEQ ID NO: 9, corresponding to UniProt Accession number P08575-4.

| |
|---|
| SEQ ID NO: 9 MTMYLWLKLLAFGFAFLDTEVFVTGQSPTPSPTDAYLNASETTTLSPSGSAVIST TTIATTPSKPTCDEKYANITVDYLYNKETKLFTAKLNVNENVECGNNTCTNNEV HNLTECKNASVSISHNSCTAPDKTLILDVPPGVEKFQLHDCTQVEKADTTICLK WKNIETFTCDTQNITYRFQCGNMIFDNKEIKLENLEPEHEYKCDSEILYNNHKFT NASKIIKTDFGSPGEPQIIFCRSEAAHQGVITWNPPQRSFHNFTLCYIKETEKDCL NLDKNLIKYDLQNLKPYTKYVLSLHAYIIAKVQRNGSAAMCHFTTKSAPPSQV WNMTVSMTSDNSMHVKCRPPRDRNGPHERYHLEVEAGNTLVRNESHKNCDF RVKDLQYSTDYTFKAYFHNGDYPGEPFILHHSTSYNSKALIAFLAFLIIVTSIALL VVLYKIYDLHKKRSCNLDEQQELVERDDEKQLMNVEPIHADILLETYKRKIADE GRLFLAEFQSIPRVFSKFPIKEARKPFNQNKNRYVDILPYDYNRVELSEINGDAG SNYINASYIDGFKEPRKYIAAQGPRDETVDDFWRMIWEQKATVIVMVTRCEEG NRNKCAEYWPSMEEGTRAFGDVVVKINQHKRCPDYIIQKLNIVNKKEKATGRE VTHIQFTSWPDHGVPEDPHLLLKLRRRVNAFSNFFSGPIVVHCSAGVGRTGTYI GIDAMLEGLEAENKVDVYGYVVKLRRQRCLMVQVEAQYILIHQALVEYNQFG ETEVNLSELHPYLHNMKKRDPPSEPSPLEAEFQRLPSYRSWRTQHIGNQEENKS KNRNSNVIPYDYNRVPLKHELEMSKESEHDSDESSDDDSDSEEPSKYINASFIMS YWKPEVMIAAQGPLKETIGDFWQMIFQRKVKVIVMLTELKHGDQEICAQYWG EGKQTYGDIEVDLKDTDKSSTYTLRVFELRHSKRKDSRTVYQYQYTNWSVEQL PAEPKELISMIQVVKQKLPQKNSSEGNKHHKSTPLLIHCRDGSQQTGIFCALLNL LESAETEEVVDIFQVVKALRKARPGMVSTFEQYQFLYDVIASTYPAQNGQVKK NNHQEDKIEFDNEVDKVKQDANCVNPLGAPEKLPEAKEQAEGSEPTSGTEGPE HSVNGPASPALNQGS |

In one embodiment, the at least one epitope is a conformational epitope. In another embodiment, the at least one epitope is a sequential epitope.

In one embodiment, the CD45RC-binding domain binds to hCD45RC with an equilibrium dissociation constant (K_{d}) of about 5×10⁻⁷ M or less, preferably of about 2.5×10⁻⁷ M or less, about 1×10⁻⁷ M or less, about 7.5×10⁻⁸ M or less, about 5×10⁻⁸ M or less, about 2.5×10⁻⁸ M or less, about 1×10⁻⁸ M or less, about 7.5×10⁻⁹ M or less, about 5×10⁻⁹ M or less, about 2.5×10⁻⁹ M or less, about 1×10⁻⁹ M or less, as may be determined, *e.g.,* by Biacore on immobilized hCD45RC.

In one embodiment, the CD45RC-binding domain binds to hCD45RC with a K_{d} of less than 10 nM, such as 9 nM, 8 nM, 7 nM, 6 nM, 5 nM, 4 nM, 3 nM or less, as may be determined, *e.g*., by Biacore on immobilized hCD45RC. In one embodiment, the CD45RC-binding domain binds to hCD45RC with a K_{d} of about 2.24 nM determined by Biacore on immobilized hCD45RC.

In one embodiment, the CD45RC-binding domain binds to hCD45RC with an association rate (Kₒₙ) of about 1×10⁴ M⁻¹sec⁻¹ or more, preferably of about 5×10⁴ M⁻¹sec⁻¹ or more, about 1×10⁵ M⁻¹sec⁻¹ or more, about 2.5×10⁵ M⁻¹sec⁻¹ or more, about 5×10⁵ M⁻¹sec⁻¹ or more.

In one embodiment, the CD45RC-binding domain binds to hCD45RC with a dissociation rate (K_{off}) of about 5×10⁻² sec⁻¹ or less, preferably of about 4×10⁻² sec⁻¹ or less, about 3×10⁻² sec⁻¹ or less, about 2×10⁻² sec⁻¹ or less, about 1.5×10⁻² sec⁻¹ or less.

In one embodiment, the CD45RC-binding domain binds to hCD45RC with at least one of, preferably at least two of, more preferably the three of:
- an equilibrium dissociation constant (K_{d}) of about 5×10⁻⁷ M or less, preferably of about 2.5×10⁻⁷ M or less, about 1×10⁻⁷ M or less, about 7.5×10⁻⁸ M or less, about 5×10⁻⁸ M or less, about 1×10⁻⁸ M or less;
- an association rate (Kₒₙ) of about 1×10⁴ M⁻¹sec⁻¹ or more, preferably of about 5×10⁴ M⁻¹sec⁻¹ or more, about 1×10⁵ M⁻¹sec⁻¹ or more, about 2.5×10⁵ M⁻¹sec⁻¹ or more, about 5×10⁵ M⁻¹sec⁻¹ or more; and
- a dissociation rate (K_{off}) of about 5×10⁻² sec⁻¹ or less, preferably of about 4×10⁻² sec⁻¹ or less, about 3×10⁻² sec⁻¹ or less, about 2×10⁻² sec⁻¹ or less, about 1.5×10⁻² sec⁻¹ or less.

Methods for determining the affinity (including, for example, determining the K_{d}, k_{off} and kₒₙ) of an antigen-binding domain or of an antibody for its ligand are well-known in the art, and include, without limitation, surface plasmon resonance (SPR), fluorescence-activated cell sorting (FACS), enzyme-linked immunosorbent assay (ELISA), AlphaLISA and KinExA.

A preferred method is BIAcore^{®}, which relies on SPR using immobilized CD45RC to determine the affinity of an antigen-binding domain or of an antibody.

In one embodiment, the CD45RC-binding domain is an isolated CD45RC-binding domain.

By **"isolated",** it is meant that the CD45RC-binding domain is substantially free of other antigen-binding domains having different antigenic specificities; in other words, the CD45RC-binding domain is substantially free of antigen-binding domains that specifically bind antigens other than CD45RC. An isolated CD45RC-binding domain may, however, have cross-reactivity to other antigens, in particular to CD45RC proteins from other species. Moreover, an isolated CD45RC-binding domain may be substantially free of other cellular material and/or chemicals, in particular those that would interfere with diagnostic or therapeutic uses of the CD45RC-binding domain, including without limitation, enzymes, hormones, and other proteinaceous or non-proteinaceous components.

In one embodiment, the isolated CD45RC-binding domain is therefore purified.

In one embodiment, the isolated CD45RC-binding domain is purified to:
(1) greater than 80%, 85%, 90%, 91%, 92%, 93%, 94%, 95% or more by weight of CD45RC-binding domain, as may be determined by the Lowry method, and most preferably more than 96%, 97%, 98% or 99% by weight;
(2) a degree sufficient to obtain at least 15 residues of N-terminal or internal amino acid sequence by use of a spinning cup sequenator; or
(3) homogeneity, as may be shown by SDS-PAGE under reducing or non-reducing conditions and using Coomassie blue or, preferably, silver staining.

In one embodiment, the antigen-binding domain is a molecule selected from the group comprising or consisting of a single-chain antibody, a Fv, a Fab, a Fab', a Fab'-SH, and a F(ab')2.

In one embodiment, the antigen-binding domain is a single-chain antibody. In one embodiment, the single-chain antibody is selected from the group comprising or consisting of a single-chain variable fragment (scFv), a tandem-di-scFv, a tandem-tri-scFv, a scFv-Fc, a (scFv-C_{H}3)₂ (also termed minibody), a (scFv-C_{H}2-C_{H}3)₂ (also termed maxibody), a diabody, and a triabody.

In one embodiment, the antigen-binding domain is a scFv.

In one embodiment, the scFv comprises a linker between the V_{H} and the V_{L} domains that enables the scFv to form the desired structure for antigen binding. In one embodiment, the linker is a short polypeptide, preferably having a length ranging from 2 to 25 amino acids.

For example, glycine-serine linkers provide particularly suitable linkers. Examples of glycine-serine linkers include, but are not limited to, (GS)ₙ linkers, (G₂S)ₙ linkers, (G₃S)ₙ linkers, (G₄S)ₙ linkers, etc.; with n being a positive integer. In one embodiment, n is equal to 1, 2, 3, 4, 5 or more.

Particular examples of glycine-serine linkers include, but are not limited to, SEQ ID NOs: 30-39.

| |
|---|
| **SEQ ID NO: 30** |
| GGGS |
| **SEQ ID NO: 31** |
| GGGSGGGS |
| **SEQ ID NO: 32** |
| GGGSGGGSGGGS |
| **SEQ ID NO: 33** |
| GGGSGGGSGGGSGGGS |
| **SEQ ID NO: 34** |
| GGGSGGGSGGGSGGGSGGGS |
| **SEQ ID NO: 35** |
| GGGGS |
| **SEQ ID NO: 36** |
| GGGGSGGGGS |
| **SEQ ID NO: 37** |
| GGGGSGGGGSGGGGS |
| **SEQ ID NO: 38** |
| GGGGSGGGGSGGGGSGGGGS |
| **SEQ ID NO: 39** |
| GGGGSGGGGSGGGGSGGGGSGGGGS |

In the following, and unless explicitly mentioned otherwise, CDR numbering and definitions are according to the Kabat/Chothia definition.

In one embodiment, the CD45RC-binding domain comprises a heavy chain variable region (HCVR) which comprises at least one, preferably at least two, more preferably the following three CDRs:
**V_{H}-CDR1:** NYYIG (SEQ ID NO: 10);
**V_{H}-CDR2**: DIFPGGDYANYNEKFQG (SEQ ID NO: 11); and
**V_{H}-CDR3:** RNFDY (SEQ ID NO: 12).

In one embodiment, the CD45RC-binding domain comprises a HCVR which comprises the following three CDRs:
**V_{H}-CDR1**: NYYIG (SEQ ID NO: 10);
**V_{H}-CDR2**: DIFPGGDYANYNEKFQG (SEQ ID NO: 11); and
**V_{H}-CDR3:** RNFDY (SEQ ID NO: 12).

In one embodiment, the CD45RC-binding domain comprises a light chain variable region (LCVR) which comprises at least one, preferably at least two, more preferably the following three CDRs:
**V_{L}-CDR1:** RASSSVS-X-YMH (SEQ ID NO: 13);
**V_{L}-CDR2:** NTANLPS (SEQ ID NO: 14); and
**V_{L}-CDR3:** QQRSSYPLTF (SEQ ID NO: 15).
with X being absent or being selected from the group comprising or consisting of Asn (N), Ser (S) and Gly (G), preferably with X being absent.

In one embodiment, the CD45RC-binding domain comprises a LCVR which comprises the following three CDRs:
**V_{L}-CDR1:** RASSSVS-X-YMH (SEQ ID NO: 13);
**V_{L}-CDR2:** NTANLPS (SEQ ID NO: 14); and
**V_{L}-CDR3:** QQRSSYPLTF (SEQ ID NO: 15).
with X being absent or being selected from the group comprising or consisting of Asn (N), Ser (S) and Gly (G), preferably with X being absent.

In one embodiment, the CD45RC-binding domain comprises:
- a HCVR which comprises at least one, preferably at least two, more preferably the following three CDRs:
   **V_{H}-CDR1:** NYYIG (SEQ ID NO: 10);
   **V_{H}-CDR2**: DIFPGGDYANYNEKFQG (SEQ ID NO: 11); and
   **V_{H}-CDR3:** RNFDY (SEQ ID NO: 12); and
- a LCVR which comprises at least one, preferably at least two, more preferably the following three CDRs:
   **V_{L}-CDR1:** RASSSVS-X-YMH (SEQ ID NO: 13);
   **V_{L}-CDR2:** NTANLPS (SEQ ID NO: 14); and
   **V_{L}-CDR3:** QQRSSYPLTF (SEQ ID NO: 15).
with X being absent or being selected from the group comprising or consisting of Asn (N), Ser (S) and Gly (G), preferably with X being absent.

In one embodiment, the CD45RC-binding domain comprises:
- a HCVR which comprises the following three CDRs:
   **V_{H}-CDR1:** NYYIG (SEQ ID NO: 10);
   **V_{H}-CDR2**: DIFPGGDYANYNEKFQG (SEQ ID NO: 11); and
   **V_{H}-CDR3:** RNFDY (SEQ ID NO: 12); and
- a LCVR which comprises the following three CDRs:
   **V_{L}-CDR1:** RASSSVS-X-YMH (SEQ ID NO: 13);
   **V_{L}-CDR2:** NTANLPS (SEQ ID NO: 14); and
   **V_{L}-CDR3:** QQRSSYPLTF (SEQ ID NO: 15).
with X being absent or being selected from the group comprising or consisting of Asn (N), Ser (S) and Gly (G), preferably with X being absent.

In one embodiment, any of V_{H}-CDR1, V_{H}-CDR2, V_{H}-CDR3, V_{L}-CDR1, V_{L}-CDR2 and/or V_{L}-CDR3 as defined hereinabove can be characterized as having an amino acid sequence that shares at least 70%, 75%, 80%, 85%, 90%, 95%, 96%, 97%, 98%, 99% or more of identity with the particular CDR or sets of CDRs as defined hereinabove, while the CD45RC-binding domain retains its overall functional properties, in particular its binding specificity to CD45RC, preferably to hCD45RC.

In one embodiment, the CD45RC-binding domain comprises a HCVR which comprises at least one, preferably at least two, more preferably at least three, even more preferably the four following framework regions (FRs):
**V_{H}-FR1:** QVQLVQSGAEVKKPGASVKVSCKASGYTFT (SEQ ID NO: 16);
**V_{H}-FR2**: WVRQAPGQGLEWIG (SEQ ID NO: 17);
**V_{H}-FR3**: RVTLTADTSISTAYMELSRLRSDDTVVYYCVR (SEQ ID NO: 18);
**V_{H}-FR4**: WGQGTLVTVSS (SEQ ID NO: 19).

In one embodiment, the CD45RC-binding domain comprises a HCVR which comprises the four following FRs:
**V_{H}-FR1**: QVQLVQSGAEVKKPGASVKVSCKASGYTFT (SEQ ID NO: 16);
**V_{H}-FR2:** WVRQAPGQGLEWIG (SEQ ID NO: 17);
**V_{H}-FR3**: RVTLTADTSISTAYMELSRLRSDDTVVYYCVR (SEQ ID NO: 18);
**V_{H}-FR4:** WGQGTLVTVSS (SEQ ID NO: 19).

In one embodiment, the CD45RC-binding domain comprises a LCVR which comprises at least one, preferably at least two, more preferably at least three, even more preferably the four following FRs:
**VL-FR1:** DIQLTQSPSFLSASVGDRVTITC (SEQ ID NO: 20);
**VL-FR2:** WYQQKPGKAPKLWIY (SEQ ID NO: 21);
**VL-FR3**: GVPSRFSGSGSGTE-X-TLTISSLQPEDFATYYC (SEQ ID NO: 22);
**VL-FR4:** GGGTKVEIK (SEQ ID NO: 23)
with X being selected from the group comprising or consisting of Tyr (Y) and Phe (F), preferably with X being Tyr (Y).

In one embodiment, the CD45RC-binding domain comprises a LCVR which comprises the four following FRs:
**VL-FR1**: DIQLTQSPSFLSASVGDRVTITC (SEQ ID NO: 20);
**VL-FR2:** WYQQKPGKAPKLWIY (SEQ ID NO: 21);
**VL-FR3:** GVPSRFSGSGSGTE-X-TLTISSLQPEDFATYYC (SEQ ID NO: 22);
**VL-FR4**: GGGTKVEIK (SEQ ID NO: 23).
with X being selected from the group comprising or consisting of Tyr (Y) and Phe (F), preferably with X being Tyr (Y).

In one embodiment, the CD45RC-binding domain comprises a HCVR comprising or consisting of the amino acid sequence of SEQ ID NO: 24.

In one embodiment, the CD45RC-binding domain comprises a HCVR comprising the three CDRs with SEQ ID NOs: 10, 11 and 12, and framework regions sharing at least 70%, 75%, 80%, 85%, 90%, 95%, 96%, 97%, 98%, 99% or more sequence identity with the framework regions of SEQ ID NO: 24.

| |
|---|
| SEQ ID NO: 24 QVQLVQSGAEVKKPGASVKVSCKASGYTFTNYYIGWVRQAPGQGLEWIGDIFP GGDYANYNEKFQGRVTLTADTSISTAYMELSRLRSDDTVVYYCVRRNFDYWG QGTLVTVSS |

In one embodiment, the CD45RC-binding domain comprises a LCVR comprising or consisting of the amino acid sequence of SEQ ID NO: 25.

In one embodiment, the CD45RC-binding domain comprises a LCVR comprising the three CDRs with SEQ ID NOs: 13, 14 and 15, and framework regions sharing at least 70%, 75%, 80%, 85%, 90%, 95%, 96%, 97%, 98%, 99% or more sequence identity with the framework regions of SEQ ID NO: 25, with:
- X₁ being absent or being selected from the group comprising or consisting of Asn (N), Ser (S) and Gly (G), preferably with X₁ being absent; and
- X₂ being selected from the group comprising or consisting of Tyr (Y) and Phe (F), preferably with X₂ being Tyr (Y).

| |
|---|
| SEQ ID NO: 25 DIQLTQSPSFLSASVGDRVTITCRASSSVS-X1- YMHWYQQKPGKAPKLWIYNTANLPSGVPSRFSGSGSGTE-X2- TLTISSLQPEDFATYYCQQRSSYPLTFGGGTKVEIK |

In one embodiment, X₁ is absent and X₂ is Tyr (Y).

In one embodiment, the CD45RC-binding domain comprises:
- a HCVR comprising or consisting of the amino acid sequence of SEQ ID NO: 24; and
- a LCVR comprising or consisting of the amino acid sequence of SEQ ID NO: 25.

In one embodiment, the CD45RC-binding domain comprises:
- a HCVR comprising the three CDRs with SEQ ID NOs: 10, 11 and 12, and framework regions sharing at least 70%, 75%, 80%, 85%, 90%, 95%, 96%, 97%, 98%, 99% or more sequence identity with the framework regions of SEQ ID NO: 24; and
- a LCVR comprising the three CDRs with SEQ ID NOs: 13, 14 and 15, and framework regions sharing at least 70%, 75%, 80%, 85%, 90%, 95%, 96%, 97%, 98%, 99% or more sequence identity with the framework regions of SEQ ID NO: 25.

In one embodiment, the HCVR and/or LCVR as defined hereinabove can be characterized as having an amino acid sequence that shares at least 70%, 75%, 80%, 85%, 90%, 95%, 96%, 97%, 98%, 99% or more of identity with the particular HCVR and/or LCVR as defined hereinabove, while the CD45RC-binding domain retains its overall functional properties, in particular its binding specificity to CD45RC, preferably to hCD45RC.

In one embodiment, the sequence of the framework regions of the HCVR and/or LCVR as defined hereinabove can be characterized as having an amino acid sequence that shares at least 70%, 75%, 80%, 85%, 90%, 95%, 96%, 97%, 98%, 99% or more of identity with the sequence of the framework regions of the particular HCVR and/or LCVR as defined hereinabove, while the CD45RC-binding domain retains its overall functional properties, in particular its binding specificity to CD45RC, preferably to hCD45RC.

In one embodiment, the CD45RC-binding domain is conjugated, optionally through a linker, to a payload.

Examples of suitable payloads include, but are not limited to, peptides, polypeptides, proteins, polymers, nucleic acid molecules, small molecules, mimetic agents, synthetic drugs, inorganic molecules, organic molecules and radioisotopes.

Alternatively or additionally, examples of suitable payloads include, but are not limited to, chemotherapeutic agents, targeted therapy agents, cytotoxic agents (or cytotoxins), cell cycle-synchronizing agents, ligands for cellular receptor(s), immunomodulatory agents, pro-apoptotic agents, lytic peptides, anti-angiogenic agents, cytokines, growth factors, hormones, coding or non-coding oligonucleotides, photodetectable labels, contrast agents, radiolabels, and the like.

It will be apparent that some payloads may fall into more than one category.

In one embodiment, the payload is a chemotherapeutic agent. As used herein, the term **"chemotherapeutic agent"** refers to any molecule that is effective in inhibiting tumor growth.

Suitable examples of chemotherapeutic agents include those described under subgroup L01 of the Anatomical Therapeutic Chemical Classification System.

Suitable examples of chemotherapeutic agents include, but are not limited to:
- alkylating agents, such as, *e.g*.:
   ▪ nitrogen mustards, including chlormethine, cyclophosphamide, ifosfamide, trofosfamide, chlorambucil, melphalan, prednimustine, bendamustine, uramustine, chlornaphazine, cholophosphamide, estrarnustine, mechlorethamine, mechlorethamine oxide hydrochloride, novembichin, phenesterine, uracil mustard and the like;
   ▪ nitrosoureas, including carmustine, lomustine, semustine, fotemustine, nimustine, ranimustine, streptozocin, chlorozotocin, and the like;
   ▪ alkyl sulfonates, including busulfan, mannosulfan, treosulfan, and the like;
   ▪ aziridines, including carboquone, thiotepa, triaziquone, triethylenemelamine, benzodopa, meturedopa, uredopa, and the like; hydrazines, including procarbazine, and the like;
   ▪ triazenes, including dacarbazine, temozolomide, and the like; ethylenimines and methylamelamines, including altretamine, triethylenemelamine, trietylenephosphoramide, triethylenethiophosphaorarnide, trimethylolomelamine and the like;
   ▪ and others, including mitobronitol, pipobroman, actinomycin, bleomycin, mitomycins (including mitomycin C, and the like), plicamycin, and the like;
- acetogenins, such as, *e.g*., bullatacin, bullatacinone, and the like;
- benzodiazepines, such as, *e.g*., 2-oxoquazepam, 3-hydroxyphenazepam, bromazepam, camazepam, carburazepam, chlordiazepoxide, cinazepam, cinolazepam, clonazepam, cloniprazepam, clorazepate, cyprazepam, delorazepam, demoxepam, desmethylflunitrazepam, devazepide, diazepam, diclazepam, difludiazepam, doxefazepam, elfazepam, ethyl carfluzepate, ethyl dirazepate, ethyl loflazepate, flubromazepam, fletazepam, fludiazepam, flunitrazepam, flurazepam, flutemazepam, flutoprazepam, fosazepam, gidazepam, halazepam, iclazepam, irazepine, kenazepine, ketazolam, lorazepam, lormetazepam, lufuradom, meclonazepam, medazepam, menitrazepam, metaclazepam, motrazepam, N-desalkylflurazepam, nifoxipam, nimetazepam, nitemazepam, nitrazepam, nitrazepate, nordazepam, nortetrazepam, oxazepam, phenazepam, pinazepam, pivoxazepam, prazepam, proflazepam, quazepam, QH-II-66, reclazepam, RO4491533, Ro5-4864, SH-I-048A, sulazepam, temazepam, tetrazepam, tifluadom, tolufazepam, triflunordazepam, tuclazepam, uldazepam, arfendazam, clobazam, CP-1414S, lofendazam, triflubazam, girisopam, GYKI-52466, GYKI-52895, nerisopam, talampanel, tofisopam, adinazolam, alprazolam, bromazolam, clonazolam, estazolam, flualprazolam, flubromazolam, flunitrazolam, nitrazolam, pyrazolam, triazolam, bretazenil, climazolam, EVT-201, FG-8205, flumazenil, GL-II-73, imidazenil, ¹²³I-iomazenil, L-655,708, loprazolam, midazolam, PWZ-029, remimazolam, Ro15-4513, Ro48-6791, Ro48-8684, Ro4938581, sarmazenil, SH-053-R-CH3-2'F, cloxazolam, flutazolam, haloxazolam, mexazolam, oxazolam, bentazepam, clotiazepam, brotizolam, ciclotizolam, deschloroetizolam, etizolam, fluclotizolam, israpafant, JQ1, metizolam, olanzapine, telenzepine, lopirazepam, zapizolam, razobazam, ripazepam, zolazepam, zomebazam, zometapine, premazepam, clazolam, anthramycin, avizafone, rilmazafone, and the like;
- antimetabolites, such as, *e.g*.:
   ▪ antifolates, including aminopterin, methotrexate, pemetrexed, pralatrexate, pteropterin, raltitrexed, denopterin, trimetrexate, pemetrexed, and the like;
   ▪ purine analogues, including pentostatin, cladribine, clofarabine, fludarabine, nelarabine, tioguanine, mercaptopurine, and the like;
   ▪ pyrimidine analogues, including fluorouracil, capecitabine, doxifluridine, tegafur, tegafur/gimeracil/oteracil, carmofur, floxuridine, cytarabine, gemcitabine, azacytidine, decitabine, and the like; and
   ▪ hydroxycarbamide;
- androgens, such as, *e.g*., calusterone, dromostanolone propionate, epitiostanol, mepitiostane, testolactone, and the like;
- anti-adrenals, such as, *e.g*., aminoglutethimide, mitotane, trilostane, and the like;
- folic acid replenishers, such as, *e.g*., frolinic acid, and the like;
- maytansinoids, such as, *e.g*., maytansine, ansamitocins, and the like;
- platinum analogs, such as, *e.g*., platinum, carboplatin, cisplatin, dicycloplatin, nedaplatin, oxaliplatin, satraplatin, and the like;
- antihormonal agents, such as, *e.g*.:
   ▪ anti-estrogens, including tamoxifen, raloxifene, aromatase inhibiting 4(5)-imidazoles, 4-hydroxytamoxifen, trioxifene, keoxifene, LY117018, onapristone, toremifene, and the like;
   ▪ anti-androgens, including flutamide, nilutamide, bicalutamide, leuprolide, goserelin, and the like;
- trichothecenes, such as, *e.g.*, T-2 toxin, verracurin A, roridinA, anguidine and the like;
- toxoids, such as, *e.g*., cabazitaxel, docetaxel, larotaxel, ortataxel, paclitaxel, tesetaxel, and the like;
- topoisomerase I inhibitors, such as, *e.g*., belotecan, camptothecin, cositecan, etirinotecan pegol, exatecan, gimatecan, irinotecan, lurtotecan, rubitecan, silatecan, topotecan, and the like;
- topoisomerase II inhibitors, such as, *e.g*., etoposide, teniposide, and the like;
- others, such as, *e.g*., camptothecin (including the synthetic analogue topotecan); bryostatin; callystatin; CC-1065 (including its adozelesin, carzelesin and bizelesin synthetic analogues); cryptophycins (including cryptophycin 1 and cryptophycin 8); dolastatin; duocarmycin (including its synthetic analogues KW-2189 and CBI-TMI); eleutherobin; pancratistatin; sarcodictyin; spongistatin; aclacinomysins; authramycin; azaserine; bleomycin; cactinomycin; carabicin; canninomycin; carzinophilin; chromomycins; dactinomycin; daunorubicin; detorubicin; 6-diazo-5-oxo-L-norleucine; doxorubicin (including morpholino-doxorubicin, cyanomorpholino-doxorubicin, 2-pyrrolino-doxorubicin, deoxydoxorubicin, and the like); epirubicin; esorubicin; idanrbicin; marcellomycin; mycophenolic acid; nogalarnycin; olivomycins; peplomycin; potfiromycin; puromycin; quelamycin; rodorubicin; streptomgrin; streptozocin; tubercidin; ubenimex; zinostatin; zorubicin; aceglatone; aldophospharnide glycoside; aminolevulinic acid; amsacrine; bestrabucil; bisantrene; edatraxate; defofamine; demecolcine; diaziquone; elfornithine; elliptinium acetate; epothilone; etoglucid; gallium nitrate; hydroxyurea; lentinan; lonidamine; mitoguazone; mitoxantrone; mopidamol; nitracrine; phenamet; pirarubicin; podophyllinic acid; 2-ethylhydrazide; PSK^{®}; razoxane; rhizoxin; sizofiran; spirogennanium; tenuazonic acid; 2,2',2"-trichlorotriethylarnine; urethan; vindesine; dacarbazine; mannomustine; mitobromtol; mitolactol; pipobroman; gacytosine; arabinoside; 6-thioguanine; vinblastine; etoposide; vincristine; vinorelbine; navelbine; novantrone; teniposide; daunomycin; xeloda; ibandronate; CPT-11; topoisomerase inhibitor RFS 2000; topoisomerase I inhibitor SN38; difluoromethylornithine; retinoic acid; and the like.

In one embodiment, the payload is a targeted therapy agent.

As used herein, the term **"targeted therapy agent"** refers to any molecule which aims at one or more particular target molecules (such as, e.g., proteins) involved in tumor genesis, tumor progression, tumor metastasis, tumor cell proliferation, cell repair, and the like.

Suitable examples of targeted therapy agents include, but are not limited to, tyrosine-kinase inhibitors, serine/threonine kinase inhibitors, monoclonal antibodies and the like.

Suitable examples of targeted therapy agents include, but are not limited to, HER1/EGFR inhibitors (such as, *e.g*., brigatinib, erlotinib, gefitinib, olmutinib, osimertinib, rociletinib, vandetanib, and the like); HER2/neu inhibitors (such as, *e.g.*, afatinib, lapatinib, neratinib, and the like); C-kit and PDGFR inhibitors (such as, *e.g.*, axitinib, masitinib, pazopanib, sunitinib, sorafenib, toceranib, and the like); FLT3 inhibitors (such as, *e.g.*, lestaurtinib, and the like); VEGFR inhibitors (such as, *e.g.*, axitinib, cediranib, lenvatinib, nintedanib, pazopanib, regorafenib, semaxanib, sorafenib, sunitinib, tivozanib, toceranib, vandetanib, and the like); RET inhibitors (such as, *e.g.*, vandetanib, entrectinib, and the like); c-MET inhibitors (such as, *e.g*., cabozantinib, and the like); bcr-abl inhibitors (such as, *e.g*., imatinib, dasatinib, nilotinib, ponatinib, radotinib, and the like); Src inhibitors (such as, *e.g*., bosutinib, dasatinib, and the like); Janus kinase inhibitors (such as, *e.g*., lestaurtinib, momelotinib, ruxolitinib, pacritinib, and the like); MAP2K inhibitors (such as, *e.g*., cobimetinib, selumetinib, trametinib, binimetinib, and the like); EML4-ALK inhibitors (such as, *e.g*., alectinib, brigatinib, ceritinib, crizotinib, and the like); Bruton's inhibitors (such as, *e.g*., ibrutinib, and the like); mTOR inhibitors (such as, *e.g*., everolimus, temsirolimus, and the like); hedgehog inhibitors (such as, *e.g*., sonidegib, vismodegib, and the like); CDK inhibitors (such as, *e.g*., palbociclib, ribociclib, and the like); anti-HER1/EGFR monoclonal antibodies (such as, *e.g*., cetuximab, necitumumab, panitumumab, and the like); anti-HER2/neu monoclonal antibodies (such as, *e.g*., ado-trastuzumab emtansine, pertuzumab, trastuzumab, trastuzumab-dkst, and the like); anti-EpCAM monoclonal antibodies (such as, *e.g*., catumaxomab, edrecolomab, and the like); anti-VEGF monoclonal antibodies (such as, *e.g*., bevacizumab, bevacizumab-awwb, and the like); anti-CD20 monoclonal antibodies (such as, *e.g*., ibritumomab, obinutuzumab, ocrelizumab, ofatumumab, rituximab, tositumomab, and the like); anti-CD30 monoclonal antibodies (such as, *e.g.*, brentuximab, and the like); anti-CD33 monoclonal antibodies (such as, *e.g*., gemtuzumab, and the like); and anti-CD52 monoclonal antibodies (such as, *e*.*g*., alemtuzumab, and the like).

In one embodiment, the payload is a cytotoxic agent.

As used herein, the terms **"cytotoxic agent"** or **"cytotoxin"** refer to any molecule that results in cell death by any mechanism.

Suitable examples of cytotoxic agents include, but are not limited to, taxanes, anthracyclines, alkylating agents, vinca alkaloids, antimetabolites, platinum agents, steroids, chemotherapeutic agents and auristatins.

Suitable examples of taxanes include, but are not limited to, cabazitaxel, docetaxel, larotaxel, ortataxel, paclitaxel and tesetaxel.

Suitable examples of anthracyclines include, but are not limited to, aclarubicin, amrubicin, daunorubicin, doxorubicin, epirubicin, idarubicin, pirarubicin, valrubicin and zorubicin.

Suitable examples of alkylating agent include, but are not limited to, nitrogen mustards (such as, *e.g*., chlormethine, cyclophosphamide, ifosfamide, trofosfamide, chlorambucil, melphalan, prednimustine, bendamustine, uramustine, and the like), nitrosoureas (such as, *e.g*., carmustine, lomustine, semustine, fotemustine, nimustine, ranimustine, streptozocin, and the like), alkyl sulfonates (such as, *e.g*., busulfan, mannosulfan, treosulfan, and the like), aziridines (such as, *e.g*., carboquone, thiotepa, triaziquone, triethylenemelamine, benzodopa, meturedopa, uredopa, and the like), hydrazines (such as, *e.g.*, procarbazine, and the like), triazenes (such as, *e.g.*, dacarbazine, temozolomide, and the like), altretamine, mitobronitol, pipobroman, actinomycin, bleomycin, mitomycins and plicamycin.

Suitable examples of vinca alkaloids include, but are not limited to, vinblastine, vincristine, vinflunine, vindesine and vinorelbine.

Suitable examples of antimetabolites include, but are not limited to, antifolates (such as, aminopterin, methotrexate, pemetrexed, pralatrexate, raltitrexed, pemetrexed, and the like), purine analogues (such as, *e.g*., pentostatin, cladribine, clofarabine, fludarabine, nelarabine, tioguanine, mercaptopurine, and the like), pyrimidine analogues (such as, *e.g.*, fluorouracil, capecitabine, doxifluridine, tegafur, tegafur/gimeracil/oteracil, carmofur, floxuridine, cytarabine, gemcitabine, azacytidine, decitabine, and the like), and hydroxycarbamide.

Suitable examples of platinum agents include, but are not limited to, carboplatin, cisplatin, dicycloplatin, nedaplatin, oxaliplatin and satraplatin.

Suitable examples of steroids include, but are not limited to, estrogen receptor modulators, androgen receptor modulators and progesterone receptor modulators.

Suitable examples of chemotherapeutic agents have been described above.

Suitable examples of auristatins include, but are not limited to, **auristatin E, auristatin F, monomethyl auristatin E, and monomethyl auristatin F.**

In one embodiment, the payload is a cell cycle-synchronizing agent.

As used herein, the term **"cell cycle-synchronizing agent"** refers to any molecule able to unify the cell cycle of a population of cells to the same phase upon administration.

Suitable examples of cell cycle-synchronizing agents include, but are not limited to, aphidicolin, **butyrolactone I,** colchicine, **cycloheximide,** demecolcine, **dimethyl sulfoxide,** 5-fluorodeoxyuridine, **Hoechst 33342, mimosine,** nocodazole, **roscovitine, and** thymidine.

In one embodiment, the payload is a ligand for a cellular receptor.

As used herein, the term **"ligand for a cellular receptor"** refers to any molecule binding to a cellular receptor (such as a cell surface receptor, an intracellular receptor or a co-receptor, including transcription factors and the like), including agonists and antagonists, as well as partial agonists, inverse agonists, and allosteric modulators.

Suitable examples of ligands for cellular receptors include, but are not limited to, ligands binding to the AATYK receptors, the acetylcholine receptors, the ADGRG receptors, the adiponectin receptors, the adrenergic α1 receptors, the adrenergic α2 receptors, the adrenergic β1 receptors, the adrenergic β2 receptors, the adrenergic β3 receptors, the adrenomedullin receptor, the AMPA receptors, the anaphylatoxin receptors, the angiopoietin receptors, the angiotensin receptors, the anti-Müllerian hormone receptor, the apelin receptor, the asialoglycoprotein receptors, the AXL receptors, the benzodiazepine receptor, the bile acid receptor, the bombesin receptors, the bone morphogenetic protein receptors, the bradykinin receptors, the brain-specific angiogenesis inhibitors, the cadherin receptors, the calcitonin receptor, the calcitonin receptor-like receptor, the calcium-sensing receptor, the cannabinoid receptors, the CD97 receptor, the chemokine receptors, the cholecystokinin receptors, the complement receptors, the corticotropin-releasing hormone receptors, the CysLT receptors, the cytokine receptors, the DDR receptors, the dopamine receptors, the EBI2 receptor, the ectodysplasin A receptor, the EGF module-containing mucin-like hormone receptors, the EGF receptors, the endothelin receptors, the EPH receptors, the estrogen receptor, the FGF receptors, the free fatty acid receptors, the frizzled receptors, the FSH receptor, the GABAB receptors, the galanin receptors, the GHB receptor, the ghrelin receptor, the glucagon receptors, the glucagon-like peptide receptors, the glutamate receptors, the glycine receptors, the gonadotropin receptors, the gonadotropin-releasing hormone receptors, the GPRC6A receptor, the growth factor receptors, the growth hormone receptors, the growth-hormone-releasing hormone receptor, the guanylate cyclase-coupled receptors, the HGF receptors, the histamine receptors, the hydroxycarboxylic acids receptors, the immunoglobulin immune receptors, the insulin receptors, the kainate receptors, the KiSSl-derived peptide receptor, the latrophilin receptors, the leptin receptor, the leukotriene B4 receptors, the lipoprotein receptor-related protein receptors, the LTK receptors, the luteinizing hormone/choriogonadotropin receptor, the lysophosphatidic acid receptors, the lysophospholipid receptors, the mannose receptor, the MAS receptors, the melanin-concentrating hormone receptors, the melanocortin receptors, the melatonin receptors, the methuselah-like proteins receptors, the motilin receptor, the MuSK receptors, the N-acetylglucosamine receptor, the neuromedin receptors, the neuropeptide B/W receptors, the neuropeptide FF receptors, the neuropeptide S receptor, the neuropeptide Y receptors, the neuropilins receptor, the neurotensin receptors, the N-formyl peptide receptor, the nicotinic acetylcholine receptors, the NMDA receptors, the nuclear receptors, the olfactory receptor, the opioid receptors, the opsin receptors, the orexin receptors, the oxoeicosanoid receptor, the oxoglutarate receptor, the oxytocin receptor, the parathyroid hormone receptors, the PDGF receptors, the pituitary adenylate cyclase-activating polypeptide type I receptor, the platelet-activating factor receptor, the progestin and adipoQ receptors, the prokineticin receptors, the prolactin receptor, the prolactin-releasing peptide receptor, the prostacyclin receptor, the prostaglandin receptors, the protease-activated receptor, the PTK7 receptors, the purinergic adenosine receptors, the purinergic P2X receptors, the purinergic P2Y receptors, the relaxin receptors, the RET receptors, the retinoic acid-inducible orphan G-protein-coupled receptors, the ROR receptors, the ROS receptors, the RYK receptors, the scavenger receptors, the secretin receptor, the serine/threonine-specific protein kinase receptors, the serotonine receptors, the smoothened receptor, the somatostatin receptors, the sphingosine-1-phosphate receptors, the SREB receptors, the stimulator of interferon genes (STING) receptor, the succinate receptor, the tachykinin receptors, the thromboxane receptor, the thyrotropin receptor, the thyrotropin-releasing hormone receptor, the toll-like receptors, the trace-amine associated receptors, the transferrin receptor, the Trk receptors, the tumor necrosis factor receptors, the tyrosine phosphatase receptors, the urotensin-II receptor, the vasoactive intestinal peptide receptors, the vasoactive intestine peptide receptors, the vasopressin receptors, the VEGF receptors, the vomeronasal receptor, and the zinc-activated ion channel receptor.

In one embodiment, the payload is an immunomodulatory agent.

Suitable examples of immunomodulatory agents include, but are not limited to, immunostimulatory agents and immunosuppressor agents.

Suitable examples of immunostimulatory agents include those described under subgroup L03 of the Anatomical Therapeutic Chemical Classification System.

Suitable examples of immunostimulatory agents include, but are not limited to, cytokines (such as, *e.g*., filgrastim, pegfilgrastim, lenograstim, molgramostim, sargramostim, ancestim, albinterferon, interferon α natural, interferon α 2a, peginterferon α 2a, interferon α 2b, peginterferon α -2b, interferon α nl, interferon alfacon-1, interferon α-n3, interferon β natural, interferon β la, interferon β 1b, interferon γ, aldesleukin, oprelvekin, and the like); immune checkpoint inhibitors (such as, e.g., inhibitors of CTLA4, PD-1, PD-L1, LAG-3, B7-H3, B7-H4, TIM3, A2AR, and/or IDO, including nivolumab, pembrolizumab, pidilizumab, AMP-224, MPDL3280A, MDX-1105, MEDI-4736, arelumab, ipilimumab, tremelimumab, pidilizumab, IMP321, MGA271, BMS-986016, lirilumab, urelumab, PF-05082566, IPH2101, MEDI-6469, CP-870,893, mogamulizumab, varlilumab, avelumab, galiximab, AMP-514, AUNP 12, indoximod, NLG-919, INCB024360, and the like); toll-like receptor agonists (such as, *e.g.*, buprenorphine, carbamazepine, ethanol, fentanyl, GS-9620, imiqimod, lefitolimod, levorphanol, methadone, morphine, (+)-morphine, morphine-3-glucuronide, oxcarbazepine, oxycodone, pethidine, resiquimod, SD-101, tapentadol, tilsotolimod, VTX-2337, glucuronoxylomannan from *Cryptococcus,* MALP-2 from *Mycoplasma,* MALP-404 from *Mycoplasma,* OspA from *Borrelia,* porin from *Neisseria* or *Haemophilus,* hsp60, hemmaglutinin, LcrV from *Yersinia,* bacterial flagellin, lipopolysaccharide, lipoteichoic acid, lipomannan from *Mycobacterium,* glycosylphosphatidylinositol, lysophosphatidylserine, lipophosphoglycan from *Leishmania,* zymosan from *Saccharomyces,* Pam2CGDPKHPKSF, Pam3CSK4, CpG oligodeoxynucleotides, poly(I:C) nucleic acid sequences, poly(A:U) nucleic acid sequences, double-stranded viral RNA, and the like); STING receptor agonists (such as, *e.g.*, those described in WO2017100305, vadimezan, CL656, ADU-S100, 3'3'-cGAMP, 2'3'-cGAMP, ML RR-S2 CDG, ML RR-S2 cGAMP, cyclic di-GMP, DMXAA, DiABZI, and the like); CD1 ligands; growth hormone; immunocyanin; pegademase; prolactin; tasonermin; female sex steroids; histamine dihydrochloride; poly ICLC; vitamin D; lentinan; plerixafor; roquinimex; mifamurtide; glatiramer acetate; thymopentin; thymosin α1; thymulin; polyinosinic:polycytidylic acid; pidotimod; Bacillus Calmette-Guérin; melanoma vaccine; sipuleucel-T; and the like.

Suitable examples of immunosuppressor agents include those described under subgroup L04 of the Anatomical Therapeutic Chemical Classification System.

Suitable examples of immunosuppressor agents include, but are not limited to:
- antimetabolites, such as, *e.g*.:
   ▪ antifolates, including aminopterin, methotrexate, pemetrexed, pralatrexate, pteropterin, raltitrexed, denopterin, trimetrexate, pemetrexed, and the like;
   ▪ purine analogues, including pentostatin, cladribine, clofarabine, fludarabine, nelarabine, tioguanine, mercaptopurine, and the like;
   ▪ pyrimidine analogues, including fluorouracil, capecitabine, doxifluridine, tegafur, tegafur/gimeracil/oteracil, carmofur, floxuridine, cytarabine, gemcitabine, azacytidine, decitabine, and the like; and
   ▪ hydroxycarbamide;
- macrolides, such as, *e.g.*, tacrolimus, ciclosporin, pimecrolimus, abetimus, gusperimus, and the like;
- immunomodulatory imide drugs, such as, *e.g.,* lenalidomide, pomalidomide, thalidomide, apremilast, and the like;
- IL-1 receptor antagonists, such as, *e.g*., anakinra, and the like;
- mTOR inhibitors, such as, *e.g.,* sirolimus, everolimus, ridaforolimus, temsirolimus, umirolimus, zotarolimus, and the like;
- serum-targeting antibodies, such as, *e.g*., eculizumab, adalimumab, afelimomab, certolizumab pegol, golimumab, infliximab, nerelimomab, mepolizumab, omalizumab, faralimomab, elsilimomab, lebrikizumab, ustekinumab, secukinumab, and the like;
- cell-targeting antibodies, such as, *e.g*., muromonab-CD3, otelixizumab, teplizumab, visilizumab, clenoliximab, keliximab, zanolimumab, efalizumab, erlizumab, obinutuzumab, rituximab, ocrelizumab, pascolizumab, gomiliximab, lumiliximab, teneliximab, toralizumab, aselizumab, galiximab, gavilimomab, ruplizumab, belimumab, blisibimod, ipilimumab, tremelimumab, bertilimumab, lerdelimumab, metelimumab, natalizumab, tocilizumab, odulimomab, basiliximab, daclizumab, inolimomab, zolimomab aritox, atorolimumab, cedelizumab, fontolizumab, maslimomab, morolimumab, pexelizumab, reslizumab, rovelizumab, siplizumab, talizumab, telimomab aritox, vapaliximab, vepalimomab, and the like;
- fusion antibodies, such as, *e.g*., abatacept, belatacept, etanercept, pegsunercept, aflibercept, alefacept, rilonacept and the like.

In one embodiment, the payload is a pro-apoptotic agent.

As used herein, the term **"pro-apoptotic agent"** refers to any molecule able to induce apoptosis or programmed cell death in a cell upon administration.

Suitable examples of pro-apoptotic agents include, but are not limited to, histone deacetylase inhibitors (such as, *e.g*., sodium butyrate, depsipeptide and the like), bortezomib, deguelin, favopiridol, fenretinide, fludarabine, kaempferol, miltefosine, narciclasine, obatoclax, oblimersen, and oncrasin.

In one embodiment, the payload is a lytic peptide.

As used herein, the term **"lytic peptide"** refers to a peptide that has the ability to lyse or pierce membranes.

Examples of lytic peptides include, without limitation, LL-37, α-defensins, β-defensins, θ-defensins, dermcidin, histatin, perforin, cecropins, dermaseptin, magainon, melittin, nisin, pneumolysin, and toxins.

Examples of toxins include, without limitation, bacterial toxins (such as, *e.g.*, *Clostidium tetani* tetanus toxin, *Clostidium tetani* tetanolysin, *Clostidium perfringens* alpha toxin, *Clostidium perfringens* enterotoxin, *Clostidium difficile* toxin A, *Clostidium difficile* toxin B, *Clostidium botulinum* toxin, *Clostridium botulinum* C3 exoenzyme, anthrax toxin, listeriolysin O, *Staphylococcus aureus* alpha toxin, *Staphylococcus aureus* beta toxin, *Staphylococcus aureus* delta toxin, *Staphylococcus aureus* clumping factor A, *Staphylococcus aureus* fibronectin binding protein A, exfoliatin, toxic shock syndrome toxin, enterotoxin type B, cord factor, diphtheria toxin, streptolysin, leucocidin, shiga toxin, Shiga-like toxin, heat-stable enterotoxin, cholera toxin, pertussis toxin, *Pseudomonas* exotoxin, extracellular adenylate cyclase, *Bacillus thuringiensis* delta endotoxin), mycotoxins (such as, *e.g.,* aflatoxin, alpha-amanitin, beta-amanitin, gamma-amanitin, epsilon-amanitin, β-nitropropionic acid, citrinin, cytochalasin, ergotamine, fumonisin B1, fumonisin B2, fumonisin B3, fumonisin B4, gliotoxin, ibotenic acid, lolitrem B, muscimol, ochratoxin, patulin, phalloidin, sterigmatocystin, trichothecene, vomitoxin, zeranol, zearalenone), plant toxins (such as, e.g., amygdalin, anisatin, antiarin, brucine, chaconine, cicutoxin, daphnin, delphinine, divicine, djenkolic acid, falcarinol, gossypol, helenalin, ledol, linamarin, lotaustralin, mimosine, oenanthotoxin, oleandrin, persin, protoanemonin, pseudaconitine, retronecine, resiniferatoxin; scopolamine, solamargine, solanidine, solanine, solasodamine, solasodine, solasonine, solauricidine, solauricine, strychnine, swainsonine, tagetitoxin, tinyatoxin, tomatine, abrin, ricin, tutin), invertebrate toxins (such as, *e.g.*, *Androctonus australis hector* insect toxin, charybdotoxin, maurotoxin, agitoxin, margatoxin, slotoxin, scyllatoxin, hefutoxin, HgeTx1, HsTx1, Lq2, birtoxin, bestoxin, BmKAEP, phaiodotoxin, imperatoxin, Pi3, latrotoxin, CSTX, cupiennins, PhTx3, stromatoxin, vanillotoxin, huwentoxin, conotoxin, eledoisin, onchidal, saxitoxin, tetrodotoxin), and vertebrate toxins (such as, e.g., ciguatera, tetrodotoxin, (+)-allopumiliotoxin 267A, batrachotoxin, arenobufagin, bufotalin, bufotenine, cinobufagin, marinobufagin, epibatidine, histrionicotoxin, pumiliotoxin 251D, samandarin, samandaridine, tarichatoxin, zetekitoxin AB, alpha-bungarotoxin, beta-bungarotoxin, calciseptine, taicatoxin, calcicludine, cardiotoxin III).

In one embodiment, the payload is an anti-angiogenic agent.

As used herein, the term **"anti-angiogenic agent"** refers to a molecule that reduces or prevents angiogenesis, which is responsible for the growth and development of blood vessels.

Suitable examples of anti-angiogenic agents include, but are not limited to, inhibitors of any of the vascular endothelial growth factor VEGF-A, VEGF-B, VEGF-C, or VEGF-D, which are major inducers of angiogenesis in normal and pathological conditions, and are essential in embryonic vasculogenesis.

Additionally or alternatively, an anti-angiogenic agent also can inhibit other angiogenic factors, such as, without limitation, a member of the fibroblast growth factor (FGF) family such as FGF-1 (acidic), FGF-2 (basic), FGF-4 or FGF-5; or angiopoietin-1, a factor that signals through the endothelial cell-specific Tie2 receptor tyrosine kinase; or the receptor of any of these angiogenic factors.

In one embodiment, the payload is a cytokine.

Suitable examples of cytokines include, but are not limited to, chemokines, tumor necrosis factors, interleukins, and colony-stimulating factors.

Suitable examples of chemokines include, but are not limited to, chemokine C-C motif ligand (CCL) 1, CCL2, CCL3, CCL4, CCL5, CCL6, CCL7, CCL8, CCL9, CCL11, CCL12, CCL13, CCL14, CCL15, CCL16, CCL17, CCL18, CCL19, CCL20, CCL21, CCL22, CCL23, CCL24, CCL25, CCL26, CCL27, CCL28, chemokine C-X-C motif ligand (CXCL) 1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCL17, fractalkine, chemokine C motif ligand (XCL) 1, and XCL2.

Suitable examples of tumor necrosis factors include, but are not limited to, tumor necrosis factor (TNF) α, lymphotoxin, OX40L, CD40LG, Fas ligand, CD70, CD153, 4-1BB ligand, TNF-related apoptosis-inducing ligand (TRAIL), receptor activator of nuclear factor κ-B ligand (RANKL), a proliferation-inducing ligand (APRIL), B-cell activating factor (BAFF), and ectodysplasin A (EDA).

Suitable examples of interleukins include, but are not limited to, interleukin- (IL- ) 1α, IL-1β, IL-1Ra, IL-2, IL-3, IL-4, IL-5, IL-6, IL-7, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IL-36α, IL-36β, IL-36γ, IL-36Ra, IL-37, IL-38, interferon (IFN) α, IFNβ, IFNκ, and IFNω.

Suitable examples of colony-stimulating factors include, but are not limited to, granulocyte-macrophage colony-stimulating factor (GM-CSF) (including granulocyte-colony stimulating factor (G-CSF) and macrophage colony-stimulating factor (M-CSF)), haematopoietin, and thrombopoietin.

In one embodiment, the payload is a growth factor.

Suitable examples of growth factors include, but are not limited to, fibroblast growth factor (FGF) 1, FGF2, FGF3, FGF4, FGF5, FGF6, FGF7, FGF8, FGF9, FGF10, FGF11, FGF12, FGF13, FGF14, FGF16, FGF17, FGF18, FGF19, FGF20, FGF21, FGF23, transforming growth factor (TGF) α, epidermal growth factor (EGF), heparin-binding EGF-like growth factor (HB-EGF), transforming growth factor (TGF) β, insulin-like growth factor (IGF) 1, IGF2, Platelet-derived growth factor (PDGF) subunit A (PDGFA), PDGF subunit B (PDGFB), PDGF subunit C (PDGFC), PDGF subunit D (PDGFD), vascular endothelial growth factor (VEGF)-A, VEGF-B, VEGF-C, VEGF-D, placental growth factor (PGF), nerve growth factor (NGF), and hepatocyte growth factor (HGF).

In one embodiment, the payload is a hormone.

Suitable examples of hormones include, but are not limited to, GnRH, TRH, dopamine, CRH, GHRH, somatostatin, MCH, oxytocin, vasopressin, FSH, LH, TSH, prolactin, POMC, CLIP, ACTH, MSH, endorphins, lipotropin, GH, aldosterone, cortisol, cortisone, DHEA, DHEA-S, androstenedione, epinephrine, norepinephrine, thyroid hormone T3, thyroid hormone T4, calcitonin, PTH, testosterone, AMH, inhibin, estradiol, progesterone, activin, relaxin, GnSAF, hCG, HPL, estrogen, glucagon, insulin, amylin, pancreatic polypeptide, melatonin, N,N-dimethyltryptamine, 5-methoxy-N,N-dimethyltryptamine, thymosin α1, beta thymosins, thymopoietin, thymulin, gastrin, ghrelin, CCK, GIP, GLP-1, secretin, motilin, VIP, enteroglucagon, peptide YY, IGF-1, IGF-2, leptin, adiponectin, resistin, osteocalcin, renin, EPO, calcitriol, prostaglandin, ANP, and BNP.

In one embodiment, the payload is a coding or non-coding oligonucleotide.

Suitable examples of coding or non-coding oligonucleotides include, but are not limited to, messenger RNA (mRNA), antisense RNA (asRNA), small interfering RNA (siRNA), microRNA (miRNA), long non-coding RNA (lncRNA) (such as, *e.g*., transfer RNA [tRNA], ribosomal RNA [rRNA], and the like), small temporal RNA (stRNA), trans-acting siRNA, short hairpin RNA (shRNA), cis-natural antisense transcripts (NATs), CRISPR RNA, long noncoding RNA, piwi-interacting RNA (piRNA), repeatassociated siRNA (rasiRNA), RNA aptamers, ribozymes, and the like.

Further suitable examples of coding or non-coding oligonucleotides include, but are not limited to, recapuldencel-T, TriMix, BI-1361849, nusinersen, volanesorsen sodium, eteplirsen, ATL1105, ASM-8, inclisiran, patisiran, RXI-109, fitusiran, cemdisiran, QPI-1002, BMS-986263, PF-655, pegaptanib, avacincaptad pegol sodium, olaptesed pegol, emapticap pegol, SPC3649, bevasiranib, AGN-745, QPI-1007, TD101, SYL040012, SYL1001, Excellair, ALN-RSV01, CEQ508, siG12D LODER, TKM-ApoB, TKM-PLK1, ALN-VSP02, ALN-TTR01, Bcr-Abl siRNA, Atu027, 15NP, CALAA-01, FANG vaccine, iPsiRNA, Tat/Rev shRNA, ARC1779, ARC19499, AS1411 (AGRO001), Fovista, NOX-A12, NOX-E36, NOX-H94, NU172, RB006 plus RB007, ARC1905, as well as those described in Table 1 and Table 2 of Crooke et al., 2018 (Cell Metab. 27(4):714-739), herein incorporated by reference.

In one embodiment, the payload is a photodetectable label.

As used herein, the terms **"photodetectable label"** or **"fluorophore"** refer to a moiety that can re-emit light upon light excitation.

Suitable examples of photodetectable labels include, but are not limited to, Alexa Fluor^{®} dyes, BODIPY^{®} dyes, fluorescein, 5-carboxyfluorescein, 5-(4,6-dichlorotriazin-2-yl) aminofluorescein, 2'7'-dimethoxy-4'5'-dichloro-6-carboxyfluorescein, fluorescein isothiocyanate (FITC), QFITC, Oregon Green^{™} 488, Oregon Green^{®} 514, rhodamine and derivatives thereof (such as, *e.g.,* rhodamine green, rhodamine green-X, rhodamine red-X, X-rhodamine, 6-carboxy-X-rhodamine (ROX), 6-carboxyrhodamine (R6G), N,N,N',N'-tetramethyl-6-carboxyrhodamine (TAMRA), lissamine rhodamine B, rhodamine 123, rhodamine X isothiocyanate, sulforhodamine B, sulforhodamine 101 (Texas Red), tetramethyl rhodamine, tetramethyl rhodamine isothiocyanate (TRITC)), eosin, eosin isothiocyanate, erythrosine, erythrosine B, erythrosin isothiocyanate, Texas Red^{®}, Texas Red^{®-}X, naphthofluorescein, malachite green, malachite green isothiocyanate, coumarin derivatives, Pacific Orange, cascade blue, cascade yellow, dansyl chloride, dapoxyl dye, 1-dimethylamine-*N*(2-azido-ethyl)naphthalene-5-sulfonamide, 6-(6-amino-2-(2-azidoethyl)-1,3-dioxo-1*H*- benzo(*de*)-2(3*H*)isoquinoline, 6-(6-amino-2-(2-propinyl)-1,3-dioxo-1*H*-benzo(*de*)-2(3*H*)isoquinoline, 8-(4-azidoethyloxyphenyl)-2,6-diethyl-1,3,5,7-tetramethyl-4,4-difluoro-4-bora-3a,4a-diaza-s-indacene, 8-(4-propynyioxyphenyl)-2,6-diethyl-1,3,5,7-tetramethyl-4,4-difluoro-4-bora-3a,4a-diaza-s-indacene, 1-(3-azido-propoxy)-7-methylamino-phenoxazin-3-one, 1-(2-propynyl)-7-methylamino-phenoxazm-3-one, *N*-(5-(3-azidopropylamino)-9*H-*benzo(*a*)-phenoxa-2-in-9-ylidene)-*N*-methyl-methanaminium chloride, *N*-(5-(3-propynyl-amino)-9*H*-benzo(*a*)-phenoxazin-9-ylene)-*N*-methyl-methanaminium chloride, (9-(3-azido-propoxy)-7-piperidin-l-yl-phenoxazin-3-ylidene)-dimethylammonium perchlorate, 4-acetamido-4'-isothiocyanatostilbene-2,2'-disulfonic acid, acridine, acridine isothiocyanate, 5-(2'-aminoethyl)aminonaphthalene-1-sulfonic acid, 4-amino-*N*-[3-vinylsulfonyl)phenyl]naphthalimide-3,5 disulfonate, *N*-(4-anilino-1-naphthyl)maleimide, anthranilamide, Brilliant Yellow, coumarin, coumarin derivatives, 7-amino-4-methylcoumarin, 7-amino-trifluoromethylcouluarin, cyanosine, 4',6-diaminidino-2-phenylindole, 5',5"-dibromopyrogallol-sulfonephthalein, 7-diethylamino-3-(4'-isothiocyanatophenyl)-4-methylcoumarin-4,4'-diisothiocyanatodihydro-stilbene-2,2'-disulfonic acid, 4,4'-diisothiocyanatostilbene-2,2'-disulfonic acid, ethidium, IR144, IR1446, 4-methylumbelliferone, *o*-cresolphthalein, nitrotyrosine, pararosaniline, Phenol Red, B-phycoerythrin, o-phthaldialdehyde, pyrene, pyrene butyrate, succinimidyl 1-pyrene butyrate, Reactive Red 4, riboflavin, rosolic acid, lanthanide chelates, quantum dots, cyanines, pyrelium dyes, and squaraines.

In one embodiment, the payload is a contrast agent.

As used herein, the term **"contrast agent"** refers to any molecule used to increase the contrast of structures or fluids within the body in medical imaging. Contrast agents absorb or alter external electromagnetism or ultrasound (which differs from radiolabels which emit radiation themselves).

Suitable examples of radiolabels include those described under subgroup V08 of the Anatomical Therapeutic Chemical Classification System.

Suitable examples of contrast agents include, but are not limited to, diatrizoic acid, metrizoic acid, iodamide, iotalamic acid, ioxitalamic acid, ioglicic acid, acetrizoic acid, iocarmic acid, methiodal, diodone, metrizamide, iohexol, ioxaglic acid, iopamidol, iopromide, iotrolan, ioversol, iopentol, iodixanol, iomeprol, iobitridol, ioxilan, iodoxamic acid, iotroxic acid, ioglycamic acid, adipiodone, iobenzamic acid, iopanoic acid, iocetamic acid, sodium iopodate, tyropanoic acid, calcium iopodate, iopydol, propyliodone, iofendylate, lipiodol, barium sulfate, gadobenic acid, gadobutrol, gadodiamide, gadofosveset, gadolinium, gadopentetic acid, gadoteric acid, gadoteridol, gadoversetamide, gadoxetic acid, ferric ammonium citrate, mangafodipir, ferumoxsil, ferristene, perflubron, microspheres of human albumin, microparticles of galactose, perflenapent, microspheres of phospholipids, sulfur hexafluoride, and the like.

In one embodiment, the payload is a radiolabel.

As used herein, the terms **"radiolabel", "radiopharmaceutical"** or **"radioisotope"** refer to any molecule which emits radiation. Radiolabels can be used for therapeutics or diagnostic purposes.

Suitable examples of radiolabels include those described under subgroups V09 and V10 of the Anatomical Therapeutic Chemical Classification System.

Suitable examples of radiolabels include, but are not limited to, ^{99m}Tc compounds (such as, *e.g*., exametazime, medronic acid, macroaggregated albumin, sestamibi, tetrofosmin, exametazime, sulesomab, tilmanocept, arcitumomab, votumumab, hynicoctreotide, and the like); ¹²³I, ¹²⁵I or ¹³¹I compounds (such as, *e.g*., ioflupane, iofetamine, iomazenil, sodium iodohippurate, iobenguane, iodocholesterol, minretumomab, tositumomab, and the like); ¹⁸F compounds (such as, *e.g.*, florbetapir, flutemetamol, fluciclovine, fludeoxyglucose, fluoroethyltyrosine, sodium fluoride, and the like); ⁶⁴Cu compounds (such as, *e.g.,* Cu-ETS2, and the like); ⁷⁵Se compounds (such as, *e.g.,* SeHCAT); ¹¹¹In compounds (such as, *e.g*., imciromab, capromab pendetide, satumomab pendetide, and the like); ⁸²Rb compounds (such as, *e.g*., rubidium chloride); ¹⁵³Sm compounds (such as, *e.g.,* lexidronam, and the like); ⁸⁹Sr compounds (such as, *e.g.,* strontium-89 chloride, and the like); ⁹⁰Y compounds (such as, *e.g*., ibritumomab tiuxetan, and the like); ²²³Ra compounds (such as, *e.g*., radium-223 chloride, and the like); ¹⁷⁷Lu compounds (such as, *e.g*., oxodotreotide, and the like); and any compounds comprising at least one ²H, ³H, ¹¹C, ¹³N, ¹⁴C, ¹⁵O, ¹⁸F, ²²Na, ²⁴Na, ²P, ³³P, ⁴⁰K, ⁴⁷Ca, ⁵¹Cr, ⁵⁵Eu, ⁵⁷Co, ⁵⁸Co, ⁶⁰Co, ⁵⁹Fe, ⁶⁴Cu, ⁶⁷Ga, ⁶⁸Ga, ⁷⁵Se, ⁸¹mKr, ⁸²Rb, ⁸⁹Sr, ⁹⁰Sr, ⁹⁰Y, ⁹⁷Tc, ^{99m}Tc, ¹⁰³Pd, ¹⁰⁶Ru, ¹⁰⁷Pd, ¹¹¹In, ¹¹³Cd, ¹²¹Sn, ¹²³I, ¹²⁴I, ¹²⁵I, ¹²⁶Sn, ¹²⁹I, ¹³¹I, ¹³³Xe, ¹³⁵Cs, ¹³⁷Cs, ¹⁵³Sm, ¹⁶⁵Dy, ¹⁶⁹Er, ¹⁷⁷Lu, ¹⁸⁶Re, ¹⁹²Ir, ¹⁹⁸Au, ²⁰¹Tl, ²¹³Bi, ²²³Ra, and/or ²²⁵Ra atom.

In one embodiment, the CD45RC-binding domain is conjugated to auristatin or an analog, derivative or prodrug thereof. Auristatins have been shown to interfere with microtubule dynamics, GTP hydrolysis and nuclear and cellular division (Woyke et al., 2001. Antimicrob Agents Chemother. 46(12):3802-8) and have anti-cancer activity (US patent 5,663,149) and antifungal activity (Pettit et al., 1998. Antimicrob Agents Chemother. 42(11):2961-5). For example, auristatin E can be reacted with para-acetyl benzoic acid or benzoylvaleric acid to produce AEB and AEVB, respectively. Other typical auristatin derivatives include auristatin F, MMAF (monomethyl auristatin F), and MMAE (monomethyl auristatin E). Suitable auristatins and auristatin analogs, derivatives and prodrugs, as well as suitable linkers for conjugation of auristatins to antibodies, are described in, *e.g.,* US patents 5,635,483, 5,780,588 and 6,214,345 and in International patent publications WO 2002/088172, WO 2004/010957, WO 2005/081711, WO 2005/084390, WO 2006/132670, WO 2003/026577, WO 2007/00860, WO 2007/011968 and WO 2005/082023.

In one embodiment, the CD45RC-binding domain is conjugated to a pyrrolo[2,1-c][1,4]-benzodiazepine (PDB) (such as, *e.g*., anthramycin) or an analog, derivative or prodrug thereof. Suitable PDBs and PDB derivatives, and related technologies are described in the art.

In one embodiment, the CD45RC-binding domain is conjugated to a cytotoxic moiety selected from anthracycline, maytansine, calicheamicin, duocarmycin, rachelmycin (CC-1065), dolastatin 10, dolastatin 15, irinotecan, monomethyl auristatin E, monomethyl auristatin F, a PDB, or an analog, derivative, or prodrug of any thereof.

In one embodiment, the CD45RC-binding domain is conjugated to an anthracycline or an analog, derivative or prodrug thereof.

In one embodiment, the CD45RC-binding domain is conjugated to maytansine or an analog, derivative or prodrug thereof.

In one embodiment, the CD45RC-binding domain is conjugated to calicheamicin or an analog, derivative or prodrug thereof.

In one embodiment, the CD45RC-binding domain is conjugated to duocarmycin or an analog, derivative or prodrug thereof.

In one embodiment, the CD45RC-binding domain is conjugated to rachelmycin (CC-1065) or an analog, derivative or prodrug thereof.

In one embodiment, the anti-CD45RC antibody or antigen-binding fragment thereof is conjugated to dolastatin or an analog, derivative or prodrug thereof.

In one embodiment, the CD45RC-binding domain is conjugated to monomethyl auristatin E or an analog, derivative or prodrug thereof.

In one embodiment, the CD45RC-binding domain is conjugated to monomethyl auristatin F or an analog, derivative or prodrug thereof.

In one embodiment, the CD45RC-binding domain is conjugated to irinotecan or an analog, derivative or prodrug thereof.

In a second aspect, the present invention relates to an antibody or antigen-binding fragment thereof, specifically binding to CD45RC, and preferably to hCD45RC. Hence, the antibody or antigen-binding fragment thereof of the invention is an **"anti-CD45RC antibody or antigen-binding fragment thereof",** and preferably an **"anti-hCD45RC antibody or antigen-binding fragment thereof".**

In one embodiment, the anti-CD45RC antibody or antigen-binding fragment thereof comprises at least one CD45RC-binding domain, as defined hereinabove.

All features and embodiments described above for CD45RC-binding domains apply *mutatis mutandis* to the anti-CD45RC antibody or antigen-binding fragment thereof. Additional features are defined hereafter.

In one embodiment, the anti-CD45RC antibody or antigen-binding fragment thereof is a polyclonal antibody. Preferably, the anti-CD45RC antibody or antigen-binding fragment thereof is a monoclonal antibody.

Methods of producing polyclonal and monoclonal antibodies as well as fragments thereof are well known in the art (see, for example, Harlow and Lane, Antibodies: A Laboratory Manual, Cold Spring Harbor Laboratory, New York, 1988).

In one embodiment, the anti-CD45RC antibody or antigen-binding fragment thereof has an isotype selected from the group comprising or consisting of IgG (including IgG1, IgG2, IgG3 and IgG4), IgM, IgA (including IgA1 and IgA2), IgD and IgE.

The constant region of an antibody is important in the ability of an antibody to fix complement and mediate cell-dependent cytotoxicity and phagocytosis. Thus, as discussed herein, the isotype of an antibody may be selected on the basis of whether it is desirable for the antibody to mediate cytotoxicity/phagocytosis. Determination or selection of the isotype of an antibody may be performed by known methods in the art.

In one embodiment, the anti-CD45RC antibody or antigen-binding fragment thereof comprises a heavy chain constant region (abbreviated as HCCR or C_{H}) and/or light chain constant region (abbreviated as LCCR or C_{L}).

In one embodiment, the HCCR and/or light chain constant region LCCR are murine HCCR and/or LCCR.

In one embodiment, the HCCR and/or light chain constant region LCCR, preferably both, are human or humanized.

In one embodiment, the anti-CD45RC antibody or antigen-binding fragment thereof comprises a human or humanized HCCR comprising or consisting of the amino acid sequence of SEQ ID NO: 26; or a human or humanized HCCR comprising or consisting of an amino acid sequence sharing at least 70%, 75%, 80%, 85%, 90%, 95%, 96%, 97%, 98%, 99% or more sequence identity with the amino acid sequence of SEQ ID NO: 26.

| |
|---|
| SEQ ID NO: 26 ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKRVEPKSCDKTHT CPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAP IEKTISKAKGQPREPQVYTLPPSREEMTKNQVSLTCLVKGFYPSDIAVEWESNG QPENNYKTTPPVLDSDGSFFLYSKLTVDKSRWQQGNVFSCSVMHEALHNHYT QKSLSLSPGK |

In one embodiment, the anti-CD45RC antibody or antigen-binding fragment thereof comprises a human or humanized HCCR comprising or consisting of the amino acid sequence of SEQ ID NO: 27; or a human or humanized HCCR comprising or consisting of an amino acid sequence sharing at least 70%, 75%, 80%, 85%, 90%, 95%, 96%, 97%, 98%, 99% or more sequence identity with the amino acid sequence of SEQ ID NO: 27.

| |
|---|
| SEQ ID NO: 27 ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKRVEPKSCDKTHT CPPCPAPEAAGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPSS IEKTISKAKGQPREPQVYTLPPSREEMTKNQVSLTCLVKGFYPSDIAVEWESNG QPENNYKTTPPVLDSDGSFFLYSKLTVDKSRWQQGNVFSCSVMHEALHNHYT QKSLSLSPGK |

In one embodiment, the anti-CD45RC antibody or antigen-binding fragment thereof comprises a human or humanized HCCR comprising or consisting of the amino acid sequence of SEQ ID NO: 28; or a human or humanized HCCR comprising or consisting of an amino acid sequence sharing at least 70%, 75%, 80%, 85%, 90%, 95%, 96%, 97%, 98%, 99% or more sequence identity with the amino acid sequence of SEQ ID NO: 28.

| |
|---|
| SEQ ID NO: 28 ASTKGPSVFPLAPSSKSTSGGTAALGCLVKDYFPEPVTVSWNSGALTSGVHTFP AVLQSSGLYSLSSVVTVPSSSLGTQTYICNVNHKPSNTKVDKRVEPKSCVAAAH HHHHH |

In one embodiment, the anti-CD45RC antibody or antigen-binding fragment thereof comprises a human or humanized LCCR comprising or consisting of the amino acid sequence of SEQ ID NO: 29; or a human or humanized LCCR comprising or consisting of an amino acid sequence sharing at least 70%, 75%, 80%, 85%, 90%, 95%, 96%, 97%, 98%, 99% or more sequence identity with the amino acid sequence of SEQ ID NO: 29.

| |
|---|
| SEQ ID NO: 29  |

Hence, in one embodiment, the anti-CD45RC antibody or antigen-binding fragment thereof comprises:
- a HCVR comprising or consisting of the amino acid sequence of SEQ ID NO: 24;
- a LCVR comprising or consisting of the amino acid sequence of SEQ ID NO: 25;
- a HCCR comprising or consisting of the amino acid sequence of any one of SEQ ID NOs: 26, 27 or 28, preferably the amino acid sequence of SEQ ID NO: 26; and
- a LCCR comprising or consisting of the amino acid sequence of SEQ ID NO: 29.

In one embodiment, the anti-CD45RC antibody or antigen-binding fragment thereof comprises:
- a HCVR comprising the three CDRs with SEQ ID NOs: 10, 11 and 12, and framework regions sharing at least 70%, 75%, 80%, 85%, 90%, 95%, 96%, 97%, 98%, 99% or more sequence identity with the framework regions of SEQ ID NO: 24;
- a LCVR comprising the three CDRs with SEQ ID NOs: 13, 14 and 15, and framework regions sharing at least 70%, 75%, 80%, 85%, 90%, 95%, 96%, 97%, 98%, 99% or more sequence identity with the framework regions of SEQ ID NO: 25;
- a HCCR comprising or consisting of an amino acid sequence sharing at least 70%, 75%, 80%, 85%, 90%, 95%, 96%, 97%, 98%, 99% or more sequence identity with the amino acid sequence of any one of SEQ ID NOs: 26, 27 or 28, preferably with the amino acid sequence of SEQ ID NO: 26; and
- a LCCR comprising or consisting of an amino acid sequence sharing at least 70%, 75%, 80%, 85%, 90%, 95%, 96%, 97%, 98%, 99% or more sequence identity with the amino acid sequence of SEQ ID NO: 29;
while the anti-CD45RC antibody or antigen-binding fragment thereof retains its overall functional properties, in particular its binding specificity to CD45RC, preferably to hCD45RC.

It will be appreciated that the anti-CD45RC antibody or antigen-binding fragment thereof can be modified using methods well known in the art, *e.g.,* to improve the properties of the isolated antibody or antigen-binding fragment thereof, or enhance antibody-dependent cell-mediated cytotoxicity (ADCC), antibody-dependent cell-mediated phagocytosis (ADCP), and/or complement dependent cytotoxicity (CDC). Such modifications are well-known in the art.

For example, to slow clearance *in vivo* and obtain a more desirable pharmacokinetic profile, the anti-CD45RC antibody or antigen-binding fragment thereof may be modified with polyethylene glycol (PEG). Methods for site-specifically conjugating PEG to an antibody or antigen-binding fragment thereof are described in, *e.g.,* Leong et al., 2001. Cytokine. 16(3):106-19 or Delgado et al., 1996. Br J Cancer. 73(2):175-82.

Another non-limiting example of modification consists in the modification of the human Fc region of the antibody in order to enhance its affinity for an Fcy receptor. Methods of enhancing Fcy receptor binding include modifications of the primary amino acid sequence of the Fc fragment, in particular of the HCCR, and modifications of the Fc fragment carbohydrate structures and content.

Accordingly, antibodies comprising a reduced fucose content are known to enhance ADCC and/or ADCP response. Indeed, the removal of the core α-1,6-fucose moiety from the Fc fragment oligosaccharides has been demonstrated to improve ADCC and/or ADCP activity through improved FcγRIII receptor binding (see, *e.g.,* Carter, 2001. Nat Rev Cancer. 1(2):118-29; Kanda et al., 2007. Glycobiology. 17(1):104-18; Shields et al., 2002. J Biol Chem. 277(30):26733-40; Shinkawa et al., 2003. J Biol Chem. 278(5):3466-73; Niwa et al., 2004. Cancer Res. 64(6):2127-33; International patent publication WO 2014/140322).

Thus, the anti-CD45RC antibody or antigen-binding fragment thereof may comprise a reduced fucose content.

The term "fucose content", as used herein, refers to the percentage of fucosylated forms within the N-glycans attached to the N297 residue of the Fc fragment of each heavy chain of each antibody (corresponding to N180 of SEQ ID NO: 26 or 27).

The term **"reduced fucose content",** as used herein, refers to a fucose content of less than, or equal to, 85%. Advantageously, the fucose content ranges from about 65% to about 85%. Alternatively, the fucose content may be less or equal to 85%, 80%, 75%, 70%, 65%, or even less, such as 60%, 55%, 50%, 45%, 40%, 35%, 30%, 25% or 20%. However, it is not necessary that the fucose content be zero, and it may for example be greater than or equal to 5%, 10%, 15%, or 20%.

The level of another glycoform, bisected N-linked carbohydrate, has also been suggested to increase ADCC and/or ADCP (see, *e.g.,* Umaña et al., 1999. Nat Biotechnol. 17(2):176-80; Hodoniczky et al., 2005. Biotechnol Prog. 21(6):1644-52).

In one embodiment, the anti-CD45RC antibody or antigen-binding fragment thereof may further comprises different types of glycosylation (N-glycans of the oligomannose or biantennary complex type, with a variable proportion of bisecting N-acetylglucosamine (GlcNAc) residues or galactose residues in the case of N-glycans of the biantennary complex type), provided that they have a reduced fucose content as defined above (International patent publication WO 2007/048077). For example, antibodies having a reduced fucose content can be obtained by methods described, *e.g*., in European patent publication EP 1 176 195 or in International patent publications WO 2001/077181 or WO 2012/041768.

The N-glycans of the oligomannose type have reduced half-life *in vivo* as compared to N-glycans of the biantennary complex type. Consequently, advantageously, the anti-CD45RC antibody or antigen-binding fragment thereof have on their N-glycosylation sites of the Fc fragment glycan structures of the biantennary complex type, with a reduced fucose content, as defined above.

A variety of Fc fragment variants with optimized binding affinity for Fcy receptors and/or enhanced ADCC and/or ADCP have been described and are known in the art.

In one embodiment, the anti-CD45RC antibody or antigen-binding fragment thereof is conjugated, optionally through a linker, to a payload. Suitable examples of payloads have been described above in the context of CD45RC-binding domains, which apply here *mutatis mutandis.*

In one embodiment, the payload is a drug. Conjugates between an antibody or antigen-binding fragment thereof and a drug are known in the art as **"antibody-drug conjugates"** or **"ADCs".**

In a third aspect, the present invention relates to a chimeric antigen receptor (CAR) specifically binding to CD45RC, and preferably to hCD45RC. Hence, the CAR of the invention is an **"anti-CD45RC CAR",** and preferably an **"anti-hCD45RC CAR".**

In one embodiment, the anti-CD45RC CAR comprises:
(i) at least one extracellular CD45RC-binding domain, preferably at least one extracellular hCD45RC-binding domain, as described above;
(ii) optionally, an extracellular hinge domain;
(iii) at least one transmembrane domain; and
(iv) at least one intracellular signaling domain comprising at least one primary signaling domain and optionally, one or more costimulatory signaling domain.

In one embodiment, the CAR comprises at least one extracellular CD45RC-binding domain, as described above.

In one embodiment, the extracellular CD45RC-binding domain is a CD45RC-binding scFv.

In one embodiment, the CAR comprises two or more extracellular antigen-binding domain, at least a first one of which is a CD45RC-binding domain, as described above, and at least a second one of which specifically binds to an antigen other than CD45RC. Such a CAR is therefore capable of binding to at least two different antigens, one of which being CD45RC.

In one embodiment, the extracellular antigen-binding domain, in particular the extracellular CD45RC-binding domain, is connected to a transmembrane domain through a hinge domain.

In one embodiment, the hinge domain is a short polypeptide linker, preferably having a length ranging from 2 to 25 amino acids. In one embodiment, the hinge domain is a linker similar to the linker between the V_{H} and the V_{L} domains in a scFv, as described hereinabove.

In one embodiment, the hinge domain is a glycine-serine linker as described hereabove, such as, *e.g.,* a (GS)ₙ linker, a (G₂S)ₙ linker, a (G₃S)ₙ linker, a (G₄S)ₙ linker, etc.; with n being a positive integer. In one embodiment, n is equal to 1, 2, 3, 4, 5 or more. In one embodiment, the hinge domain may be a glycine-serine linker with any one of SEQ ID NOs: 30-39.

Another example of suitable hinge domain is described in International patent publication WO 2012/138475, incorporated herein by reference.

In one embodiment, the hinge domain may be any one of SEQ ID NOs: 40-44.

| |
|---|
| **SEQ ID NO: 40** |
| AGSSSSGGSTTGGSTT |
| **SEQ ID NO: 41** |
| GTTAASGSSGGSSSGA |
| **SEQ ID NO: 42** |
| SSATATAGTGSSTGST |
| **SEQ ID NO: 43** |
| TSGSTGTAASSTSTST |
| **SEQ ID NO: 44** |
| KIRRDSS |

In one embodiment, the hinge domain comprises or consists in the amino acid sequence of a CD8 hinge with SEQ ID NO: 45 or an amino acid sequence sharing at least 70%, 75%, 80%, 85%, 90%, 91%, 92%, 93%, 94%, 95%, 96%, 97%, 98% or 99% amino acid sequence identity with SEQ ID NO: 45.

| |
|---|
| **SEQ ID NO: 45** |
| TTTPAPRPPTPAPTIASQPLSLRPEACRPAAGGAVHTRGLDFACD |

In another embodiment, the hinge domain comprises or consists in the amino acid sequence of a IgG4 hinge with SEQ ID NO: 46 or an amino acid sequence sharing at least 70%, 75%, 80%, 85%, 90%, 91%, 92%, 93%, 94%, 95%, 96%, 97%, 98% or 99% amino acid sequence identity with SEQ ID NO: 46.

| |
|---|
| SEQ ID NO: 46 ESKYGPPCPPCPAPEFLGGPSVFLFPPKPKDTLMISRTPEVTCVVVDVSQEDPEV QFNWYVDGVEVHNAKTKPREEQFNSTYRVVSVLTVLHQDWLNGKEYKCKVS NKGLPSSIEKTISKAKGQPREPQVYTLPPSQEEMTKNQVSLTCLVKGFYPSDIAV EWESNGQPENNYKTTPPVLDSDGSFFLYSRLTVDKSRWQEGNVFSCSVMHEAL HNHYTQKSLSLSLGKM |

In another embodiment, the hinge domain comprises or consists in the amino acid sequence of a IgD hinge with SEQ ID NO: 47 or an amino acid sequence sharing at least 70%, 75%, 80%, 85%, 90%, 91%, 92%, 93%, 94%, 95%, 96%, 97%, 98% or 99% amino acid sequence identity with SEQ ID NO: 47.

| |
|---|
| SEQ ID NO: 47 RWPESPKAQASSVPTAQPQAEGSLAKATTAPATTRNTGRGGEEKKKEKEKEEQ EERETKTPECPSHTQPLGVYLLTPAVQDLWLRDKATFTCFVVGSDLKDAHLTW EVAGKVPTGGVEEGLLERHSNGSQSQHSRLTLPRSLWNAGTSVTCTLNHPSLPP QRLMALREPAAQAPVKLSLNLLASSDPPEAASWLLCEVSGFSPPNILLMWLEDQ REVNTSGFAPARPPPQPGSTTFWAWSVLRVPAPPSPQPATYTCVVSHEDSRTLL NASRSLEVSYVTDH |

In another embodiment, the hinge domain comprises or consists in the amino acid sequence of a CD28 hinge with SEQ ID NO: 48 or an amino acid sequence sharing at least 70%, 75%, 80%, 85%, 90%, 91%, 92%, 93%, 94%, 95%, 96%, 97%, 98% or 99% amino acid sequence identity with SEQ ID NO: 48.

| |
|---|
| **SEQ ID NO: 48** |
| IEVMYPPPYLDNEKSNGTIIHVKGKHLCPSPLFPGPSKP |

Examples of transmembrane domains that may be used in CAR according to the present invention include, but are not limited to, transmembrane domains of an α, β or ζ chain of a T-cell receptor, or of CD28, CD3γ, CD3δ, CD3ε, CD3ζ, CD45, CD4, CD5, CD8, CD9, CD16, CD22, CD33, CD37, CD64, CD80, CD86, CD134, CD137, CD154, KIRDS2, OX40, CD2, CD27, LFA-1 α subunit (CD11a), LFA-1 β subunit (CD18), ICOS (CD278), 4-1BB (CD137), GITR, CD40, BAFFR, HVEM (LIGHTR), SLAMF7, NKp80 (KLRF1), CD160, CD19, IL2Rβ, IL2Rγ, IL7Rα, ITGA1, VLA1, CD49a, ITGA4, IA4, CD49d, ITGA6, VLA-6, CD49f, ITGAD, CD11d, ITGAE, CD103, ITGAL, ITGAM, CD11b, PD1, ITGAX, CD11c, ITGB1, CD29, ITGB2, ITGB7, TNFR2, DNAM1 (CD226), SLAMF4 (CD244), CD84, CD96 (Tactile), CEACAM1, CRTAM, Ly-9 (CD229), CD160 (BY55), PSGL1, CD100 (SEMA4D), SLAMF6 (Ly-108), SLAMF1 (CD150), BLAME (SLAMF8), SELPLG (CD162), LTBR, PAG/Cbp, NKp44, NKp30, NKp46, NKG2D, and NKG2C.

In one embodiment, the transmembrane domain may comprise the entire transmembrane domain of the molecule from which it is derived, or it may comprise only a functional and/or structural fragment or variant thereof.

In one embodiment, the transmembrane domain comprises or consists in the amino acid sequence of a CD8 transmembrane domain with SEQ ID NO: 49, or an amino acid sequence sharing at least 70%, 75%, 80%, 85%, 90%, 91%, 92%, 93%, 94%, 95%, 96%, 97%, 98% or 99% amino acid sequence identity with SEQ ID NO: 49.

| |
|---|
| **SEQ ID NO: 49** |
| IYIWAPLAGTCGVLLLSLVITLYC |

In another embodiment, the transmembrane domain comprises or consists in the amino acid sequence of a CD28 transmembrane domain with SEQ ID NO: 50, or an amino acid sequence sharing at least 70%, 75%, 80%, 85%, 90%, 91%, 92%, 93%, 94%, 95%, 96%, 97%, 98% or 99% amino acid sequence identity with SEQ ID NO: 50.

| |
|---|
| **SEQ ID NO: 50** |
| FWVLVVVGGVLACYSLLVTVAFIIFWV |

As used herein, the term **"intracellular signaling domain"** refers to an intracellular portion of a molecule. The intracellular signaling domain generates a signal that promotes an immune effector function of CAR-containing immune cell. Examples of immune effector functions in a CAR-immune cell include, without limitation, cytolytic activity, suppressive activity, regulatory activity and helper activity, including the secretion of cytokines.

In one embodiment, the intracellular signaling domain comprises at least one primary signaling domain, and optionally, one or more costimulatory signaling domains.

The primary signaling domain, also called endodomain, is cytoplasmic, and allows to transduce the effector function signal and direct the CAR-containing immune cell to perform its specialized function upon binding of the extracellular binding domain to its antigen.

Examples of primary signaling domain include, but are not limited to, ζ chain of the T cell receptor or any of its homologs (such as, *e.g.,* η chain, FcεR1γ and β chains, MB1 (Igα) chain, B29 (Igβ) chain, etc.), CD3 polypeptides (such as, *e.g.,* Δ, δ and ε), syk family tyrosine kinases (such as, *e.g.,* Syk, ZAP 70, etc.), src family tyrosine kinases (such as, *e.g.,* Lck, Fyn, Lyn, etc.) and other molecules involved in T cell transduction, such as, *e.g.,* CD2, CD5 and CD28.

In one embodiment, the intracellular signaling domain may be human CD3ζ chain, FcγRIII, FcεRI, cytoplasmic tails of Fc receptors, immunoreceptor tyrosine-based activation motif (ITAM) bearing cytoplasmic receptors or combinations thereof. Additional intracellular signaling domains will be apparent to those skilled in the art and may be used in connection with alternate embodiments of the invention.

In one embodiment, the at least one intracellular signaling domain may comprise the entire intracellular portion, or the entire native intracellular signaling domain, of the molecule from which it is derived, or a functional fragment or derivative thereof.

In one embodiment, the at least one intracellular primary signaling domain comprises or consists of a T cell primary signaling domain (or a sequence derived therefrom).

In one embodiment, the T cell primary signaling domain comprises or consists of a signaling domain of a protein selected in the group of CD3ζ, CD3γ, CD3δ, CD3ε, common FcRy (FCER1G), FcRε (Fc Epsilon Rib), CD79a, CD79b, FcγRIIa, DAP10, DAP12, and sequences derived therefrom.

In one embodiment, the T cell primary signaling domain comprises or consists of a functional signaling domain of CD3ζ.

In one embodiment, the T cell primary signaling domain comprises or consists of a functional signaling domain of CD3ζ with SEQ ID NO: 51; or an amino acid sequence with at least about 70%, 75%, 80%, 85%, 90%, 91%, 92%, 93%, 94%, 95%, 96%, 97%, 98% or 99% identity to SEQ ID NO: 51.

| |
|---|
| SEQ ID NO: 51 RVKFSRSADAPAYKQGQNQLYNELNLGRREEYDVLDKRRGRDPEMGGKPRRK NPQEGLYNELQKDKMAEAYSEIGMKGERRRGKGHDGLYQGLSTATKDTYDAL HMQALPPR |

T cell primary signaling domains that act in a stimulatory manner may additionally or alternatively comprise signaling motifs known as immunoreceptor tyrosine-based activation motifs (ITAMS).

Examples of ITAM-containing T cell primary intracellular signaling domains include, but are not limited to, those of (or derived from) CD3ζ, common FcRy (FCER1G), FcγRIIa, FcRβ (FcεR1b), CD3γ, CD3δ, CD3ε, CD5, CD22, CD66b, CD79a, CD79b, DAP10, and DAP12.

In one embodiment, the T cell primary signaling domain comprises a modified ITAM domain *(e.g.,* a mutated ITAM domain which has altered *e.g.,* increased or decreased) activity as compared to the native ITAM domain. In one embodiment, a primary signaling domain comprises a modified ITAM-containing primary intracellular signaling domain, *e.g*., an optimized and/or truncated ITAM-containing primary intracellular signaling domain. In one embodiment, a primary signaling domain comprises one, two, three, four or more ITAM motifs.

In one embodiment, the CAR comprises more than one intracellular primary signaling domain, such as 2, 3, 4, 5, or more, intracellular primary signaling domains.

In one embodiment, these two or more intracellular primary signaling domains may be linked to each other in tandem, in a random or in a specified order. Optionally, a linker, such as a flexible peptidic linker as described above, may form the linkage between two distinct intracellular primary signaling domains. Besides glycine-serine linkers described above, a single amino acid (such as, *e.g.,* an alanine or a glycine residue) may also be a suitable linker.

In one embodiment, the CAR of the invention can optionally comprise one or several costimulatory signaling domains.

As used herein, the term **"costimulatory signaling domain"** refers to the intracellular portion of a costimulatory molecule, *i.e.,* a cognate binding partner on a T cell that specifically binds with a costimulatory ligand, thereby mediating a costimulatory response by the T cell, such as, but not limited to, enhanced cell proliferation, enhanced cell survival and development of memory cells.

The one or several costimulatory signaling domains can be, individually, all or a portion of the intracellular domain of a co-stimulatory molecule including, but not limited to, 4-1BB (CD137), ICOS (CD278), CD27, CD28, CTLA-4 (CD152), PD-1, an MHC class I molecule, BTLA, a Toll ligand receptor, OX40, CD30, CD40, lymphocyte function-associated antigen-1 (LFA-1), CD2, CD7, LIGHT, NKG2C, B7-H3, a ligand that specifically binds with CD83, CDS, ICAM-1, GITR, ARHR, BAFFR, HVEM (LIGHTR), SLAMF7, NKp80 (KLRF1), NKp44, NKp30, NKp46, CD160 (BY55), CD19, CD19a, CD4, CD8α, CD8β, IL2Rα, IL6Rα, IL2Rβ, IL2Rγ, IL7Rα, IL-13Rα1, IL-13Rα2, IL-33R, IL-10Rα, IL-10Rβ, IL-4R, IL-5R (CSF2RB), IL-21R, ITGA4, VLA1, CD49a, ITGA4, IA4, CD49d, ITGA6, VLA-6, CD49f, ITGAD, CD11d, ITGAE, CD103, ITGAL, CD11a/CD18, ITGAM, CD11b, ITGAX, CD11c, ITGB1, CD29, ITGB2, CD18, ITGB7, NKG2D, NKG2C, CD95, TGFbR1/2/3, TRANCE/RANKL, DNAM1 (CD226), SLAMF4 (CD244), CD84, CD96 (Tactile), CEACAM1, CRTAM, Ly9 (CD229), PSGL1, CD100 (SEMA4D), CD69, SLAMF6 (NTB-A, Lyl08), SLAM (SLAMF1, CD150, IPO-3), BLAME (SLAMF8), SELPLG (CD162), LTBR, LAT, GADS, SLP-76, PAG/Cbp, common gamma chain, a ligand that specifically binds with CD83, NKp44, NKp30, NKp46, NKG2D, and any combination thereof.

In one embodiment, the CAR comprises at least one costimulatory signaling domain comprising all or a portion of the intracellular domain of 4-1BB, ICOS, OX40, CD28, CTLA4 or PD-1.

In one embodiment, the intracellular costimulatory signaling domain comprises or consists of all or a portion of the intracellular domain of 4-1BB.

In one embodiment, the intracellular costimulatory signaling domain comprises or consists of the intracellular domain of 4-1BB with SEQ ID NO: 52; or an amino acid sequence with at least about 70%, 75%, 80%, 85%, 90%, 91%, 92%, 93%, 94%, 95%, 96%, 97%, 98% or 99% identity to SEQ ID NO: 52; or a functionally active fragment thereof.

| |
|---|
| **SEQ ID NO: 52** |
| KRGRKKLLYIFKQPFMRPVQTTQEEDGCSCRFPEEEEGGCEL |

In one embodiment, the intracellular costimulatory signaling domain comprises or consists of all or a portion of the intracellular domain of ICOS.

In one embodiment, the intracellular costimulatory signaling domain comprises or consists of the intracellular domain of ICOS with SEQ ID NO: 53; or an amino acid sequence with at least about 70%, 75%, 80%, 85%, 90%, 91%, 92%, 93%, 94%, 95%, 96%, 97%, 98% or 99% identity to SEQ ID NO: 53; or a functionally active fragment thereof.

| |
|---|
| **SEQ ID NO: 53** |
| CWLTKKKYSSSVHDPNGEYMFMRAVNTAKKSRLTDVTL |

In one embodiment, the intracellular costimulatory signaling domain comprises or consists of all or a portion of the intracellular domain of OX40.

In one embodiment, the intracellular costimulatory signaling domain comprises or consists of the intracellular domain of OX40 with SEQ ID NO: 54; or an amino acid sequence with at least about 70%, 75%, 80%, 85%, 90%, 91%, 92%, 93%, 94%, 95%, 96%, 97%, 98% or 99% identity to SEQ ID NO: 54; or a functionally active fragment thereof.

| |
|---|
| **SEQ ID NO: 54** |
| ALYLLRRDQRLPPDAHKPPGGGSFRTPIQEEQADAHSTLAKI |

In one embodiment, the intracellular costimulatory signaling domain comprises or consists of all or a portion of the intracellular domain of CD28.

In one embodiment, the intracellular costimulatory signaling domain comprises or consists of the intracellular domain of CD28 with SEQ ID NO: 55; or an amino acid sequence with at least about 70%, 75%, 80%, 85%, 90%, 91%, 92%, 93%, 94%, 95%, 96%, 97%, 98% or 99% identity to SEQ ID NO: 55; or a functionally active fragment thereof.

| |
|---|
| **SEQ ID NO: 55** |
| RSKRSRLLHSDYMNMTPRRPGPTRKHYQPYAPPRDFAAYRS |

In one embodiment, the intracellular costimulatory signaling domain comprises or consists of all or a portion of the intracellular domain of CTLA4.

In one embodiment, the intracellular costimulatory signaling domain comprises or consists of the intracellular domain of CTLA4 with SEQ ID NO: 56; or an amino acid sequence with at least about 70%, 75%, 80%, 85%, 90%, 91%, 92%, 93%, 94%, 95%, 96%, 97%, 98% or 99% identity to SEQ ID NO: 56; or a functionally active fragment thereof.

| |
|---|
| **SEQ ID NO: 56** |
| AVSLSKMLKKRSPLTTGVYVKMPPTEPECEKQFQPYFIPIN |

In one embodiment, the intracellular costimulatory signaling domain comprises or consists of all or a portion of the intracellular domain of PD-1.

In one embodiment, the intracellular costimulatory signaling domain comprises or consists of the intracellular domain of PD-1 with SEQ ID NO: 57; or an amino acid sequence with at least about 70%, 75%, 80%, 85%, 90%, 91%, 92%, 93%, 94%, 95%, 96%, 97%, 98% or 99% identity to SEQ ID NO: 57; or a functionally active fragment thereof.

| |
|---|
| SEQ ID NO: 57 CSRAARGTIGARRTGQPLKEDPSAVPVFSVDYGELDFQWREKTPEPPVPCVPEQ TEYATIVFPSGMGTSSPARRGSADGPRSAQPLRPEDGHCSWPL |

In one embodiment, the CAR comprises more than one intracellular costimulatory signaling domain, such as 2, 3, 4, 5, or more, intracellular costimulatory signaling domains.

In one embodiment, these two or more intracellular costimulatory signaling domains may be linked to each other in tandem, in a random or in a specified order. Optionally, a linker, such as a flexible peptidic linker as described above, may form the linkage between two distinct intracellular costimulatory signaling domains. Besides glycine-serine linkers as described above, a single amino acid (such as, *e.g.,* an alanine or a glycine residue) may also be a suitable linker.

In one embodiment, the at least one intracellular primary signaling domain and at least one intracellular costimulatory signaling domain may be linked to each other in tandem, in a random or in a specified order. Optionally, a linker, such as a flexible peptidic linker as described above, may form the linkage between an intracellular primary signaling domain and an intracellular costimulatory signaling domain. Besides glycine-serine linkers described above, a single amino acid (such as, *e.g.,* an alanine or a glycine residue) may also be a suitable linker.

The CAR according to the present invention may be a first-generation, second-generation or third-generation CAR.

The first generation of CARs was developed more than 30 years ago (Kuwana et al., 1987. Biochem Biophys Res Commun. 149(3):960-968; Gross et al., 1989. Transplant Proc. 21(1 Pt 1):127-130; Gross et al., 1989. Proc Natl Acad Sci USA. 86(24):10024-10028).

In one embodiment, the CAR of the invention is a first-generation CAR and comprises:
(i) at least one extracellular CD45RC-binding domain, preferably at least one extracellular hCD45RC-binding domain, as described above,
(ii) optionally, an extracellular hinge domain,
(iii) at least one transmembrane domain, and
(iv) one or more intracellular primary signaling domain(s).

A first-generation CAR can be, for example, a CAR in which signaling is provided by CD3ζ, *i.e.,* the intracellular primary signaling domain is CD3ζ.

Second-generation CARs add an intracellular costimulatory signaling domain, such as, *e.g.,* CD28 or 4-1BB. The involvement of these intracellular signaling domains improve T cell proliferation, cytokine secretion, resistance to apoptosis, and *in vivo* persistence.

In one embodiment, the CAR of the invention is a second-generation CAR and comprises:
(i) at least one extracellular CD45RC-binding domain, preferably at least one extracellular hCD45RC-binding domain, as described above,
(ii) optionally, an extracellular hinge domain,
(iii) at least one transmembrane domain,
(iv) a costimulatory signaling domain, and
(v) one or more intracellular primary signaling domain(s).

Third-generation CARs combine multiple co-stimulatory domains, such as, *e.g.,* CD28-4-1BB or CD28-OX40, to increase T cell activity.

In one embodiment, the CAR of the invention is a third-generation CAR and comprises:
(i) at least one extracellular CD45RC-binding domain, preferably at least one extracellular hCD45RC-binding domain, as described above,
(ii) optionally, an extracellular hinge domain,
(iii) at least one transmembrane domain,
(iv) at least two costimulatory signaling domains, and
(v) one or more intracellular primary signaling domain(s).

In a fourth aspect, the present invention relates to a nucleic acid encoding the CD45RC-binding domain, or the anti-CD45RC antibody or antigen-binding fragment thereof, or the anti-CD45RC CAR, as described above.

It also relates to a vector comprising the nucleic acid encoding the CD45RC-binding domain, or the anti-CD45RC antibody or antigen-binding fragment thereof, or the anti-CD45RC CAR, described above.

In one embodiment, the vector is an expression vector and further comprises regulatory elements allowing for expression of the CD45RC-binding domain, of the anti-CD45RC antibody or the antigen-binding fragment thereof, or of the anti-CD45RC CAR, in a cell.

By **"expression vector",** it is meant any type of genetic construct comprising a nucleic acid coding for a sequence capable of being transcribed. Expression vectors can contain a variety of regulatory elements, which refer to nucleic acid sequences necessary for the transcription and possibly translation of an operably-linked coding sequence in a particular host cell. Examples of such regulatory elements include, but are not limited to, promoters, enhancers, internal ribosome entry sites (IRES), splicing sites, termination signals and the like.

Examples of vectors include, but are not limited to, DNA vectors, RNA vectors, plasmids, phagemids, phage derivatives, viruses and cosmids.

In one embodiment, the expression vector is a plasmid.

In one embodiment, the expression vector is a viral vector. Examples of viral vectors include, but are not limited to, adenoviral vectors, adeno-associated viral (AAV) vectors, retroviral vectors (such as lentiviruses), vaccinia virus vectors, sindbis virus vectors, cytomegalovirus vectors, herpes simplex virus vectors, and the like.

In one embodiment, the expression vector may be monocistronic. By **"monocistronic",** it is meant that a single nucleic acid encoding a single protein is expressed in a single expression vector.

In one embodiment, the expression vector comprises a sequence encoding the HCVR of the CD45RC-binding domain, of the anti-CD45RC antibody or the antigen-binding fragment thereof, or of the anti-CD45RC CAR, preferably operably linked to regulatory elements. In one embodiment, the expression vector comprises a sequence encoding the heavy chain, including the HCVR and the HCCR, of the CD45RC-binding domain, the anti-CD45RC antibody or the antigen-binding fragment thereof, preferably operably linked to regulatory elements. In one embodiment, the sequences of the HCVR and the HCCR are in the same open reading frame and therefore can be expressed as a single polypeptide chain.

In one embodiment, the expression vector comprises a sequence encoding the LCVR of the CD45RC-binding domain, of the anti-CD45RC antibody or the antigen-binding fragment thereof, or of the anti-CD45RC CAR, preferably operably linked to regulatory elements. In one embodiment, the expression vector comprises a sequence encoding the light chain, including the LCVR and the LCCR, of the CD45RC-binding domain, the anti-CD45RC antibody or the antigen-binding fragment thereof, preferably operably linked to regulatory elements. In one embodiment, the sequences of the LCVR and the LCCR are in the same open reading frame and therefore can be expressed as a single polypeptide chain.

Hence, the invention also relates to an expression vector system comprising or consisting of at least two expression vectors, one comprising a sequence encoding the HCVR of the CD45RC-binding domain, of the anti-CD45RC antibody or the antigen-binding fragment thereof, or of the anti-CD45RC CAR, preferably operably linked to regulatory elements; and one comprising a sequence encoding the LCVR of the CD45RC-binding domain, of the anti-CD45RC antibody or the antigen-binding fragment thereof, or of the anti-CD45RC CAR, preferably operably linked to regulatory elements.

In one embodiment, the expression vector comprises a sequence encoding the extracellular CD45RC-binding domain, optionally, the extracellular hinge domain, at least one transmembrane domain, and at least one intracellular signaling domain comprising at least one primary signaling domain and optionally, one or more costimulatory signaling domain, preferably operably linked to regulatory elements. In one embodiment, these sequences are in the same open reading frame and therefore can be expressed as a single polypeptide chain.

In one embodiment, the expression vector may be polycistronic. By **"polycistronic",** it is meant that at least two or more nucleic acids, each encoding a single protein, are expressed in a single expression vector.

In one embodiment, the expression vector comprises:
- a sequence encoding the HCVR of the CD45RC-binding domain, of the anti-CD45RC antibody or the antigen-binding fragment thereof, or of the anti-CD45RC CAR, preferably operably linked to regulatory elements; and
- a sequence encoding the LCVR of the CD45RC-binding domain, of the anti-CD45RC antibody or the antigen-binding fragment thereof, or of the anti-CD45RC CAR, preferably operably linked to regulatory elements.

It will be readily understood that the one skilled in the art can design nucleic acid sequences encoding the HCVR, LCVR, HCCR and/or LCCR disclosed herein. It is further understood that the one skilled in the art is familiar with molecular biology methods aiming at modifying a nucleic acid sequence in order to improve, *e.g.*, recombinant production rates, such as by codon optimization. Ultimately, the present application encompasses thus any nucleic acid encoding any HCVR, LCVR, HCCR and/or LCCR disclosed herein.

In one embodiment, the nucleic acid or vector is an isolated nucleic acid or vector.

By **"isolated",** it is meant that the nucleic acid or vector is substantially free of genomic DNA sequences, as well as proteins or complexes such as ribosomes and polymerases, which naturally accompany a native sequence. The term embraces a nucleic acid sequence that has been removed from its naturally occurring environment, and includes recombinant or cloned DNA isolates and chemically synthesized analogues or analogues biologically synthesized by heterologous systems.

In one embodiment, the isolated nucleic acid or vector is purified.

In one embodiment, the isolated nucleic acid or vector is purified to:
(1) greater than 80%, 85%, 90%, 91%, 92%, 93%, 94%, 95% or more by weight of nucleic acid as determined by absorbance methods or fluorescence methods (such as, *e.g.,* by measuring the ratio of absorbance at 260 and 280 nm (A_{260/280})), and most preferably more than 96%, 97%, 98% or 99% by weight; or
(2) homogeneity as shown by agarose gel electrophoresis and using an intercalating agent such as ethidium bromide, SYBR Green, GelGreen or the like.

In a fifth aspect, the present invention relates to a method of producing and purifying the CD45RC-binding domain, or the anti-CD45RC antibody or antigen-binding fragment thereof described above.

In one embodiment, the method comprises:
- culturing host cells comprising the nucleic acid, preferably the expression vector described above, under conditions suitable for expression of the CD45RC-binding domain, or the anti-CD45RC antibody or antigen-binding fragment thereof, and
- recovering the expressed CD45RC-binding domain, or anti-CD45RC antibody or antigen-binding fragment thereof.

This recombinant process can be used for large scale production of antibodies or antigen-binding fragments thereof, including monoclonal antibodies intended for *in vitro, ex vivo* and/or *in vivo* therapeutic and/or diagnostic uses.

The nucleic acid or expression vector may be propagated and expressed according to any of a variety of routinely practiced procedures for nucleic acid excision, ligation, transformation, and transfection. In certain embodiments, expression may be carried out in a prokaryotic host cell *(i.e.,* the host cell comprising the nucleic acid or expression vector is a prokaryotic host cell), such as *Escherichia coli.* In other embodiments, the expression may be carried out in a eukaryotic host cell *(i.e.,* the host cell comprising the nucleic acid or expression vector is a eukaryotic host cell), including animal cells (such as mammalian cells), yeast (*e.g., Saccharomyces cerevisiae, Schizosaccharomyces pombe,* or *Pichia pastoris*); and plant cells.

By methods known to those having ordinary skill in the art and based on the present disclosure, a nucleic acid or expression vector may be designed for expressing foreign sequences in a particular host system, and then polynucleotide sequences encoding the cellular polypeptide may be inserted. The regulatory elements will vary as appropriate for the particular host.

All these processes are well known in the art (see, *e.g*., Subramanian (Ed.), 2004. Antibodies (1st ed., Vol. 1: Production and Purification). New York, NY: Springer US).

In one embodiment, the expressed CD45RC-binding domain, or anti-CD45RC antibody or antigen-binding fragment thereof, is further purified.

Methods to purify antibodies or antigen-binding fragment thereof are well known in the art (see, *e.g.,* Subramanian (Ed.), 2004. Antibodies (1st ed., Vol. 1: Production and Purification). New York, NY: Springer US).

In a sixth aspect, the present invention relates to an immune cell expressing the anti-CD45RC CAR described herein, and to a population of such immune cells.

As used herein, the term **"immune cell"** refers to white blood cells (or leukocytes) that are derived from hematopoietic stem cells (HSC) produced in the bone marrow.

In one embodiment, the immune cell comprises the nucleic acid or vector, preferably expression vector, encoding the anti-CD45RC CAR described above, thereby generating an engineered immune cell expressing the anti-CD45RC CAR on its cell surface.

In one embodiment, the immune cell is a mammalian immune cell, *e.g.,* a human immune cell, an immune cell from a farm animal *(e.g.,* a cow, pig, or horse), or an immune cell from a pet *(e.g.,* a cat or a dog).

In one embodiment, the immune cells are autologous cells, heterologous cells, or allogenic cells.

In one embodiment, the immune cell is selected from the group comprising or consisting of lymphocytes, myeloid-derived cells, and any combination thereof.

In one embodiment, the immune cell is a lymphocyte, *e.g*., a cell selected from the group comprising or consisting of T cells, B cells, natural killer (NK) cells, natural killer T (NKT) cells and any combination thereof.

In one embodiment, the immune cell is a T cell, which can be selected from the group comprising or consisting of CD4⁺ T cells, CD8⁺ T cells, γδ T cells, double negative (DN) T cells, and any combination thereof.

In one embodiment, the immune cell is a CD4⁺ T cell, such as, *e.g.,* a T helper cell, a regulatory T cell, an effector T cell, and any combination thereof. In one embodiment, the immune cell is a CD8⁺ T cell, such as, *e.g.,* a cytotoxic CD8⁺ T cell or a CD8⁺ regulatory T cell. In one embodiment, the immune cell is a γδ T cell. In one embodiment, the immune cell is a T cell engineered to express a defined γδTCR (TEGs cells). In one embodiment, the immune cell is a DN T cell. In one embodiment, the immune cell is a NK cell. In one embodiment, the immune cell is a NKT cell.

In one embodiment, the immune cell is a regulatory immune cell, such as, for example, any regulatory immune cell suitable for use in cellular therapy. In one embodiment, the regulatory immune cell is selected from the group consisting of a regulatory T cell, a CD4⁺ regulatory T cell, a CD8⁺ regulatory T cell, a regulatory γδ T cell, a regulatory DN T cell, a regulatory B cell, a regulatory NK cell, a regulatory NKT cell, a regulatory macrophage, a regulatory dendritic cell, and any combination thereof.

In one embodiment, the regulatory immune cell is a regulatory T cell (Treg), in particular, a thymus-derived Treg or an adaptive or induced Treg. In one embodiment, the immune cell is a CD4⁺ Treg. In one embodiment, the Treg is a thymus-derived Treg or an adaptive or induced Treg. In one embodiment, the Treg is a CD4⁺FoxP3⁺ Treg or a CD4⁺FoxP3⁻ Treg (Tr1 cell). In one embodiment, the immune cell is a CD4⁺FoxP3⁺ regulatory T cell. In one embodiment, the immune cell is a CD8⁺ Treg. Examples of CD8⁺ Treg include, but are not limited to, a CD8⁺CD28⁻ Treg, a CD8⁺CD103⁺ Treg, a CD8⁺FoxP3⁺ Treg, a CD8⁺CD122⁺ Treg, and any combination thereof.

In one embodiment, the regulatory immune cell is a regulatory γδ T cell. In one embodiment, the regulatory immune cell is a regulatory DN T cell. In one embodiment, the regulatory immune cell is a regulatory NK cell. In one embodiment, the regulatory immune cell is a regulatory NKT cell.

In one embodiment, the immune cell is an effector immune cell, such as, *e.g.,* any effector immune cell suitable for use in cellular therapy. In one embodiment, the effector immune cell is selected from the group comprising or consisting of an effector T cell, a CD4⁺ effector T cell, a CD8⁺ effector T cell, an effector γδ T cell, an effector DN T cell, an effector NK cell, an effector NKT cell, and any combination thereof.

In one embodiment, the immune cell is an effector T cell (Teff). In one embodiment, the effector immune cell is a CD4⁺ Teff. Examples of CD4⁺ Teff include, but are not limited to, Tₕ1 cells, Tₕ2 cells, Tₕ9 cells, Tₕ17 cells, Tₕ22 cells, CD4⁺T follicular helper (Tfh) cells, and any combination thereof. In one embodiment, the effector immune cell is a CD8⁺ Teff. Examples of CD8⁺ effector T cells include, but are not limited to, a CD8⁺CD45RO⁺CCR7⁻CD62L⁻ Teff, a CD8⁺CD45RA⁺CCR7⁻CD62L⁻ Teff, and any combination thereof.

In one embodiment, the immune cell is an effector γδ T cell. In one embodiment, the immune cell is an effector DN T cell. In one embodiment, the immune cell is an effector NK cell. In one embodiment, the immune cell is an effector NKT cell.

The invention also relates to an isolated and/or substantially purified population of immune cells as described above.

As used herein, an **"isolated population"** refers to a cell population that is removed from its natural environment (such as the peripheral blood) and that is isolated, purified or separated, and is at least about 75% free, 80% free, 85% free, and in certain embodiments about 90%, 95%, 96%, 97%, 98%, 99% free, from other cells with which it is naturally present, but which lack the cell surface markers based on which the cells were isolated.

The invention also relates to an enriched population of immune cells as described above. In one embodiment, the enriched population comprises at least 30%, preferably at least 40%, 50%, 60%, 70%, 80%, 90%, or more of immune cells of interest.

In a seventh aspect, the present invention relates to a composition comprising the CD45RC-binding domain, or the anti-CD45RC antibody or antigen-binding fragment thereof, or the anti-CD45RC CAR, as described above; or a nucleic acid or vector, preferably expression vector, encoding therefor, as described above; or an immune cell expressing the anti-CD45RC CAR, as described above.

In one embodiment, the composition is a pharmaceutical composition and further comprises at least one pharmaceutically acceptable excipient.

The term **"pharmaceutically acceptable excipient"** includes any and all solvents, dispersion media, coatings, antibacterial and antifungal agents, isotonic and absorption delaying agents and the like. Said excipient does not produce an adverse, allergic or other untoward reaction when administered to an animal, preferably a human. For human administration, preparations should meet sterility, pyrogenicity, and general safety and purity standards as required by regulatory offices, such as, for example, FDA Office or EMA.

Pharmaceutically acceptable excipients that may be used in these compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (for example sodium carboxymethylcellulose), polyethylene glycol, polyacrylates, waxes, polyethylene-polyoxypropylene-block polymers, polyethylene glycol and wool fat.

In one embodiment, the pharmaceutical composition comprises vehicles which are pharmaceutically acceptable for a formulation capable of being injected to a subject. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

In one embodiment, the composition is a medicament.

In an eighth aspect, the present invention relates to uses of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, and the composition described above, in various methods of therapeutic purposes, as will be further described hereafter.

In one embodiment, the invention relates to methods of inducing immune tolerance in a subject in need thereof, by administering to the subject either of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above. It also relates to the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above, for use in inducing immune tolerance in a subject in need thereof. It also relates to the use of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above, in the preparation of a medicament for inducing immune tolerance in a subject in need thereof.

The term **"immune tolerance",** as used herein, relates to a state of unresponsiveness of the immune system to specific substances or tissues that have the capacity to elicit an immune response while preserving immune response against other substances or tissues.

The term **"immune response",** as used herein, includes T cell-mediated and/or B cell-mediated immune responses. Exemplary immune responses include, but are not limited to, T cell responses (*e.g*., cytokine production and cellular cytotoxicity), but also immune responses that are indirectly affected by T cell activation (*e.g*., macrophages). Immune cells involved in the immune response include lymphocytes (such as B cells and T cells, including CD4⁺, CD8⁺, Tₕ1 and Tₕ2 cells), antigen presenting cells (*e.g.,* professional antigen presenting cells such as dendritic cells), natural killer cells, myeloid cells (such as macrophages, eosinophils, mast cells, basophils, and granulocytes).

In one embodiment, the invention relates to methods of depleting CD45RC^{high} cells in a subject in need thereof, by administering to the subject either of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above. It also relates to the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above, for use in depleting CD45RC^{high} cells in a subject in need thereof. It also relates to the use of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above, in the preparation of a medicament for depleting CD45RC^{high} cells in a subject in need thereof.

The relative level of expression of CD45RC may be measured using flow cytometry. Three types of cells can be distinguished: cells presenting a high, intermediary or negative level of CD45RC expression.

In one embodiment, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above depletes CD45RC^{high} cells.

"**CD45RC^{high} cells**" are cells (such as, *e.g.,* T cells, NK cells, NKT cells, B cells, etc.) that express the CD45RC marker at a high level, as defined above.

As used herein, the terms **"deplete" or "depleting",** with respect to cells expressing CD45RC, refer to a measurable decrease in the number of cells in the subject. The reduction can be at least about 10%, *e.g.,* at least about 20%, 30%, 40%, 50%, 60%, 70%, 80%, 90%, 95%, 96%, 97%, 98%, 99%, or more. In one embodiment, the terms refer to a decrease in the number of CD45RC^{high} cells in a subject or in a sample to an amount below detectable limits.

In particular, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above deplete CD45RC^{high} cells by binding to CD45RC and transducing pro-apoptotic signals and/or by activating antibody-dependent cell mediated cytotoxicity (ADCC), antibody-dependent cell-mediated phagocytosis (ADCP), and/or complement dependent cytotoxicity (CDC).

In one embodiment, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above mediates complement dependent cytotoxicity.

In one embodiment, the CD45RC-binding domain, the anti-CD45RC antibody or antigen-binding fragment thereof, or the anti-CD45RC CAR described above may be conjugated to a cytotoxic agent or a growth inhibitory agent.

In one embodiment, the invention relates to methods of expanding and/or potentiating regulatory T cells in a subject in need thereof, by administering to the subject either of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above. It also relates to the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above, for use in expanding and/or potentiating regulatory T cells in a subject in need thereof. It also relates to the use of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above, in the preparation of a medicament for expanding and/or potentiating regulatory T cells in a subject in need thereof.

As used herein, the term **"expand"** refers to the process of converting and/or amplifying a given population of cells (*e.g*., immune cells such as Tregs). Expansion of a population of cells can occur *in vivo, in vitro* or *ex vivo.*

As used herein, the term "potentiate" refers to the process of increasing the function of a given population of cells (*e.g.*, increasing the suppressive capacity of Tregs). Potentiation of a population of cells can occur *in vivo, in vitro* or *ex vivo.*

**"Regulatory T cells"** or **"Tregs"** are T cells that suppress an abnormal or excessive immune response and play a role in immune tolerance. Tregs are typically "forkhead box P3 (Foxp3⁺) regulatory T cells" and/or "CD45RC^{low/-} cells".

As used herein, the terms **"forkhead box P3 (Foxp3⁺) regulatory T cells"** and "CD45RC^{low/-} **cells"** refer to 0.1-10% of CD4⁺ and/or CD8⁺ T cells in humans and rodents whose characteristic marker is the transcription factor Foxp3.

In one embodiment, the methods and uses are for expanding and/or potentiating Foxp3⁺ and/or CD45RC^{low/-} Tregs.

In one embodiment, CD45RC^{low/-} Tregs are expanded by stimulation. In one embodiment, CD45RC^{low/-} Tregs are expanded by stimulation in the presence of IL-2 and/or IL-15. In one embodiment, CD45RC^{low/-} Tregs are expanded by stimulation with anti-CD3/anti-CD28 antibodies and/or allogeneic antigen-presenting cells (APCs) and/or specific antigens.

Additionally or alternatively, the invention relates to *in vitro* or *ex vivo* methods of purifying CD45RC^{low/-} Tregs.

In one embodiment, CD45RC^{low/-} Tregs are CD8⁺/CD4⁺ T cells. In one embodiment, CD45RC^{low/-} Tregs are CD8⁺/CD4⁻ T cells. In one embodiment, CD45RC^{low/-} Tregs are CD8⁻/CD4⁺ T cells.

In one embodiment, purified CD45RC^{low/-} Tregs can be further expanded and/or potentiated prior to, concomitantly with or after administration to a subject in need thereof.

In one embodiment, the invention relates to methods of preventing and/or reducing transplant rejection in a subject in need thereof, by administering to the subject either of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above. It also relates to the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above, for use in preventing and/or reducing transplant rejection in a subject in need thereof. It also relates to the use of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above, in the preparation of a medicament for preventing and/or reducing transplant rejection in a subject in need thereof.

The terms **"preventing transplant rejection"** and **"reducing transplant rejection"** are meant to encompass prevention or inhibition of immune transplant rejection, as well as delaying the onset or the progression of immune transplant rejection. The terms are also meant to encompass prolonging survival of a transplant in a subject, or reversing failure of a transplant in a subject. Further, the terms are meant to encompass ameliorating a symptom of an immune transplant rejection, including, for example, ameliorating an immunological complication associated with immune rejection, such as, *e.g*., interstitial fibrosis, chronic graft arteriosclerosis, or vasculitis.

The term **"transplantation"** and variations thereof refer to the insertion of a transplant (also called graft) into a recipient, whether the transplantation is syngeneic (where the donor and recipient are genetically identical), allogeneic (where the donor and recipient are of different genetic origins but of the same species), or xenogeneic (where the donor and recipient are from different species). Thus, in a typical scenario, the host is human and the graft is an isograft, derived from a human of the same or different genetic origins. In another scenario, the graft is derived from a species different from that into which it is transplanted, including animals from phylogenetically widely separated species, for example, a baboon heart being transplanted into a human host.

The term **"transplant rejection",** as used herein, encompasses both acute and chronic transplant rejection.

**"Acute rejection"** is the rejection by the immune system of a tissue transplantrecipient when the transplanted tissue is immunologically foreign. Acute rejection is characterized by infiltration of the transplant tissue by immune cells of the recipient, which carry out their effector function and destroy the transplant tissue. The onset of acute rejection is rapid and generally occurs in humans within a few weeks after transplant surgery. Generally, acute rejection can be inhibited or suppressed with immunosuppressive drugs such as rapamycin, cyclosporin, anti-CD40L monoclonal antibody and the like.

**"Chronic rejection"** generally occurs in humans within several months to years after engraftment, even in the presence of successful immunosuppression of acute rejection. Fibrosis is a common factor in chronic rejection of all types of organ transplants.

In one embodiment, the transplant rejection is an allogeneic transplant rejection. Accordingly, in one embodiment, the donor of the transplant is a human. The donor of the transplant can be a living donor or a deceased donor, namely a cadaveric donor.

In one embodiment, the transplant is an organ, a tissue or cells.

As used herein, the term **"organ"** refers to a solid vascularized organ that performs a specific function or group of functions within an organism. The term organ includes, but is not limited to, heart, lung, kidney, liver, pancreas, skin, uterus, bone, cartilage, small or large bowel, bladder, brain, breast, blood vessels, esophagus, fallopian tube, gallbladder, ovaries, pancreas, prostate, placenta, spinal cord, limb including upper and lower, spleen, stomach, testes, thymus, thyroid, trachea, ureter, urethra, uterus.

As used herein, the term **"tissue"** refers to any type of tissue in human or animals, and includes, but is not limited to, vascular tissue, skin tissue, hepatic tissue, pancreatic tissue, neural tissue, urogenital tissue, gastrointestinal tissue, skeletal tissue including bone and cartilage, adipose tissue, connective tissue including tendons and ligaments, amniotic tissue, chorionic tissue, dura, pericardia, muscle tissue, glandular tissue, facial tissue, ophthalmic tissue.

The term "cells" refers here to a composition enriched for cells of interest, preferably a composition comprising at least 30%, preferably at least 50%, even more preferably at least 65 % of said cells. In one embodiment, the cells are selected from the group comprising or consisting of multipotent hematopoietic stem cells derived from bone marrow, peripheral blood, or umbilical cord blood; or pluripotent *(i.e.,* embryonic stem cells [ES] or induced pluripotent stem cells [iPS]) or multipotent stem cell-derived differentiated cells of different cell lineages, including, but not limited to, cardiomyocytes, β-pancreatic cells, hepatocytes, neurons and the like.

In one embodiment where the transplantation is an allogeneic hematopoietic stem cell transplantation (HSCT), the cells are selected from the group comprising or consisting of multipotent hematopoietic stem cells, usually derived from bone marrow, peripheral blood, or umbilical cord blood.

**"HSCT"** or **"hematopoietic stem cell transplantation"** is a transplantation therapy which can be curative for patients affected with leukemia and lymphomas (including, without limitation, acute myeloid leukemia (AML), acute lymphoid leukemia (ALL), chronic myeloid leukemia (CML), myelodysplasia syndrome (MDS), myeloproliferative syndrome, Hodgkin lymphomas, non-Hodgkin lymphomas, chronic lymphatic leukemia (CLL) and multiple myeloma). However, an important limitation of allogeneic HSCT is the development of graft-versus-host-disease (GvHD), which occurs in a severe form in about 30-50% of humans who receive this therapy.

Therefore, in one embodiment, the methods and uses are for preventing and/or reducing graft-versus-host-disease (GvHD).

In one embodiment, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above may be used in combination with multipotent hematopoietic stem cells to prevent and/or treat malignant diseases, such as leukemia and/or lymphomas (including, without limitation, acute myeloid leukemia (AML), acute lymphoid leukemia (ALL), chronic myeloid leukemia (CML), myelodysplasia syndrome (MDS), myeloproliferative syndrome, Hodgkin lymphomas, non-Hodgkin lymphomas, chronic lymphatic leukemia (CLL), neuroblastoma, Ewing sarcoma, myelodysplastic syndromes, glioma, and multiple myeloma), or non-malignant diseases, such as adrenoleukodystrophy, thalassemia, drepanocytic anemia, aplastic anemia, sickle cell anemia, Fanconi anemia, bone marrow failure syndromes, hemoglobinopathies, malignant infantile osteopetrosis, mucopolysaccharidosis, pyruvate kinase deficiency, immune deficiency syndromes, hemagophagocytic lymphohistiocytosis, inborn errors of metabolism, osteosclerotic myeloma (a.k.a. POEMS syndrome) and primary amyloidosis.

Additionally or alternatively, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above may be used for graft engineering.

In one embodiment, the transplant to be grafted is treated with the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above prior to transplantation, to deplete CD45RC^{high} cells.

In one embodiment, the transplant is bone marrow, and is treated with the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above prior to transplantation to deplete CD45RC^{high} T cells. In one embodiment, the bone marrow comprises CD34⁺ cells containing CD45RC^{high} T cells and CD45RC^{low/-} T cells.

In one embodiment, the invention relates to methods of preventing, reducing and/or treating CD45RC^{high}-related diseases, disorders or conditions in a subject in need thereof, by administering to the subject either of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above. It also relates to the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above, for use in preventing, reducing and/or treating CD45RC^{high}-related diseases, disorders or conditions in a subject in need thereof. It also relates to the use of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above, in the preparation of a medicament for preventing, reducing and/or treating CD45RC^{high}-related diseases, disorders or conditions in a subject in need thereof.

As used herein, the term "**CD45RC^{high}-related diseases, disorders or conditions"** refers to diseases, disorders or conditions caused by, or potentialized by, or characterized by, an increased proportion of cells expressing CD45RC cells in a subject and/or by an increased level of expression of CD45RC in cells of the subject.

By **"increased proportion of cell expressing CD45RC",** it is meant an increase of about 5%, preferably about 10%, 15%, 20%, 25%, 30%, 35%, 40%, 45%, 50%, 60%, 70%, 80%, 90%, 100% or more of the number of cells expressing CD45RC (*i.e.,* CD45RC^{high} cells) in a given subject as compared to a reference, such as, *e.g.,* the number of CD45RC^{high} cells in a substantially healthy subject *(i.e.,* a subject not suffering from a CD45RC^{high}-related disease, disorder or condition at stake).

By **"increased level of expression of CD45RC",** it is meant an increase of about 5%, preferably about 10%, 15%, 20%, 25%, 30%, 35%, 40%, 45%, 50%, 60%, 70%, 80%, 90%, 100% or more of the expression level of CD45RC, whether at the mRNA level or at the protein level, in cells of a given subject as compared to a reference, such as, *e.g.,* the expression level of CD45RC in cells of a substantially healthy subject (*i.e., a* subject not suffering from a CD45RC^{high}-related disease, disorder or condition at stake).

In one embodiment, CD45RC^{high}-related diseases, disorders or conditions are selected from the group comprising or consisting of autoimmune diseases, undesired immune responses, monogenic diseases and lymphoma or cancer.

In one embodiment, CD45RC^{high}-related diseases, disorders or conditions are selected from the group comprising or consisting of autoimmune diseases, undesired immune responses and monogenic diseases.

As used herein, the term **"autoimmune disease"** refers to a disease in which the immune system produces an immune response *(e.g.,* a B cell or a T cell response) against an antigen that is part of the normal host (that is an auto-antigen), with consequent injury to tissues. In an autoimmune disease, the immune system of the host fails to recognize a particular antigen as "self" and an immune reaction is mounted against the host's tissues expressing the antigen.

Exemplary autoimmune diseases contemplated in the present invention include, but are not limited to, rheumatoid arthritis, juvenile oligoarthritis, collagen-induced arthritis, adjuvant-induced arthritis, Sjogren's syndrome, multiple sclerosis, experimental autoimmune encephalomyelitis, inflammatory bowel disease (including Crohn's disease and ulcerative colitis), autoimmune gastric atrophy, pemphigus vulgaris, psoriasis, vitiligo, alopecia areata, type 1 diabetes, non-obese diabetes, myasthenia gravis, Grave's disease, Hashimoto's thyroiditis, sclerosing cholangitis, sclerosing sialadenitis, systemic lupus erythematosis, autoimmune thrombocytopenia purpura, Goodpasture's syndrome, Addison's disease, systemic sclerosis, polymyositis, dermatomyositis, acquired hemophilia, thrombotic thrombocytopenic purpura, uveitis, IgG4-associated autoimmune diseases (such as, *e.g.,* diseases listed in Table 1 of Kleger et al., 2015. Dtsch Arztebl Int. 112(8):128-135, which Table is incorporated by reference) and the like.

In one embodiment, the autoimmune disease is systemic lupus erythematosis.

In one embodiment, the autoimmune disease is inflammatory bowel disease, including Crohn's disease, colitis and ulcerative colitis. In one embodiment, the autoimmune disease is Crohn's disease. In one embodiment, the autoimmune disease is ulcerative colitis.

As used herein, the term **"undesired immune response"** refers to any unwanted immune reaction, preferably any unwanted immune reaction directed to (i) proteins expressed in the course of gene therapy, (ii) vectors (such as, *e.g.,* viral vectors) used in the course of gene therapy and/or (iii) therapeutic proteins. Such proteins include, *e.g.,* factor VIII (hemophilia A) and other coagulation factors, enzyme replacement therapies, monoclonal antibodies (e.g., natalizumab, rituximab, infliximab), polyclonal antibodies, enzymes and cytokines (e.g., IFNβ). The term **"undesired immune response"** also refers to allergies and allergic reactions.

In one embodiment, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above may be administered to a subject in order to suppress an immune response, especially to prevent immune reactions to specific proteins when their expression is restored by gene therapy in those subjects with corresponding genetic deficiencies. Thus, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above may be used to prevent immune reactivity towards proteins normally absent in the subject due to mutations, while their reconstitution is achieved by gene therapy. Moreover, protein therapy is an area of medical innovation that is becoming more widespread, and involves the application of proteins, such as enzymes, antibodies or cytokines, directly to subjects as therapeutic products. One of the major hurdles in delivery of such medicaments involves the immune responses directed against the therapeutic protein themselves. Administration of protein-based therapeutics is often accompanied by administration of immune suppressants, which are used in order to facilitate a longer lifetime of the protein and therefore increased uptake of the protein into the cells and tissues of the organism. General immune suppressants can however be disadvantageous due to the unspecific nature of the immune suppression that is carried out, resulting in unwanted side effects in the patient. Therefore, this approach can be applied to suppress an immune response against therapeutic proteins and peptides, such as therapeutic antibodies, cytokines, enzymes or any other protein administered to a patient.

In one embodiment, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above may be administered to a subject in order to suppress an immune response, especially to prevent immune reactions to vectors used in gene therapy, in particular viral vectors used in gene therapy. Such viral vectors include, *e.g*., adeno-associated virus (AAV) vectors, adenoviral (Ad) vectors, lentiviral vectors and the like. For a review, see Nayak & Herzog, 2010. Gene Ther. 17(3):295-304.

As used herein, the term **"allergy"** or **"allergies"** refers to an improper reaction of the immune system. Allergic reactions occur to normally harmless environmental substances known as allergens; these reactions are acquired, predictable and rapid. Strictly, allergy is one of four forms of hypersensitivity and is called type I (or immediate) hypersensitivity. It is characterized by excessive activation of certain white blood cells called mast cells and basophils by a type of antibody known as IgE, resulting in an extreme inflammatory response. Common allergic reactions include eczema, hives, hay fever, asthma, food allergies, celiac diseases, and reactions to the venom of stinging insects such as wasps and bees.

The term **"monogenic diseases",** as used herein, refers to diseases resulting from a mutation in a single gene selected among the following genes:
(i) genes which are not associated with immune function but whose deficiency is associated with inflammation and/or autoimmune reactions, such as genes deficient in the following diseases: Duchenne muscular dystrophy (DMD), cystic fibrosis, lysosomal diseases and α1-anti-trypsin deficiency; and
(ii) genes involved in the immune system and whose deficiency generates inflammation and/or autoimmune reactions, such as genes deficient in the following diseases: T cell primary immunodeficiency, such as IPEX (immunodysregulation polyendocrinopathy enteropathy X-linked syndrome), autoimmune polyendocrine syndrome type 1 (APS-1) (a.k.a. autoimmune polyendocrinopathy-candidiasis-ectodermal dystrophy or APECED), autoimmune polyendocrine syndrome type 2 (APS-2) (a.k.a. Schmidt's syndrome), autoimmune polyendocrine syndrome type 3 (APS-3) (a.k.a. autoimmune polyendocrinopathy), B cell primary immunodeficiencies, Muckle-Wells syndrome, mixed autoinflammatory and autoimmune syndrome, NLRP12-associated hereditary periodic fever syndrome, and tumor necrosis factor receptor 1 associated periodic syndrome.

Other autoimmune diseases from genetic origin (*i.e.,* monogenic disease as defined herein) as known in the art and encompassed herein under the term "monogenic diseases", as described, *e.g.,* in Table 1 of Ramos et al., 2015 (J Hum Genet. 60(11):657-64), which Table is herein incorporated by reference. In particular, autoimmune diseases from genetic origin encompassed herein are those associated with mutations in any one of the following genes: HLA-DR alleles, STAT4, PTPN22, IFIH1, TRAF3IP2, TRAF1-C5, PADI4, TNF, IL-1, IL-6, IL-4, IL-5, OPN, IL12A and IL12RB2.

In one embodiment, the monogenic disease is APECED (autoimmune polyendocrinopathy-candidiasis-ectodermal dystrophy).

In one embodiment, the monogenic disease is Duchenne muscular dystrophy (DMD).

The term **"lymphoma or cancer",** as used herein, encompass lymphoma or cancer which are associated with CD45RC^{high} cells. Exemplary lymphoma or cancer associated with CD45RC^{high} cells include, but are not limited to, acute myeloid leukemia (AML), acute lymphoid leukemia (ALL), chronic myeloid leukemia (CML), myelodysplasia syndrome (MDS)/myeloproliferative syndrome, lymphomas (such as, *e.g.,* Hodgkin and non-Hodgkin lymphomas), chronic lymphatic leukemia (CLL) and multiple myeloma.

In one embodiment, the CD45RC^{high}-related disease, disorder or condition is systemic lupus erythematosis, inflammatory bowel disease (including Crohn's disease and ulcerative colitis), APECED (autoimmune polyendocrinopathy-candidiasis-ectodermal dystrophy), or Duchenne muscular dystrophy (DMD).

In one embodiment, the CD45RC^{high}-related disease, disorder or condition is systemic lupus erythematosis, inflammatory bowel disease (including Crohn's disease and ulcerative colitis), or APECED (autoimmune polyendocrinopathy-candidiasis-ectodermal dystrophy).

In one embodiment, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above will be formulated for administration to the subject.

In one embodiment, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above is to be administered systemically or locally.

In one embodiment, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above is to be administered by injection, orally, topically, nasally, buccally, rectally, vaginaly, intratracheally, by endoscopy, transmucosally, or by percutaneous administration.

In one embodiment, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above is to be injected, preferably systemically injected.

Examples of formulations adapted for injection include, but are not limited to, solutions, such as, for example, sterile aqueous solutions, gels, dispersions, emulsions, suspensions, solid forms suitable for using to prepare solutions or suspensions upon the addition of a liquid prior to use, such as, for example, powder, liposomal forms and the like.

Examples of systemic injections include, but are not limited to, intravenous (iv), subcutaneous, intramuscular (im), intradermal (id), intraperitoneal (ip) injection and perfusion.

It will be understood that other suitable routes of administration are also contemplated in the present invention, and the administration mode will ultimately be decided by the attending physician within the scope of sound medical judgment. Apart from administration by injection (iv, ip, im and the like), other routes are available, such as nebulization (Respaud et al., 2014. MAbs. 6(5):1347-55; Guilleminault et al., 2014. J Control Release. 196:344-54; Respaud et al., 2015. Expert Opin Drug Deliv. 12(6):1027-39) or subcutaneous administration (Jackisch et al., 2014. Geburtshilfe Frauenheilkd. 74(4):343-349; Solal-Celigny, 2015. Expert Rev Hematol. 8(2):147-53).

In one embodiment, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above is to be administered to the subject in need thereof in a therapeutically effective amount.

The term **"therapeutically effective amount",** as used herein, refers to an amount effective, at dosages and for periods of time necessary, to achieve a desired preventive and/or therapeutic result.

It will be however understood that the total daily usage of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above will be decided by the attending physician within the scope of sound medical judgment. The specific therapeutically effective dose level for any particular patient will depend upon a variety of factors including the disease being treated and the severity of the disease; activity of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition employed; the age, body weight, general health, sex and diet of the subject; the time of administration, route of administration, and rate of excretion of the specific CD45RC-binding domain, anti-CD45RC antibody or antigen-binding fragment thereof, anti-CD45RC CAR, nucleic acid or vector encoding therefor, immune cell expressing the anti-CD45RC CAR or population of such immune cells, or composition employed; the duration of the treatment; drugs used in combination or coincidental with the specific CD45RC-binding domain, anti-CD45RC antibody or antigen-binding fragment thereof, anti-CD45RC CAR, nucleic acid or vector encoding therefor, immune cell expressing the anti-CD45RC CAR or population of such immune cells, or composition employed; and like factors well known in the medical arts. For example, it is well within the skill of the art to start doses of the compound at levels lower than those required to achieve the desired therapeutic effect and to gradually increase the dosage until the desired effect is achieved. The total dose required for each treatment may be administered by multiple doses or in a single dose.

In one embodiment, a therapeutically effective amount of the CD45RC-binding domain or of the anti-CD45RC antibody or antigen-binding fragment thereof ranges from about 0.1 mg/kg to about 5 mg/kg, from about 0.2 mg/kg to about 4 mg/kg, from about 0.3 mg/kg to about 3 mg/kg, from about 0.4 mg/kg to about 2.5 mg/kg, from about 0.5 mg/kg to about 2 mg/kg.

In one embodiment, a therapeutically effective amount of the CD45RC-binding domain or of the anti-CD45RC antibody or antigen-binding fragment thereof ranges from about 10 µg/kg to about 400 µg/kg, from about 20 µg/kg to about 300 µg/kg, from about 30 µg/kg to about 250 µg/kg, from about 35 µg/kg to about 200 µg/kg, from about 40 µg/kg to about 160 µg/kg.

In one embodiment, a therapeutically effective amount of the immune cell expressing the anti-CD45RC CAR or the population of such immune cells ranges from about 1 × 10² to about 1 × 10⁹ cells per kg of body weight, preferably from about 1 × 10³ to about 1 × 10⁸ cells per kg of body weight, including all integer values within those ranges.

In one embodiment, a therapeutically effective amount of the immune cell expressing the anti-CD45RC CAR or the population of such immune cells is at least 1 × 10², 1 × 10³, 1 × 10⁴, 1 × 10⁵, 1 × 10⁶, 1 × 10⁷, 1 × 10⁸ or 1 × 10⁹ cells per kg of body weight.

In one embodiment, a therapeutically effective amount of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition is to be administered once a day, twice a day, three times a day or more.

In one embodiment, a therapeutically effective amount of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition is to be administered every day, every two days, every three days, every four days, every five days, every six days.

In one embodiment, a therapeutically effective amount of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition is to be administered every week, every two weeks, every three weeks.

In one embodiment, a therapeutically effective amount of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition is to be administered every month, every two months, every three months, every four months, every five months, every six months.

In a preferred embodiment, a therapeutically effective amount of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition is to be administered every 12 hours, every 24 hours, every 36 hours, every 48 hours, every 60 hours, every 72 hours, every 96 hours.

In a preferred embodiment, a therapeutically effective amount of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition is to be administered every 60 hours.

In one embodiment, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition is for acute administration. In one embodiment, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition is for chronic administration.

In one embodiment, a therapeutically effective amount of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition is to be administered for about 5 days, 7 days, 10 days, 14 days, 21 days, 28 days, 1 month, 2 months, 3 months, 6 months, 1 year or more.

In one embodiment, a therapeutically effective amount of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition is to be administered for a period of time ranging from about one week to about eight weeks, from about two weeks to about seven weeks, from about two weeks to about six weeks, from about two weeks to about five weeks.

In a preferred embodiment, a therapeutically effective amount of the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition is to be administered for a period of time ranging from about 10 days to about 40 days, from about 15 days to about 35 days, from about 20 days to about 30 days.

In one embodiment, the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above is to be administered before, concomitantly with or after a therapeutic drug.

Examples of suitable therapeutic drugs have been described above as suitable payloads to be conjugated with the CD45RC-binding domain, the anti-CD45RC antibody or antigen-binding fragment thereof, the anti-CD45RC CAR, and include chemotherapeutic agents, targeted therapy agents, cytotoxic agents (or cytotoxins), cell cycle-synchronizing agents, ligands for cellular receptor(s), immunomodulatory agents, pro-apoptotic agents, lytic peptides, anti-angiogenic agents, cytokines, growth factors, and hormones.

It is readily understood that these therapeutic drugs may therefore be administered in a conjugated form, but also in a non-conjugated (or free) form along with the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above.

It will also be understood by the skilled artisan that the particular therapeutic drug for co-administration will depend on the disease or condition to be prevented and/or treated.

In an exemplary embodiment, where the CD45RC-binding domain, the anti-CD45RC antibody or antigen-binding fragment thereof, the anti-CD45RC CAR, the nucleic acid or vector encoding therefor, the immune cell expressing the anti-CD45RC CAR or the population of such immune cells, or the composition described above is for inducing immune tolerance in a subject in need thereof, or preventing and/or reducing transplant rejection, it may be desirable that an immunosuppressant agent be co-administered.

### BRIEF DESCRIPTION OF THE DRAWINGS

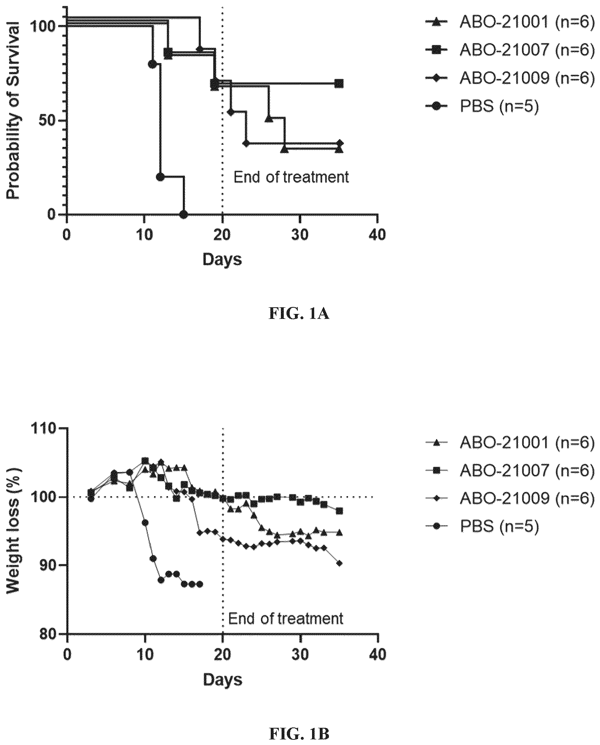

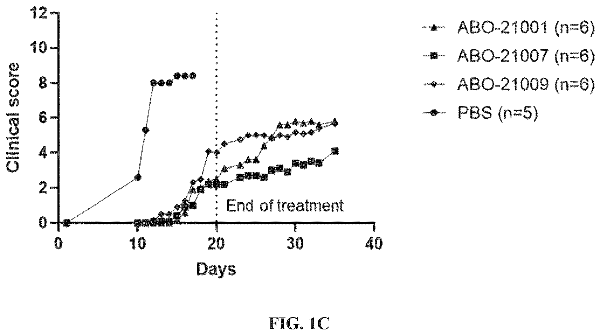

**Figures 1A-C** are a set of three graphs showing the effect of three anti-hCD45RC antibodies (ABO-21001, ABO-21007 and ABO-21009) to treat GvHD in NSG immunodeficient mice. **Figure 1A****:** survival curves. **Figure 1B****:** weight loss. **Figure 1C****:** clinical score.

### EXAMPLES

The present invention is further illustrated by the following examples. Throughout the examples, the following nomenclature applies:

**"ABIS-45RC":** a murine anti-hCD45RC antibody corresponding to the antibody #1 disclosed in Table 2 of WO2020/058495.

**"ABO-21001":** one of the best-in-class humanized variant of ABIS-45RC of WO2020/058495, corresponding to the antibody #92 disclosed in Table 2 of WO2020/058495.

**"ABO-21009":** one of the best-in-class humanized variant of ABIS-45RC of WO2020/058495, corresponding to the antibody #103 disclosed in Table 2 of WO2020/058495.

**"ABO-21007":** a humanized variant of ABIS-45RC, object of the present invention, comprising:
- a heavy chain variable region (HCVR) with SEQ ID NO: 24;
- a light chain variable region (LCVR) with SEQ ID NO: 25, wherein X₁ is absent, and X₂ is Tyr (Y);
- a heavy chain constant region (HCCR) with SEQ ID NO: 26; and
- a light chain constant region (LCCR) with SEQ ID NO: 29.

### Example 1: treatment of graft-versus-host-disease (GvHD) with ABO-21007

### Material and methods

### PBMC isolation

Blood was collected at the Établissement Français du Sang (Nantes, France) from healthy individuals. Written informed consent was provided according to institutional guidelines. PBMC were isolated by Ficoll-Paque density-gradient centrifugation (Eurobio, Courtaboeuf, France). Remaining red cells and platelets were eliminated with a hypotonic solution and centrifugation.

### Animals

8- to 12-week-old NOD/SCID/IL2Rγ^{-/-} (NSG) mice were bred in our own animal facilities in SPF conditions (accreditation number C44-278).

### GvHD model

Adult NSG immunodeficient mice were whole-body sublethaly irradiated (irradiation dose of 2 Gy at day -1) to induce lesions in tissues that will favor the development of GvHD. The following day (day 0), 1.5 × 10⁷ PBMCs (including CD45RC^{high} and CD45RC^{low/-} T cells) from healthy volunteers were injected intravenously in these mice. Human PBMCs, and in particular T cells, react against and attack mouse tissues inducing lesions. These T cells and the lesions observed in liver, intestine, lungs and skin mimic the GvHD observed following bone marrow transplantation in humans or other GvHD experimental systems using rodents as donors and recipients. In particular, these tissue lesions typically induce a body weight loss that begins - depending on the number of PBMCs injected and in our experimental system - around day 13 after injection of the PBMCs. Body weight loss is monitored daily and animals are sacrificed when it drops to 20 % of the original body weight to avoid unnecessary suffering.

### Treatment

NSG mice were treated intraperitoneally with the purified anti-CD45RC antibodies ABO-21001, ABO-21007 and ABO-21009, or with PBS at 0.8 mg/kg from day 0 and every 2.5 days during 20 days.

### Global clinical scoring

A GvHD global clinical score (from 0 to 12) was determined by compiling six individual scores, namely:
- body weight loss score (from 0 to 2);
- posture score (from 0 to 2);
- skin integrity score (from 0 to 2);
- activity score (from 0 to 2);
- fur texture score (from 0 to 2); and
- paleness score (from 0 to 2).

### Results

Treatment with PBMCs only (PBS) induced mice death initiated around day 11, and, as shown in **Figure 1A****,** death of all mice by day 15. Treatment with ABO-21001 and ABO-21009 increased the survival of the mice in comparison to PBS treated mice. At day 35, an average of 40 % of treated mice are still alive **(****Figure 1A****).** Surprisingly however, treatment with ABO-21007 significantly increased the survival of mice with an average of 70 % of mice that were still alive at 35 days **(****Figure 1A****).**

Similarly, upon treatment with ABO-21001 or ABO-21009, NSG mice lost weight although significantly less than NSG mice treated with PBS **(****Figure 1B****).** Surprisingly however, treatment with ABO-21007 did not lead to any weight loss.

Finally, the GvHD clinical score was also the lowest in mice treated with ABO-21007 **(****Figure 1C****).**

Altogether, these results demonstrate the improved characteristics of ABO-21007, compared to the already-improved anti-human CD45RC antibodies of WO 2020/058495.

## Claims

1. An antigen-binding domain specifically binding to CD45RC, wherein said antigen-binding domain comprises:
(a) a heavy chain variable region (HCVR) which comprises the following three CDRs:
(i) V_{H}-CDR1 of sequence SEQ ID NO: 10;
(ii) V_{H}-CDR2 of sequence SEQ ID NO: 11; and
(iii) V_{H}-CDR3 of sequence SEQ ID NO: 12; and
(b) a light chain variable region (LCVR) which comprises the following three CDRs:
(i) V_{L}-CDR1 of sequence SEQ ID NO: 13;
(ii) V_{L}-CDR2 of sequence SEQ ID NO: 14; and
(iii) V_{L}-CDR3 of sequence SEQ ID NO: 15,
wherein X in SEQ ID NO: 13 is either absent or is selected from the group consisting of Asn (N), Ser (S) and Gly (G); preferably X in SEQ ID NO: 13 is absent.

2. The antigen-binding domain according to claim 1, wherein said antigen-binding domain comprises:
1) a HCVR of sequence SEQ ID NO: 24 and a LCVR of sequence SEQ ID NO: 25; or
2) a HCVR comprising the three CDRs with SEQ ID NOs: 10, 11 and 12 and framework regions sharing at least 70% sequence identity with the framework regions of SEQ ID NO: 24, and a LCVR comprising the three CDRs with SEQ ID NOs: 13, 14 and 15 and framework regions sharing at least 70% sequence identity with the framework regions of SEQ ID NO: 25, wherein the antigen-binding domain retains its binding specificity to CD45RC;
wherein X₁ in SEQ ID NO: 25 is either absent or is selected from the group consisting of Asn (N), Ser (S) and Gly (G); preferably X in SEQ ID NO: 13 is absent; and
wherein X₂ in SEQ ID NO: 25 is selected from the group consisting of Tyr (Y) and Phe (F), preferably X₂ in SEQ ID NO: 25 is Tyr (Y).

3. The antigen-binding domain according to claim **1** or **2,** wherein said antigen-binding domain is selected from the group consisting of a single-chain variable fragment (scFv), a tandem-di-scFv, a tandem-tri-scFv, a scFv-Fc, a minibody, a maxibody, a diabody, a triabody, a Fv, a Fab, a Fab', a Fab'-SH, and a F(ab')2.

4. An antibody or antigen-binding fragment thereof specifically binding to CD45RC, wherein said antibody or antigen-binding fragment comprises an antigen-binding domain according to any one of claims **1** to **3.**

5. The antibody or antigen-binding fragment thereof according to claim **4,** further comprising a heavy chain constant region (HCCR) and a light chain constant region (LCCR),
preferably wherein the HCCR comprises an amino acid sequence sharing at least 70% sequence identity with the amino acid sequence of any one of SEQ ID NOs: 26 to 28, more preferably with SEQ ID NO: 26 and the LCCR comprises an amino acid sequence sharing at least 70% sequence identity with the amino acid sequence of SEQ ID NO: 29; and
wherein the antibody or antigen-binding fragment thereof retains its binding specificity to CD45RC.

6. A chimeric antigen receptor (CAR) specifically binding to CD45RC, comprising:
i) at least one extracellular antigen-binding domain according to any one of claims **1** to **3;**
ii) optionally, an extracellular hinge domain,
iii) at least one transmembrane domain,
preferably the transmembrane domain is selected from the group consisting of the CD8 transmembrane domain and the CD28 transmembrane domain, more preferably the transmembrane domain is selected from the group consisting of the CD8 transmembrane domain with an amino acid sequence sharing at least 70% sequence identity with the amino acid sequence of SEQ ID NO: 49 and the CD28 transmembrane domain with an amino acid sequence sharing at least 70% sequence identity with the amino acid sequence of SEQ ID NO: 50;
iv) at least one intracellular signaling domain comprising at least one primary signaling domain and optionally, one or more costimulatory signaling domain,
preferably the at least one primary signaling is a signaling domain of CD3ζ, preferably the at least one primary signaling is a signaling domain of CD3ζ with an amino acid sequence sharing at least 70% sequence identity with the amino acid sequence of SEQ ID NO: 51.

7. An immune cell population expressing the chimeric antigen receptor according to claim **6** at its cell surface.

8. The immune cell population according to claim **7,** wherein the immune cells of said population are selected from the group comprising CD4⁺ T cells, CD8⁺ T cells, double positive T cells, double negative T cells, γδ T cells, NK cells, NKT cell, B cells, macrophages, or dendritic cells.

9. A nucleic acid encoding: the antigen-binding domain according to any one of claims **1** to **3,** the antibody or antigen-binding fragment thereof according to claim **4** or **5,** or the CAR according to claim 6, preferably under control of regulatory elements.

10. The antigen-binding domain according to any one of claims **1** to **3,** the antibody or antigen-binding fragment thereof according to claim **4** or **5,** the immune cell population according to claim **7** or **8,** or the nucleic acid according to claim **9,** for use as a medicament.

11. The antigen-binding domain according to any one of claims **1** to **3,** the antibody or antigen-binding fragment thereof according to claim **4** or **5,** the immune cell population according to claim **7** or **8,** or the nucleic acid according to claim **9,** for use in inducing immune tolerance in a subject in need thereof.

12. The antigen-binding domain according to any one of claims **1** to **3,** the antibody or antigen-binding fragment thereof according to claim **4** or **5,** the immune cell population according to claim **7** or **8,** or the nucleic acid according to claim **9,** for use in depleting CD45RC^{high} cells in a subject in need thereof.

13. The antigen-binding domain according to any one of claims **1** to **3,** the antibody or antigen-binding fragment thereof according to claim **4** or **5,** the immune cell population according to claim **7** or **8,** or the nucleic acid according to claim **9,** for use in preventing and/or reducing transplant rejection in a subject in need thereof.

14. The antigen-binding domain according to any one of claims **1** to **3,** the antibody or antigen-binding fragment thereof according to claim **4** or **5,** the immune cell population according to claim **7** or **8,** or the nucleic acid according to claim **9,** for use in preventing and/or reducing graft-versus-host-disease (GvHD) in a subject in need thereof.

15. The antigen-binding domain according to any one of claims **1** to **3,** the antibody or antigen-binding fragment thereof according to claim **4** or **5,** the immune cell population according to claim **7** or **8,** or the nucleic acid according to claim **9,** for use in preventing, reducing and/or treating CD45RC^{high}-related diseases, disorders or conditions in a subject in need thereof
preferably wherein CD45RC^{high}-related diseases, disorders or conditions are selected from the group consisting of autoimmune diseases, undesired immune responses, monogenic diseases, lymphoma, and cancer.

## Patentansprüche

1. Antigen-bindende Domäne, die speziell an CD45RC bindet, wobei die Antigen-bindende Domäne Folgendes umfasst:
(a) eine variable Region der schweren Kette (HCVR), die die folgenden drei CDRs umfasst:
(i) V_{H}-CDR1 der Sequenz SEQ ID NO: 10;
(ii) V_{H}-CDR2 der Sequenz SEQ ID NO: 11; und
(iii) V_{H}-CDR3 der Sequenz SEQ ID NO: 12; und
(b) eine variable Region der leichten Kette (LCVR), die die folgenden drei CDRs umfasst:
(i) V_{L}-CDR1 der Sequenz SEQ ID NO: 13;
(ii) V_{L}-CDR2 der Sequenz SEQ ID NO: 14; und
(iii) V_{L}-CDR3 der Sequenz SEQ ID NO: 15,
wobei X in SEQ ID NO: 13 entweder fehlt oder aus der Gruppe bestehend aus Asn (N), Ser (S) und Gly (G) ausgewählt ist; wobei X vorzugsweise in SEQ ID NO: 13 fehlt.

2. Antigen-bindende Domäne nach Anspruch **1,** wobei die Antigen-bindende Domäne Folgendes umfasst:
1) eine HCVR der Sequenz SEQ ID NO: 24 und eine LCVR der Sequenz SEQ ID NO: 25; oder
2) eine HCVR, die die drei CDRs mit den SEQ ID NO: 10, 11 und 12 und Gerüstregionen umfasst, die mindestens 70 % der Sequenzidentität mit den Gerüstregionen der SEQ ID NO: 24 teilen, und eine LCVR, die die drei CDRs mit den SEQ ID NO: 13, 14 und 15 und Gerüstregionen umfasst, die mindestens 70 % der Sequenzidentität mit den Gerüstregionen der SEQ ID NO: 25 teilen, wobei die Antigen-bindende Domäne ihre Bindungsspezifität für CD45RC beibehält;
wobei X₁ in SEQ ID NO: 25 entweder fehlt oder aus der Gruppe bestehend aus Asn (N), Ser (S) und Gly (G) ausgewählt ist; wobei X vorzugsweise in SEQ ID NO: 13 fehlt; und
wobei X₂ in SEQ ID NO: 25 aus der Gruppe bestehend aus Tyr (Y) und Phe (F) ausgewählt ist, wobei X₂ vorzugsweise in SEQ ID NO: 25 Tyr (Y) ist.

3. Antigen-bindende Domäne nach Anspruch **1** oder **2,** wobei die Antigen-bindende Domäne aus der Gruppe ausgewählt ist, die aus einem einkettigen variablen Fragment (scFv), einem Tandem-di-scFv, einem Tandem-tri-scFv, einem scFv-Fc, einem Minibody, einem Maxibody, einem Diabody, einem Triabody, einem Fv, einem Fab, einem Fab', einem Fab'-SH und einem F(ab')2 besteht.

4. Antikörper oder Antigen-bindendes Fragment davon, der/das speziell an CD45RC bindet, wobei der Antikörper oder das Antigen-bindende Fragment eine Antigen-bindende Domäne nach einem der Ansprüche **1** bis **3** umfasst.

5. Antikörper oder Antigen-bindendes Fragment davon nach Anspruch **4,** ferner umfassend eine konstante Region der schweren Kette (HCCR) und eine konstante Region der leichten Kette (LCCR),
wobei die HCCR vorzugsweise eine Aminosäuresequenz umfasst, die mindestens 70 % Sequenzidentität mit der Aminosäuresequenz einer der SEQ ID NO: 26 bis 28, vorzugsweise mit SEQ ID NO: 26, teilt und die LCCR eine Aminosäuresequenz umfasst, die mindestens 70 % Sequenzidentität mit der Aminosäuresequenz der SEQ ID NO: 29 teilt; und
wobei der Antikörper oder das Antigen-bindende Fragment davon seine Bindungsspezifität an CD45RC beibehält.

6. Chimärer Antigenrezeptor (CAR), der speziell an CD45RC bindet, umfassend:
i) mindestens eine extrazelluläre Antigen-bindende Domäne nach einem der Ansprüche **1** bis **3;**
ii) optional eine extrazelluläre Scharnierdomäne,
iii) mindestens eine Transmembrandomäne,
wobei die Transmembrandomäne vorzugsweise aus der Gruppe ausgewählt ist, die aus der CD8-Transmembrandomäne und der CD28-Transmembrandomäne besteht, wobei die Transmembrandomäne noch bevorzugter aus der Gruppe ausgewählt ist, die aus der CD8-Transmembrandomäne mit einer Aminosäuresequenz, die mindestens 70 % Sequenzidentität mit der Aminosäuresequenz der SEQ ID NO: 49 teilt, und der CD28-Transmembrandomäne mit einer Aminosäuresequenz besteht, die mindestens 70 % Sequenzidentität mit der Aminosäuresequenz der SEQ ID NO: 50;
iv) mindestens eine intrazelluläre Signalisierungsdomäne, die mindestens eine primäre Signalisierungsdomäne und optional eine oder mehrere kostimulierende Signalisierungsdomänen umfasst,
wobei die mindestens eine Primärsignalisierung vorzugsweise eine Signalisierungsdomäne von CD3ζ ist, wobei die mindestens eine Primärsignalisierung vorzugsweise eine Signalisierungsdomäne von CD3ζ mit einer Aminosäuresequenz ist, die mindestens 70 % Sequenzidentität mit der Aminosäuresequenz der SEQ ID NO: 51.

7. Immunzellpopulation, die den chimären Antigenrezeptor nach Anspruch **6** an ihrer Zelloberfläche exprimiert.

8. Immunzellpopulation nach Anspruch **7,** wobei die Immunzellen der Population aus der Gruppe ausgewählt sind, die CD4⁺ T-Zellen, CD8⁺ T-Zellen, doppelt positive T-Zellen, doppelt negative T-Zellen, γδ T-Zellen, NK-Zellen, NKT-Zellen, B-Zellen, Makrophagen oder dendritischen Zellen umfasst.

9. Nukleinsäure, die Folgendes codiert: die Antigen-bindende Domäne nach einem der Ansprüche **1** bis **3,** den Antikörper oder das Antigen-bindende Fragment davon nach Anspruch **4** oder **5** oder den CAR nach Anspruch **6,** vorzugsweise unter Kontrolle regulatorischer Elemente.

10. Antigen-bindende Domäne nach einem der Ansprüche **1** bis **3,** Antikörper oder Antigen-bindendes Fragment davon nach Anspruch **4** oder **5,** Immunzellpopulation nach Anspruch **7** oder **8** oder Nukleinsäure nach Anspruch **9** zur Verwendung als Arzneimittel.

11. Antigen-bindende Domäne nach einem der Ansprüche **1** bis **3,** Antikörper oder Antigen-bindendes Fragment davon nach Anspruch **4** oder **5,** Immunzellpopulation nach Anspruch **7** oder **8** oder Nukleinsäure nach Anspruch **9** zur Verwendung zur Induktion der Immuntoleranz bei einem Subjekt, der diese benötigt.

12. Antigen-bindende Domäne nach einem der Ansprüche **1** bis **3,** Antikörper oder Antigen-bindendes Fragment davon nach Anspruch **4** oder **5,** Immunzellpopulation nach Anspruch **7** oder **8** oder Nukleinsäure nach Anspruch **9** zur Verwendung bei der Depletion von CD45RC^{high}-Zellen bei einem Subjekt, der diese benötigt.

13. Antigen-bindende Domäne nach einem der Ansprüche **1** bis **3,** Antikörper oder Antigen-bindendes Fragment davon nach Anspruch **4** oder **5,** Immunzellpopulation nach Anspruch **7** oder **8** oder Nukleinsäure nach Anspruch **9** zur Verwendung zur Verhinderung und/oder Reduzierung der Transplantatabstoßung bei einem Subjekt, der diese benötigt.

14. Antigen-bindende Domäne nach einem der Ansprüche **1** bis **3,** Antikörper oder Antigen-bindendes Fragment davon nach Anspruch **4** oder **5,** Immunzellpopulation nach Anspruch **7** oder **8** oder Nukleinsäure nach Anspruch **9** zur Verwendung zur Prävention und/oder Reduzierung von Graft-versus-Host-Disease (GvHD) bei einem Subjekt, der diese benötigt.

15. Antigen-bindende Domäne nach einem der Ansprüche **1** bis **3,** Antikörper oder Antigen-bindendes Fragment davon nach Anspruch **4** oder **5,** Immunzellpopulation nach Anspruch **7** oder **8** oder Nukleinsäure nach Anspruch **9** zur Verwendung zur Vorbeugung, Reduzierung und/oder Behandlung von CD45RC^{high}-bezogenen Krankheiten, Störungen oder Zuständen bei einem Patienten, der diese benötigt wobei die CD45RC^{high}-bezogenen Krankheiten, Störungen oder Zustände vorzugsweise aus der Gruppe ausgewählt werden, die aus Autoimmunerkrankungen, unerwünschten Immunreaktionen, monogenen Erkrankungen, Lymphomen und Krebs besteht.

## Revendications

1. Domaine de liaison à l'antigène se liant spécifiquement à CD45RC, dans lequel ledit domaine de liaison à l'antigène comprend :
(a) une région variable de chaîne lourde (HCVR) qui comprend les trois CDRs suivants :
(i) VH-CDR1 de séquence SEQ ID NO: 10 ;
(ii) VH-CDR2 de séquence SEQ ID NO: 11 ; et
(iii) VH-CDR3 de séquence SEQ ID NO: 12 ; et
(b) une région variable de chaîne légère (LCVR) qui comprend les trois CDRs suivants :
(i) VL-CDR1 de séquence SEQ ID NO: 13 ;
(ii) VL-CDR2 de séquence SEQ ID NO: 14 ; et
(iii) VL-CDR3 de séquence SEQ ID NO: 15,
dans lequel X dans SEQ ID NO: 13 est soit absent, soit sélectionné dans le groupe consistant en Asn (N), Ser (S) et Gly (G) ; de préférence, X dans SEQ ID NO: 13 est absent.

2. Domaine de liaison à l'antigène selon la revendication 1, dans lequel ledit domaine de liaison à l'antigène comprend :
1) un HCVR de séquence SEQ ID NO: 24 et un LCVR de séquence SEQ ID NO: 25 ; ou
2) un HCVR comprenant les trois CDRs de séquences SEQ ID NO: 10, 11 et 12 et des régions charpentes partageant au moins 70% d'identité de séquence avec les régions charpentes de SEQ ID NO: 24, et un LCVR comprenant les trois CDRs de séquences SEQ ID NO: 13, 14 et 15 et des régions charpentes partageant au moins 70% d'identité de séquence avec les régions charpentes de SEQ ID NO: 25, dans lequel le domaine de liaison à l'antigène conserve sa spécificité de liaison à CD45RC ;
dans lequel X₁ dans SEQ ID NO: 25 est soit absent, soit sélectionné dans le groupe consistant en Asn (N), Ser (S) et Gly (G) ; de préférence X dans SEQ ID NO: 13 est absent ; et
dans lequel X₂ dans SEQ ID NO: 25 est sélectionné dans le groupe consistant en Tyr (Y) et Phe (F), de préférence X₂ dans SEQ ID NO: 25 est Tyr (Y).

3. Domaine de liaison à l'antigène selon la revendication 1 ou 2, dans lequel ledit domaine de liaison à l'antigène est sélectionné dans le groupe consistant en un fragment variable à chaîne unique (scFv), un tandem-di-scFv, un tandem-tri-scFv, un scFv-Fc, un minibody, un maxibody, un diabody, un triabody, un Fv, un Fab, un Fab', un Fab'-SH, et un F(ab')2.

4. Anticorps ou fragment de liaison à l'antigène de celui-ci se liant spécifiquement à CD45RC, dans lequel ledit anticorps ou fragment de liaison à l'antigène comprend un domaine de liaison à l'antigène selon l'une des revendications 1 à 3.

5. L'anticorps ou fragment de liaison à l'antigène de celui-ci selon la revendication 4, comprenant en outre une région constante de chaîne lourde (HCCR) et une région constante de chaîne légère (LCCR),
de préférence dans laquelle la HCCR comprend une séquence d'acides aminés partageant au moins 70 % d'identité de séquence avec la séquence d'acides aminés de l'un quelconque des SEQ ID NOs : 26 à 28, de préférence avec SEQ ID NO: 26 et la LCCR comprend une séquence d'acides aminés partageant au moins 70 % d'identité de séquence avec la séquence d'acides aminés de SEQ ID NO: 29 ; et dans lequel l'anticorps ou fragment de liaison à l'antigène de celui-ci conserve sa spécificité de liaison à CD45RC.

6. Récepteur antigénique chimérique (CAR) se liant spécifiquement à CD45RC, comprenant :
i) au moins un domaine extracellulaire de liaison à l'antigène selon l'une des revendications 1 à 3 ;
ii) optionnellement, un domaine charnière extracellulaire,
iii) au moins un domaine transmembranaire,
de préférence le domaine transmembranaire est sélectionné dans le groupe consistant en le domaine transmembranaire CD8 et le domaine transmembranaire CD28,
de préférence encore le domaine transmembranaire est sélectionné dans le groupe consistant en le domaine transmembranaire CD8 avec une séquence d'acides aminés partageant au moins 70% d'identité de séquence avec la séquence d'acides aminés de SEQ ID NO: 49 et le domaine transmembranaire CD28 avec une séquence d'acides aminés partageant au moins 70% d'identité de séquence avec la séquence d'acides aminés de SEQ ID NO: 50 ;
iv) au moins un domaine de signalisation intracellulaire comprenant au moins un domaine de signalisation primaire et, optionnellement, un ou plusieurs domaines de signalisation et de costimulation,
de préférence le au moins un domaine de signalisation primaire est un domaine de signalisation de CD3ζ,
de préférence le au moins un domaine de signalisation primaire est un domaine de signalisation de CD3ζ avec une séquence d'acides aminés partageant au moins 70 % d'identité de séquence avec la séquence d'acides aminés de SEQ ID NO: 51.

7. Population de cellules immunitaires exprimant le récepteur d'antigène chimérique selon la revendication 6 à sa surface cellulaire.

8. Population de cellules immunitaires selon la revendication 7, dans laquelle les cellules immunitaires de ladite population sont sélectionnées dans le groupe comprenant les cellules T CD4⁺, les cellules T CD8⁺, les cellules T doublement positives, les cellules T doublement négatives, les cellules T γδ, les cellules NK, les cellules NKT, les cellules B, les macrophages, ou les cellules dendritiques.

9. Acide nucléique codant pour : le domaine de liaison à l'antigène selon l'une des revendications 1 à 3, l'anticorps ou fragment de liaison à l'antigène de celui-ci selon la revendication 4 ou 5, ou le CAR selon la revendication 6, de préférence sous le contrôle d'éléments régulateurs.

10. Domaine de liaison à l'antigène selon l'une des revendications 1 à 3, anticorps ou fragment de liaison à l'antigène de celui-ci selon la revendication 4 ou 5, population de cellules immunitaires selon la revendication 7 ou 8, ou acide nucléique selon la revendication 9, pour utilisation comme médicament.

11. Domaine de liaison à l'antigène selon l'une des revendications 1 à 3, anticorps ou fragment de liaison à l'antigène de celui-ci selon la revendication 4 ou 5, population de cellules immunitaires selon la revendication 7 ou 8, ou acide nucléique selon la revendication 9, pour utilisation dans l'induction d'une tolérance immunitaire chez un sujet qui en a besoin.

12. Le domaine de liaison à l'antigène selon l'une des revendications 1 à 3, l'anticorps ou fragment de liaison à l'antigène de celui-ci selon la revendication 4 ou 5, la population de cellules immunitaires selon la revendication 7 ou 8, ou l'acide nucléique selon la revendication 9, pour utilisation dans la déplétion des cellules CD45RC^{élevé} chez un sujet qui en a besoin.

13. Domaine de liaison à l'antigène selon l'une des revendications 1 à 3, anticorps ou fragment de liaison à l'antigène de celui-ci selon la revendication 4 ou 5, population de cellules immunitaires selon la revendication 7 ou 8, ou acide nucléique selon la revendication 9, pour utilisation dans la prévention et/ou la réduction du rejet de greffe chez un sujet qui en a besoin.

14. Domaine de liaison à l'antigène selon l'une des revendications 1 à 3, anticorps ou fragment de liaison à l'antigène de celui-ci selon la revendication 4 ou 5, population de cellules immunitaires selon la revendication 7 ou 8, ou acide nucléique selon la revendication 9, pour utilisation dans la prévention et/ou la réduction de la maladie du greffon contre l'hôte (GvHD) chez un sujet qui en a besoin.

15. Domaine de liaison à l'antigène selon l'une des revendications 1 à 3, anticorps ou fragment de liaison à l'antigène de celui-ci selon la revendication 4 ou 5, population de cellules immunitaires selon la revendication 7 ou 8, ou acide nucléique selon la revendication 9, pour utilisation dans la prévention, la réduction et/ou le traitement de maladies, troubles ou états liés au CD45RC^{élevé} chez un sujet qui en a besoin de préférence dans lequel les maladies, troubles ou états liés au CD45Rc^{élevé} sont sélectionnés dans le groupe consistant en les maladies auto-immunes, les réponses immunitaires indésirables, les maladies monogéniques, le lymphome et le cancer.
